(19) Europäisches Patentamt
European Patent Office
Office européen des brevets

(11) Publication number: **0 109 255**
**A2**

# (12) EUROPEAN PATENT APPLICATION

(21) Application number: 83306820.8

(22) Date of filing: 09.11.83

(51) Int. Cl.³: C 07 C 117/00, C 07 C 93/04, C 07 C 103/78, C 07 C 125/063, C 07 C 127/19, A 61 K 31/13, A 61 K 31/14, A 61 K 31/16, A 61 K 31/17, A 61 K 31/325

(30) Priority: 11.11.82 JP 196849/82

(43) Date of publication of application: 23.05.84 Bulletin 84/21

(84) Designated Contracting States: AT BE CH DE FR GB IT LI LU NL SE

(71) Applicant: ONO PHARMACEUTICAL CO., LTD., No. 14, Doshomachi 2-chome Higashi-ku, Osaka-shi Osaka (JP)

(72) Inventor: Arai, Yoshinobu, 1-5-8-505 Wakayama-dai, Shimamoto-cho Mishima-gun Osaka (JP)
Inventor: Itoh, Hiroyuki, 3-16-20 Yamate-dai, Ibaraki-shi Osaka (JP)
Inventor: Miyamoto, Tsumoru, 1-40 Tono-Moriyama, Joyo-shi Kyoto (JP)

(74) Representative: Pearce, Anthony Richmond et al, Marks & Clerk Alpha Tower Suffolk Street Queensway, Birmingham B1 1TT (GB)

## (54) Glycerol derivatives.

(57) Glycerol derivatives of the general formula:

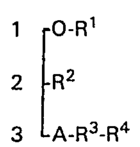

I

[wherein A represents an oxygen atom or a carbonyloxy group (i.e. -O-CO-, in which the carbonyl group should be attached to a group $R^3$ and the oxa group should be attached to the carbon atom at the third position of the glycerol skeleton), $R^1$ represents an alkyl group of 6 to 22 carbom atoms, or an alkyl group of 2 to 5 carbon atoms being substituted by a phenyl group, $R^2$ represents an azido group ($-N_3$ group), an amino group ($-NH_2$ group) or a group of the general formula: $-NHCOR^5$, $-NHCOOR^5$ or $-NHCONHR^5$, in which $R^5$ represents an alkyl group of 1 to 10 carbon atoms or a phenyl group, with the proviso that, when A represents a carbonyloxy group, $R^2$ does not represent an amino group, $R^3$ represents an alkylene group of 1 to 12 carbon atoms and $R^4$ represents an amino group, or a group of the general formula: $-NHCOR^6$, $-NHR^7$, $-N(R^7)_2$ or $-\overset{\oplus}{N}(R^7)_3 \cdot \overset{\ominus}{Y}$, in which $R^6$ represents an alkyl group of 1 to 6 carbon atoms or a phenyl group, $R^7$ represents an alkyl group of 1 to 8 carbon atoms, with the proviso that, when $R^4$ represents a group of the formula: $-N(R^7)_2$ or $-\overset{\oplus}{N}(R^7)_3 \cdot \overset{\ominus}{Y}$, two or more of $R^7$ may be same or different to each other, and $\overset{\ominus}{Y}$ represents an anion of an acid], and non-toxic acid addition salts thereof, possess antagonistic effect on PAF, hypotensive effect like PAF, inhibitory effect on phospholipase and inhibitory effect on growth of tumour cell or induction of differentiation, and are, therefore, useful as a platelet aggregation inhibitor, anti-asthmatic agent, anti-allergic agent, anti-inflammatory agent, hypotensive agent and anti-tumour agent for mammals.



ACTORUM AG

0109255

# D E S C R I P T I O N

## GLYCEROL DERIVATIVES

The present invention is concerned to new glycerol derivatives. More particularly, this invention is concerned to new glycerol derivatives having antagonistic effect on platelet activating factor (abbreviated as PAF hereafter), hypotensive effect like PAF, inhibitory effect on phospholipase and further, inhibitory effect on growth of tumour cell or induction of differentiation, and, furthermore, this invention is concerned to the process for their preparation and pharmaceutical composition comprising them as active ingredients.

Recently, the study on the release reaction of active amine dependent on leukocyte from platelet, has been energetically carried out. As a result, a substance strongly inducing platelet aggregation was discovered and it was named as PAF [cf. J. Immunol., 106, 1244 (1971), J. Exp. Med., 136, 1356 (1972) and Nature, 249, 581 (1974)]. Thereafter, it was confirmed that PAF exists in various animals including human beings. In 1979, its chemical structure was identified [cf. J. Biol. Chem., 254, 9355 (1979) and C. R. Acad. Sci., Série D (Paris), 289, 1017 (1979)] and has turned out to be a mixture of two alkylphospholipids of the following general formula wherein n is 15 and 17 [cf. J. Biol. Chem., 255, 5514 (1980)]:

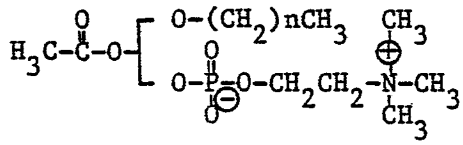

(wherein n represents 15 or 17).

It was found that PAF has not only strong secretory effect on platelet aggregation known at that time, but also powerful hypotensive effect and bronchodilating effect. PAF is considered to be one of platelet aggregating factors in human beings, and further, one of substances inducing allergy and inflammation. Accordingly, there is a great possibility that compounds having antagonistic effect on PAF (inhibitory effect on PAF) may become a platelet aggregation inhibitor, anti-asthmatic agent, anti-allergic agent or anti-inflammatory agent. Furthermore, PAF is considered to become a hypotensive agent without inhibitory effect on platelet aggregation by selecting hypotensive effect out of various effect.

Widespread investigations have been carried out in order to discover compounds possessing antagonistic effect on PAF or hypotensive effect like PAF, we have already proposed glycerol derivatives of the general formula:

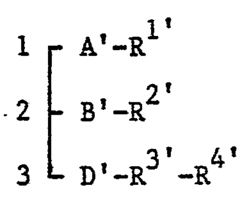

[wherein A' represents an oxygen atom or a sulfur atom, B' represents an oxygen atom or a sulfur atom, D' represents an oxygen atom or a carbonyloxy group (i.e. $-O\underset{\underset{O}{\|}}{C}-$ , in which the carbonyl group should be attached to a group $R^{3'}$ and the oxa group should be attached to the carbon atom at the third position of the glycerol skeleton), $R^{1'}$ represents an alkyl group of 6 to 22 carbon atoms, or an alkyl group of 2 to 5 carbon atoms being substituted by a phenyl group, $R^{2'}$ represents, when B' is an oxygen atom, a hydrogen atom, or a group of the general formula: $-CH_2-$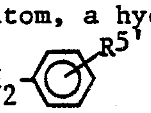 , $-COR^{6'}$ or $-CONHR^{7'}$, in which $R^{5'}$ represents a hydrogen atom, a halogen atom, an alkyl group of 1 to 4 carbon atoms or an alkoxy group of 1 to 4 carbon atoms, $R^{6'}$ represents an alkyl group of 1 to 6 carbon atoms or a phenyl group and $R^{7'}$ represents an alkyl group of 1 to 6 carbon atoms or a phenyl group, and $R^{2'}$ represents, when B' is a sulfur atom, a hydrogen atom, an alkyl group of 1 to 6 carbon atoms, or a group of the general formula: $-CH_2-$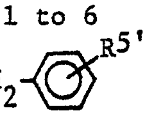 or $-CS-OR^{8'}$, in which $R^{8'}$ represents an alkyl group of 1 to 6 carbon atoms or a phenyl group and $R^{5'}$ is as hereinbefore defined, $R^{3'}$ represents an alkylene group of 1 to 12 carbon atoms and $R^{4'}$ represents an amino group, or a group of the general formula: $-NHCOR^{9'}$, $-NHR^{10'}$, $-N(R^{10'})_2$, $-\overset{\oplus}{N}(R^{10'})_3 \cdot \overset{\ominus}{Y}$, in which $R^{9'}$ represents an alkyl group of 1 to 6 carbon atoms or a phenyl group, $R^{10'}$ represents an alkyl group of 1 to 8 carbon atoms, with the proviso that, when $R^{4'}$ represents a group of the

formula $-N(R^{10'})_2$ or $-\overset{\oplus}{N}(R^{10'})_3 \cdot \overset{\ominus}{Y}$, two or more of $R^{10'}$ may be same or different to each other, and $\overset{\ominus}{Y}$ represents an anion of an acid], and non-toxic acid addition salts thereof (see the United States Patent Application No. 494481 and European Patent Application No. 83104345.5).

It has now been found that by replacing the group of the formula: $-B'-R^{2'}$ in compounds of the above general formula by an amino group or derivatives thereof, the pharmacological effects of new glycerol derivatives (hereinafter described as compounds of the general formula I) are, in some aspects of their effects, improved or modified, having completed this invention.

Furthermore, it has been found that the compounds of the present invention of the general formula I have inhibitory effect on phospholipase $A_2$ and phospholipase C. Phospholipase $A_2$ and phospholipase C dependent on calcium in a living body are concerned in the mechanism for the release of arachidonic acid from phospholipids. It is known that arachidonic acid is released by physiological stimulus and then becomes incorporated into the metabolic routes of prostaglandins and is finally metabolized to thromboxane $A_2$ having a strong effect on platelet aggregation, to other prostaglandins or to various leukotrienes being a significant bronchostrictor in an asthmatic paroxysm. Accordingly, it is considered that compounds having an

inhibitory effect on phospholipase $A_2$ and phospholipase C, which are concerned in the mechanism for the release of arachidonic acid, can become a platelet aggregation inhibitor, anti-asthmatic agent, anti-allergic agent or anti-inflammatory agent, based on the inhibition of phospholipase $A_2$ and phospholipase C, having a different mechanism from PAF antagonist and further, being different from conventional anti-inflammatory agents represented by aspirin based on the inhibition of cyclooxygenase.

Furthermore, the compounds of the present invention of the general formula I show inhibitory effect on growth of tumour cell and induction of differentiation and, therefore, are may be useful as anti-tumour agents.

Accordingly, the present invention provides new glycerol derivatives of the general formula:

1 ┌$-O-R^1$
2 ├$-R^2$          I
3 └$-A-R^3-R^4$

[wherein A represents an oxygen atom or a carbonyloxy group (i.e. -O-CO-, in which the carbonyl group should be attached to a group $R^3$ and the oxa group should be attached to the carbon atom at the third position of the glycerol skeleton), $R^1$ represents an alkyl group of 6 to 22 carbon atoms, or an alkyl

group of 2 to 5 carbon atoms being substituted by a phenyl group, $R^2$ represents an azido group ($-N_3$ group), an amino group ($-NH_2$ group) or a group of the general formula: $-NHCOR^5$, $-NHCOOR^5$ or $-NHCONHR^5$, in which $R^5$ represents an alkyl group of 1 to 10 carbon atoms or a phenyl group, with the proviso that, when A represents a carbonyloxy group, $R^2$ does not represent an amino group, $R^3$ represents an alkylene group of 1 to 12 carbon atoms and $R^4$ represents an amino group, or a group of the general formula: $-NHCOR^6$, $-NHR^7$, $-N(R^7)_2$ or $\overset{\oplus}{-N}(R^7)_3\cdot\overset{\ominus}{Y}$, in which $R^6$ represents an alkyl group of 1 to 6 carbon atoms or a phenyl group, $R^7$ represents an alkyl group of 1 to 8 carbon atoms, with the proviso that, when $R^4$ represents a group of the formula: $-N(R^7)_2$ or $\overset{\oplus}{-N}(R^7)_3\cdot\overset{\ominus}{Y}$, two or more of $R^7$ may be same or different to each other, and $\overset{\ominus}{Y}$ represents an anion of an acid], and non-toxic acid addition salts thereof.

Throughout the specification including claims, the term "alkyl" or "alkylene" means a straight- or branched-chain alkyl or alkylene group.

As is obvious to those skilled in the art, the compounds of the general formula I have at least one asymmetric center, i.e. the carbon atom at the second position of the glycerol skeleton. When the alkyl or alkylene moiety of various substituents represented by $R^1$, $R^2$, $R^3$ and $R^4$ represents a branched-chain, there is a possibility that other asymmetric centers are occurred. The existence of an asymmetric center

forms isomers as is well known. However the compounds of the general formula I in the present invention are concerned with all isomers or mixtures thereof.

Examples of the alkyl group represented by $R^1$ are hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl and docosyl group and isomers thereof. Any group is preferred and hexadecyl group is most preferred.

Examples of the alkyl group substituted by a phenyl group, represented by $R^1$ are ethyl, propyl, butyl and pentyl group and isomers thereof replaced an optional hydrogen atom by a phenyl group.

Examples of the alkyl group represented by $R^5$ in various groups represented by $R^2$ are methyl, ethyl, propyl, butyl, pentyl, hexyl, heptyl, octyl, nonyl and decyl group and isomers thereof and any group is preferred and most preferable group is an alkyl group of 1 to 5 carbon atoms, i.e. a methyl, ethyl, propyl, isopropyl, butyl, isobutyl, _sec_-butyl, _tert_-butyl, pentyl, isopentyl, neopentyl, _tert_-pentyl, 1-methylbutyl or 2-methylbutyl group. A phenyl group as $R^5$ is also preferred.

Preferable $R^2$ group is an azido group, an amino group or a group of the general formula: $-NHCOR^5$, $-NHCOOR^5$ or $-NHCONHR^5$ group, in which $R^5$ is as hereinbefore defined.

Examples of the alkylene group represented by $R^3$ are methylene, ethylene, trimethylene, tetramethylene, pentamethylene, hexamethylene, heptamethylene, octametylene, nonamethylene, decamethylene, undecamethylene and dodecamethylene group and isomers thereof, and any group is preferred.

Examples of the alkyl group represented by $R^6$ in the general formula: $-NHCOR^6$ in $R^4$ are methyl, ethyl, propyl, butyl, pentyl and hexyl group and isomers thereof and any group is preferred. A phenyl group as $R^6$ is also preferred.

Examples of the alkyl group represented by $R^7$ in the general formulae: $-NHR^7$, $-N(R^7)_2$ and $-\overset{\oplus}{N}(R^7)_3 \cdot \overset{\ominus}{Y}$ in $R^4$ are methyl, ethyl, propyl, butyl, pentyl, hexyl, heptyl and octyl and isomers thereof and any group is preferred.

The anion of an acid represented by $\overset{\ominus}{Y}$ means the anion of a pharmaceutically-acceptable non-toxic inorganic or organic acid, and examples of them are the anion of an inorganic acid such as hydrochloric acid, hydrobromic acid, hydroiodic acid, sulfuric acid, phosphoric acid, nitric acid, or the anion of an organic acid such as acetic acid, lactic acid, tartaric acid, benzoic acid, citric acid, methanesulfonic acid, ethanesulfonic acid, benzenesulfonic acid, toluenesulfonic acid, isethionic acid. Any anion is preferred and more preferable anion is a halogen ion, i.e. chlorine, bromine and iodine ions, or an anion of methanesulfonic acid or p-toluenesulfonic acid.

Preferable $R^4$ group is an amino group or a group of the general formula: $-NHR^7$, $-N(R^7)_2$ or $-\overset{\oplus}{N}(R^7)_3\cdot\overset{\ominus}{Y}$ (in which the various symbols are as hereinbefore defined) and most preferable $R^4$ is a group of the general formula: $-N(R^7)_2$ or $-\overset{\oplus}{N}(R^7)_3\cdot\overset{\ominus}{Y}$ (in which the various symbols are as hereinbefore defined).

According to a feature of the present invention, glycerol derivatives of the general formula I, wherein A represents an oxygen atom and the other symbols are as hereinbefore defined, i.e. compounds of the general formula:

$$\left[\begin{array}{l} -O-R^1 \\ -R^2 \\ -O-R^3-R^4 \end{array}\right. \qquad \text{IA}$$

(wherein the various symbols are as hereinbefore defined) may be prepared by the following methods:

(i) glycerol derivatives of the general formula IA, wherein $R^2$ represents an azido group and $R^4$ represents a group of the general formula: $-NHCOR^6$, $-N(R^7)_2$ or $-\overset{\oplus}{N}(R^7)_3\cdot\overset{\ominus}{Y}$, in which the various symbols are as hereinbefore defined, i.e. compounds of the general formula:

$$\left[\begin{array}{l} -O-R^1 \\ -N_3 \\ -O-R^3-R^{4a} \end{array}\right. \qquad \text{IA-11}$$

(wherein $R^{4a}$ represents a group of the general formula: $-NHCOR^6$, $-N(R^7)_2$ or $\overset{\oplus}{N}(R^7)_3 \cdot \overset{\ominus}{Y}$, in which the various symbols are as hereinbefore defined and the other symbols are as hereinbefore defined) may be prepared by reaction of a compound of the general formula:

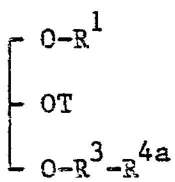

IIA

(wherein T represents an alkylsulfonyl group, having 1 to 4 carbon atoms in the alkyl group, or a substituted or unsubstituted arylsulfonyl group, preferably a mesyl group or a tosyl group and the other symbols are as hereinbefore defined) with sodium azide.

The above reaction is well known and may be carried out by using sodium azide in an inert organic solvent such as hexamethylphosphoramide (abbreviated as HMPA hereafter), N,N-dimethylformamide (abbreviated as DMF hereafter), dimethyl sulfoxide (abbreviated as DMSO hereafter) at a temperature from ambient to 80°C. The method for the conversion of an alkyl- or arylsulfonyloxy group into an azido group is abbreviated as method A hereafter.

(ii) Glycerol derivatives of the general formula IA, wherein $R^2$ represents an amino group and $R^4$ represents a group represented by $R^{4a}$, i.e. compounds of the general formula:

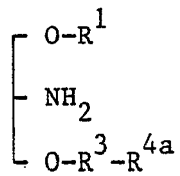

IA-12

(wherein the various symbols are as hereinbefore defined) may be prepared by reduction of an azido group of a compound of the general formula IA-11 into an amino group.

The reduction is well known and may be carried out, for example, under an atmosphere of hydrogen in the presence of a hydrogenation catalyst such as palladium on carbon, palladium black or platinum dioxide in an inert organic solvent, e.g. a lower alkanol such as methanol or ethanol, or ethyl acetate or acetic acid, or a mixture of two or more of them at a temperature from ambient to the reflux temperature of the reaction mixture, or may be carried out, when $R^{4a}$ is a group of the general formula: $-N(R^7)_2$, by using lithium aluminium hydride in an appropriate inert organic solvent, e.g. tetrahydrofuran (abbreviated as THF hereafter) or diethyl ether at a temperature from ambient to the reflux temperature of the reaction mixture. The method for the conversion of an azido group into an amino group is abbreviated as method B hereafter.

(iii) Glycerol derivatives of the general formula IA, wherein $R^2$ represents a group of the general formula: $-NHCOR^5$, in which $R^5$ is as hereinbefore defined, and $R^4$ represents a group represented by $R^{4a}$, i.e. compounds of the general formula:

$$\left[\begin{array}{l} O-R^1 \\ NHCOR^5 \\ O-R^3-R^{4a} \end{array}\right. \qquad \text{IA-13}$$

(wherein the various symbols are as hereinbefore defined) may be prepared by reaction of an amine of the general formula IA-12 with an acyl halide of the general formula:

$$X^1-CO-R^5 \qquad \text{III}$$

(wherein $X^1$ represents a halogen atom and $R^5$ is as hereinbefore defined) or with an acid anhydride of the general formula:

$$R^5-CO-O-CO-R^5 \qquad \text{IV}$$

(wherein $R^5$ is as hereinbefore defined).

(iv) Glycerol derivatives of the general formula IA, wherein $R^2$ represents a group of the general formula: $-NHCOOR^5$, in which $R^5$ is as hereinbefore defined, and $R^4$ represents a group represented by $R^{4a}$, i.e. compounds of the general formula:

$$\left[\begin{array}{l} O-R^1 \\ NHCOOR^5 \\ O-R^3-R^{4a} \end{array}\right. \qquad \text{IA-14}$$

(wherein the various symbols are as hereinbefore defined) may be prepared by reaction of an amine of the general formula IA-12 with an ester of haloformic acid of the general formula:

$$X^2-COOR^5 \qquad \text{V}$$

(wherein $X^2$ represents a halogen atom and $R^5$ is as hereinbefore defined).

(v) Glycerol derivatives of the general formula IA, wherein $R^2$ represents a group of the general formula $-NHCONHR^5$, in which $R^5$ is as hereinbefore defined, and $R^4$ represents a group represented by $R^{4a}$, i.e. compounds of the general formula:

$$\left[\begin{array}{l} O-R^1 \\ NHCONHR^5 \\ O-R^3-R^{4a} \end{array}\right. \qquad \text{IA-15}$$

(wherein the various symbols are as hereinbefore defined) may be prepared by reaction of an amine of the general formula IA-12 with an isocyanate of the general formula:

$O=C=N-R^5$ VI

(wherein $R^5$ is as hereinbefore defined).

The above reactions between an amine of the general formula IA-12 and a compound of the general formula III or IV, between an amine of the general formula IA-12 and a compound of the general formula V, and between an amine of the general formula IA-12 and a compound of the general formula VI are well known and may be carried out in an inert organic solvent, e.g. ethyl acetate, benzene, hexane, diethyl ether, THF, chloroform, methylene chloride or carbon tetrachloride, or a mixture of two or more of them, or in the absence of a solvent, in the presence of tertiary amine such as pyridine or triethylamine at a temperature from 0°C to 50°C. The methods for the conversion of an amino group into groups of the general formulae: $-NHCOR^5$, $-NHCOOR^5$ and $-NHCONHR^5$ are abbreviated hereafter as method C, D and E, respectively.

(vi) Glycerol derivatives of the general formula IA, wherein $R^4$ represents an amino group or a group of the general formula: $-NHR^7$, in which $R^7$ is as hereinbefore defined, i.e. compounds of the general formula:

$$\left[\begin{array}{l} O-R^1 \\ R^2 \\ O-R^3-NHR^8 \end{array}\right.$$ IA-2

(wherein $R^8$ represents a hydrogen atom or an alkyl group of 1 to 8 carbon atoms and the other symbols are as hereinbefore defined) may be prepared by elimination of <u>tert</u>-butoxycarbonyl group (i.e. boc group) of a compound of the general formula:

$$\left[\begin{array}{l} O{-}R^1 \\ R^2 \\ O{-}R^3{-}NR^8{-}boc \end{array}\right. \qquad \text{VII}$$

(wherein boc represents <u>tert</u>-butoxycarbonyl group and the other symbols are as hereinbefore defined).

The elimination of a boc group is well known, and may be carried out by using hydrochloric acid in an inert organic solvent such as ethyl acetate, methylene chloride, chloroform, carbon tetrachloride, a lower alkanol at a temperature from ambient to 50°C, or by using trifluoroacetic acid at room temperature.

Compounds of the general formula VII may be prepared by subjecting a compound of the general formula:

$$\left[\begin{array}{l} O{-}R^1 \\ OT \\ O{-}R^3{-}NR^8{-}boc \end{array}\right. \qquad \text{IIB}$$

(wherein the various symbols are as hereinbefore defined) to the series of method A, B, C, D and E.

Compounds of the general formulae IIA and IIB may be prepared by reaction of compounds of the general formulae:

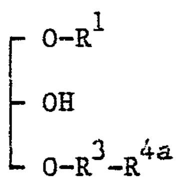

VIIIA

and

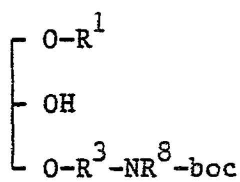

VIIIB

(wherein the various symbols are as hereinbefore defined), respectively, with a corresponding alkylsulfonyl chloride such as mesyl chloride or with a corresponding arylsulfonyl chloride such as tosyl chloride at a temperature from -30°C to 50°C (i) in the presence of a tertiary amine such as pyridine or triethylamine, in an inert organic solvent such as methylene chloride or (ii) in pyridine.

Compounds of the general formulae VIIIA and VIIIB may be prepared by reductive elimination of the benzyl group

attached to the 2nd position of compounds of the general formulae:

$$2\left[\begin{array}{l} O{-}R^1 \\ O{-}CH_2\phi \\ O{-}R^3{-}R^{4a} \end{array}\right. \qquad \text{IXA}$$

and

$$2\left[\begin{array}{l} O{-}R^1 \\ O{-}CH_2\phi \\ O{-}R^3{-}NR^8{-}boc \end{array}\right. \qquad \text{IXB}$$

(wherein $\phi$ represents a phenyl group and the other symbols are as hereinbefore defined), respectively, under an atmosphere of hydrogen in the presence of a hydrogenation catalyst such as palladium on carbon, palladium black or platinum dioxide in an inert organic solvent, e.g. a lower alkanol such as methanol or ethanol, or ethyl acetate or acetic acid, or a mixture of two or more of them at a temperature from ambient to the reflux temperature of the reaction mixture.

Compounds of the general formulae IXA and IXB may be prepared by method described in our United States Patent Application No. 494481 and European Patent Application No. 83104545.5 and by the series of reactions depicted schematically

below in Scheme A, wherein $R^9$ represents a halogen atom or a group of the formula: -OT, in which T is as hereinbefore defined, THP represents a tetrahydropyran-2-yl group, $Y^1$ represents an anion of an acid other than an anion represented by $\overset{\ominus}{R}{}^9$ ($R^9$ is as hereinbefore defined), X represents a halogen atom and the other symbols are as hereinbefore defined.

Scheme A

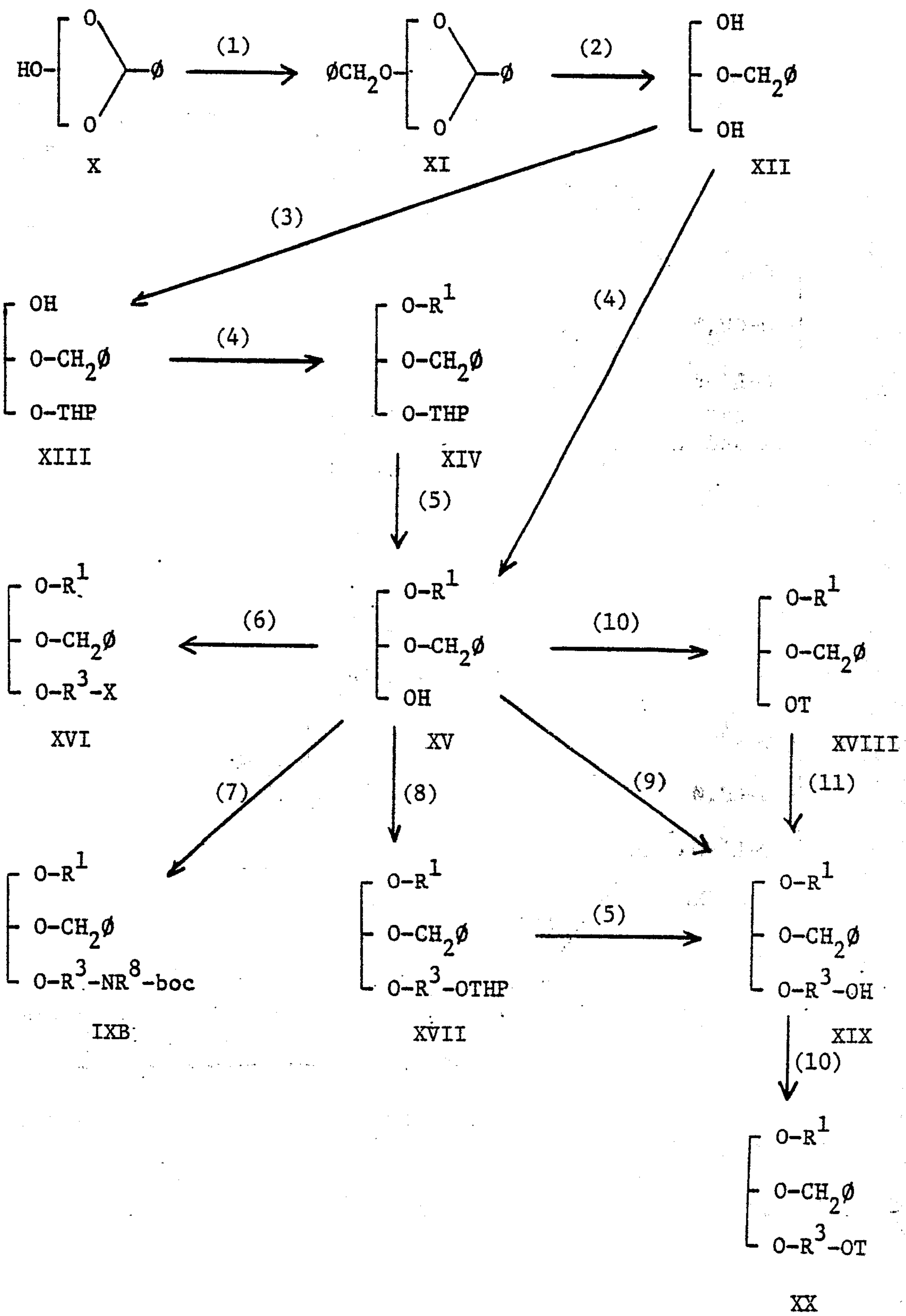


X XI XII XIII XIV XV XVI XVIII IXB XVII XIX XX

Scheme A (continued)

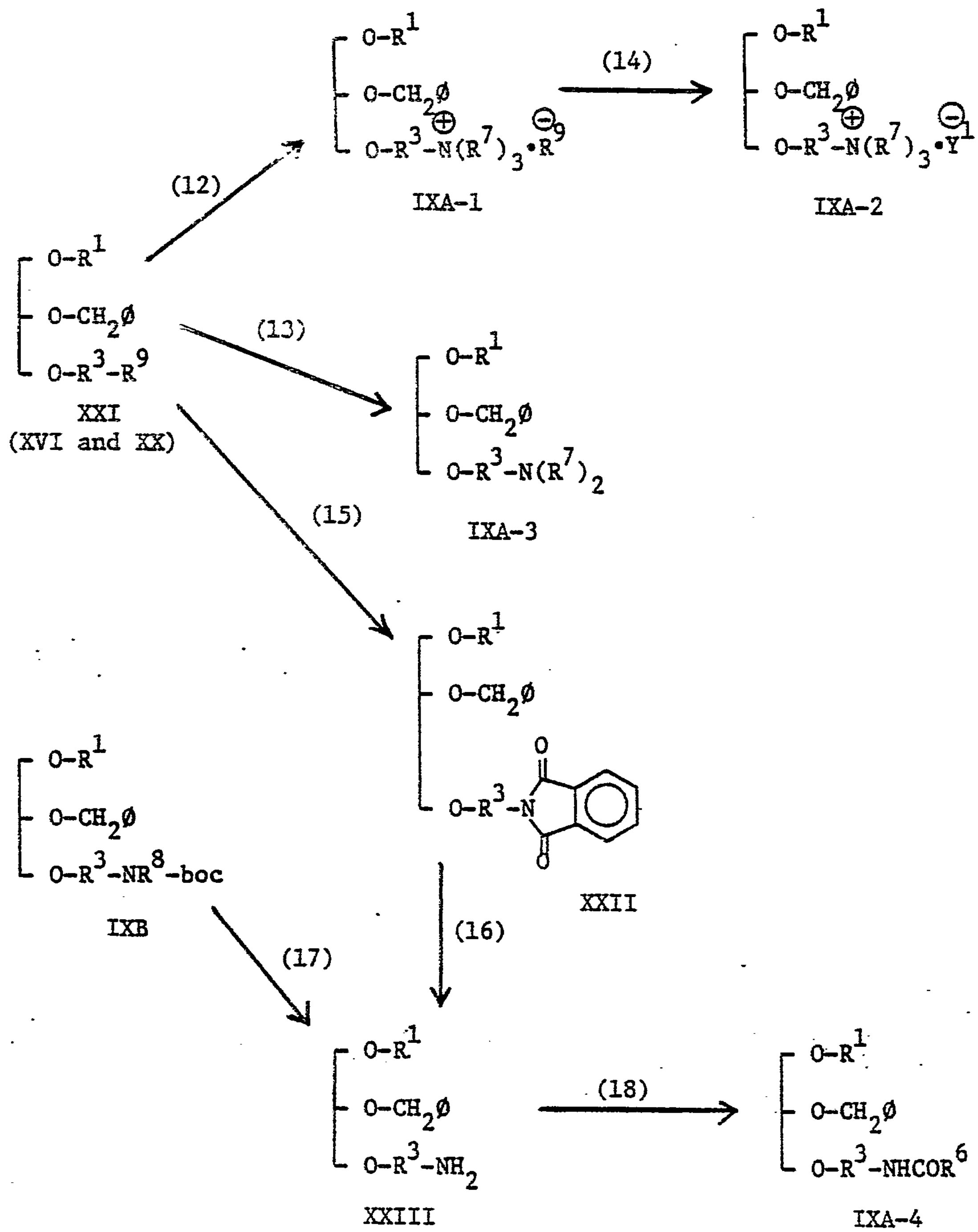


IXA-1 IXA-2 XXI (XVI and XX) IXA-3 XXII IXB XXIII IXA-4

In Scheme A, each step can be effected using methods known per se. For example, step (1) may be carried out by reacting with a benzyl halide such as benzyl chloride in the presence of sodium hydride in an inert organic solvent such as THF or DMF, or a mixture of two or more of them at a temperature from 0°C to 40°C, preferably at ambient.

Step (2) may be carried out by using a mixture of THF, acetic acid and water, a mixture of p-toluenesulfonic acid and anhydrous methanol, or a mixture of diluted hydrochloric acid and THF, at room temperature or with heating.

Step (3) may be conducted by using 2,3-dihydropyran in methylene chloride in the presence of p-toluenesulfonic acid.

Step (4) may be carried out by reacting a compound of the general formula XII or XIII with a compound of the general formula:

$$R^{10}-R^{1} \qquad \text{XXIV}$$

(wherein $R^{10}$ represents a halogen atom or an alkylsulfonyloxy group, having 1 to 4 carbon atoms in the alkyl group, or a substituted or unsubstituted arylsulfonyloxy group, preferably a mesyloxy group or a tosyloxy group and $R^1$ is as hereinbefore defined) in the presence of an alkali metal hydride such as sodium hydride in an inert organic solvent such as DMF or THF at a temperature from ambient to 100°C.

Step (5) may be carried out by methods hereinbefore described for step (2).

Step (6) may be carried out by reacting a compound of the general formula XV with a compound of the general formula:

$R^{10}-R^3-X$ XXV

(the various symbols are as hereinbefore defined) in the presence of an alkali metal hydride such as sodium hydride in an inert organic solvent such as DMF, THF or toluene at a temperature from ambient to 100°C.

Step (7) may be carried out by methods hereinbefore described for step (6) by using a compound of the general formula:

$R^{10}-R^3-NR^8-boc$ XXVI

(wherein the various symbols are as hereinbefore defined) instead of a compound of the general formula XXV.

Step (8) may be carried out by methods hereinbefore described for step (6) by using a compound of the general formula:

$R^{10}-R^3-OTHP$ XXVII

(wherein the various symbols are as hereinbefore defined) instead of a compound of the general formula XXV.

Step (9) may be carried out by methods hereinbefore described for step (6) by using a compound of the general formula:

$$R^{10}-R^{3}-OH \qquad XXVIII$$

(wherein the symbols are as hereinbefore defined) instead of a compound of the general formula XXV.

Step (10) may be carried out by methods hereinbefore described for the conversion of compounds of the general formula VIIIA to those of the general formula IIA.

Step (11) may be carried out by methods hereinbefore described for step (6) by using a compound of the general formula:

$$HO-R^{3}-OH \qquad XXIX$$

(wherein $R^3$ is as hereinbefore defined) instead of a compound of the general formula XXV.

Step (12) and (13) may be carried out by reacting a compound of the general formula XXI with compounds of the general formulae:

$\underset{XXX}{N(R^7)_3}$ and $\underset{XXXI}{HN(R^7)_2}$

(wherein $R^7$ is as hereinbefore defined), respectively, in an inert organic solvent such as a lower alkanol, e.g. methanol, ethanol, isopropyl alcohol, or DMF or a mixture of two or more of than at a temperature from ambient to a reflux temperature of the reaction mixture.

Step (14) may be carried out by using methods for salt-exchange hereinafter described.

Compounds of the general formula IXA-1 wherein $R^9$ represents a halogen atom may also be prepared by reaction of a compound of the general formula IXA-3 with an alkyl halide such as methyl iodide in a lower alkanol such as methanol or ethanol at a temperature from ambient to the reflux temperature of the reaction mixture.

Step (15) may be carried out by using phthalimide in an inert organic solvent such as DMF at a temperature from 50°C to a reflux temperature of the reaction mixture.

Step (16) may be carried out by using hydrazine in a lower alkanol such as ethanol.

Step (17) may be carried out by methods hereinbefore described for the conversion of compounds of the general formula VII to those of the general formula IA-2.

Step (18) may be carried out by using a corresponding acyl chloride, e.g. benzoyl chloride or a corresponding acid anhydride, e.g. acetic anhydride in the presence of a tertiary amine such as pyridine or triethylamine in an inert organic solvent such as methylene chloride or in the absence of a solvent at a temperature below 50°C.

According to a further feature of the present invention, glycerol derivatives of the general formula IA, wherein the various symbols are as hereinbefore defined may also be prepared by the following another methods:

(i) glycerol derivatives of the general formula IA, wherein $R^4$ represents a group of the general formula: $-\overset{\oplus}{N}(R^7)_3 \cdot \overset{\ominus}{Y}$, in which the various symbols are as hereinbefore defined, i.e. compounds of the general formula:

$$\left[\begin{array}{l} O-R^1 \\ R^2 \\ O-R^3-\overset{\oplus}{N}(R^7)_3 \cdot \overset{\ominus}{Y} \end{array}\right. \qquad \text{IA-31}$$

(wherein the various symbols are as hereinbefore defined) may be prepared from a compound of the general formula:

$$\left[\begin{array}{l} O-R^1 \\ R^2 \\ O-R^3-R^9 \end{array}\right. \qquad \text{XXXII}$$

(wherein the various symbols are as hereinbefore defined) by methods hereinbefore described for step (12) and, if desired, step (14) in Scheme A.

(ii) Glycerol derivatives of the general formula IA, wherein $R^4$ represents a group of the general formula: $-N(R^7)_2$, in which $R^7$ is as hereinbefore defined, i.e. compounds of the general formula:

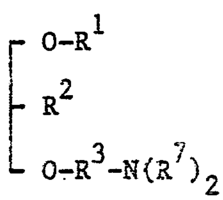

IA-32

(wherein the various symbols are as hereinbefore defined) may be prepared from a compound of the general formula XXXII by methods hereinbefore described for step (13) in Scheme A.

(iii) Glycerol derivatives of the general formula IA, wherein $R^4$ represents a group of the general formula: $-NHR^7$, in which $R^7$ is as hereinbefore defined, i.e. compounds of the general formula:

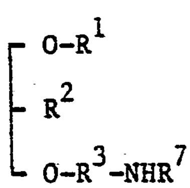

IA-33

(wherein the various symbols are as hereinbefore defined) may be prepared from a compound of the general formula XXXII by methods hereinbefore described for step (12) by using a compound of the general formula:

$H_2NR^7$ XXXIII

(wherein $R^7$ is as hereinbefore defined) instead of a compound of the general formula XXX.

(iv) Glycerol derivatives of the general formula IA, wherein $R^2$ represents an azido group, an amino group or a group of the general formula: $-NHCONHR^5$, in which $R^5$ is as hereinbefore defined, and $R^4$ represents an amino group, i.e. compounds of the general formula:

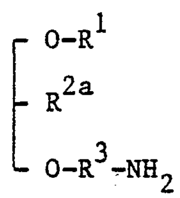

IA-34

(wherein $R^{2a}$ represents an azido group, an amino group or a group of the general formula: $-NHCONHR^5$, in which $R^5$ is as hereinbefore defined, and the other symbols are as hereinbefore defined) may be prepared from a compound of the general formula XXXII, wherein $R^2$ represents a group represented by $R^{2a}$, i.e. a compound of the general formula:

$$\left[\begin{array}{l} O\text{-}R^1 \\ R^{2a} \\ O\text{-}R^3\text{-}R^9 \end{array}\right. \qquad XXXIIA$$

(wherein the various symbols are as hereinbefore defined) by methods hereinbefore described for step (15) and (16) in Scheme A, successively.

(v) Glycerol derivatives of the general formula IA, wherein $R^2$ represents a group of the general formula: $-NHCOR^5$ or $-NHCOOR^5$, in which $R^5$ is as hereinbefore defined, and $R^4$ represents an amino group, i.e. compounds of the general formula:

$$\left[\begin{array}{l} O\text{-}R^1 \\ R^{2b} \\ O\text{-}R^3\text{-}NH_2 \end{array}\right. \qquad IA\text{-}35$$

(wherein $R^{2b}$ represents a group of the general formula: $-NHCOR^5$ or $-NHCOOR^5$, in which $R^5$ is as hereinbefore defined, and the other symbols are as hereinbefore defined) may be prepared from a compound of the general formula XXXII, wherein $R^2$ represents a group represented by $R^{2b}$, and $R^9$ represents a group of the formula: $-OT$, in which T is as hereinbefore defined, i.e. a compound of the general formula:

$$\left[\begin{array}{l} O-R^1 \\ R^{2b} \\ O-R^3-OT \end{array}\right. \qquad \text{XXXIIB}$$

(wherein the various symbols are as hereinbefore defined) by methods hereinbefore described as method A and B, successively.

(vi) Glycerol derivatives of the general formula IA, wherein $R^2$ represents a group other than an amino group and $R^4$ represents a group of the general formula: $-NHCOR^6$, in which $R^6$ is as hereinbefore defined, i.e. compounds of the general formula:

$$\left[\begin{array}{l} O-R^1 \\ R^{2c} \\ O-R^3-NHCOR^6 \end{array}\right. \qquad \text{IA-36}$$

(wherein $R^{2c}$ represents an azido group or a group of the general formula: $-NHCOR^5$, $-NHCOOR^5$ or $-NHCONHR^5$, in which $R^5$ is as hereinbefore defined, and the other symbols are as hereinbefore defined) may be prepared from a compound of the general formula IA-34, wherein $R^{2a}$ represents an azido group or a group of the general formula: $-NHCONHR^5$, or from a compound of the general formula IA-35, i.e. a compound of the general formula:

$$\left[\begin{array}{l} O-R^1 \\ R^{2c} \\ O-R^3-NH_2 \end{array}\right. \qquad \text{IA-37}$$

(wherein the various symbols are as hereinbefore defined) by methods hereinbefore described for step (18) in Scheme A.

(vii) Glycerol derivatives of the general formula IA, wherein $R^2$ represents an amino group and $R^4$ represents a group of the general formula: $-NHCOR^6$, in which $R^6$ is as hereinbefore defined, i.e. compounds of the general formula:

$$\left[\begin{array}{l} O-R^1 \\ NH_2 \\ O-R^3-NHCOR^6 \end{array}\right. \qquad \text{IA-38}$$

(wherein the various symbols are as hereinbefore defined) may be prepared by protection of an amino group of a compound of the general formula XXXII, wherein $R^2$ represents an amino group, i.e. a compound of the general formula:

$$\left[\begin{array}{l} O-R^1 \\ NH_2 \\ O-R^3-R^9 \end{array}\right. \qquad \text{XXXIIC}$$

(wherein the various symbols are as hereinbefore defined) by a

boc group by reacting with di-*tert*-butyldicarbonate in an aqueous solution of sodium hydroxide at room temperature, or in methylene chloride in the presence of a tertiary amine such as triethylamine at room temperature or below, and subjecting the resulting compound of the general formula:

$$\left[\begin{array}{l} O-R^1 \\ NH-boc \\ O-R^3-R^9 \end{array}\right. \qquad \text{XXXIV}$$

(wherein the various symbols are as hereinbefore defined) to the series of methods (15), (16) and (18) in Scheme A, and then eliminating a boc group of the obtained compound of the general formula:

$$\left[\begin{array}{l} O-R^1 \\ NH-boc \\ O-R^3-NHCOR^6 \end{array}\right. \qquad \text{XXXV}$$

(wherein the various symbols are as hereinbefore defined) by methods hereinbefore described for the conversion of compounds of the general formula VII to those of the general formula IA-2 to obtain compounds of the general formula IA-38.

Compounds of the general formula XXXII may be prepared by the series of reactions depicted schematically below in

Scheme B and C, wherein $R^{2d}$ represents an amino group or a group of the general formula: $-NHCOR^5$, $-NHCOOR^5$ or $-NHCONHR^5$, in which $R^5$ is as hereinbefore defined, and the other symbols are as hereinbefore defined.

Scheme B

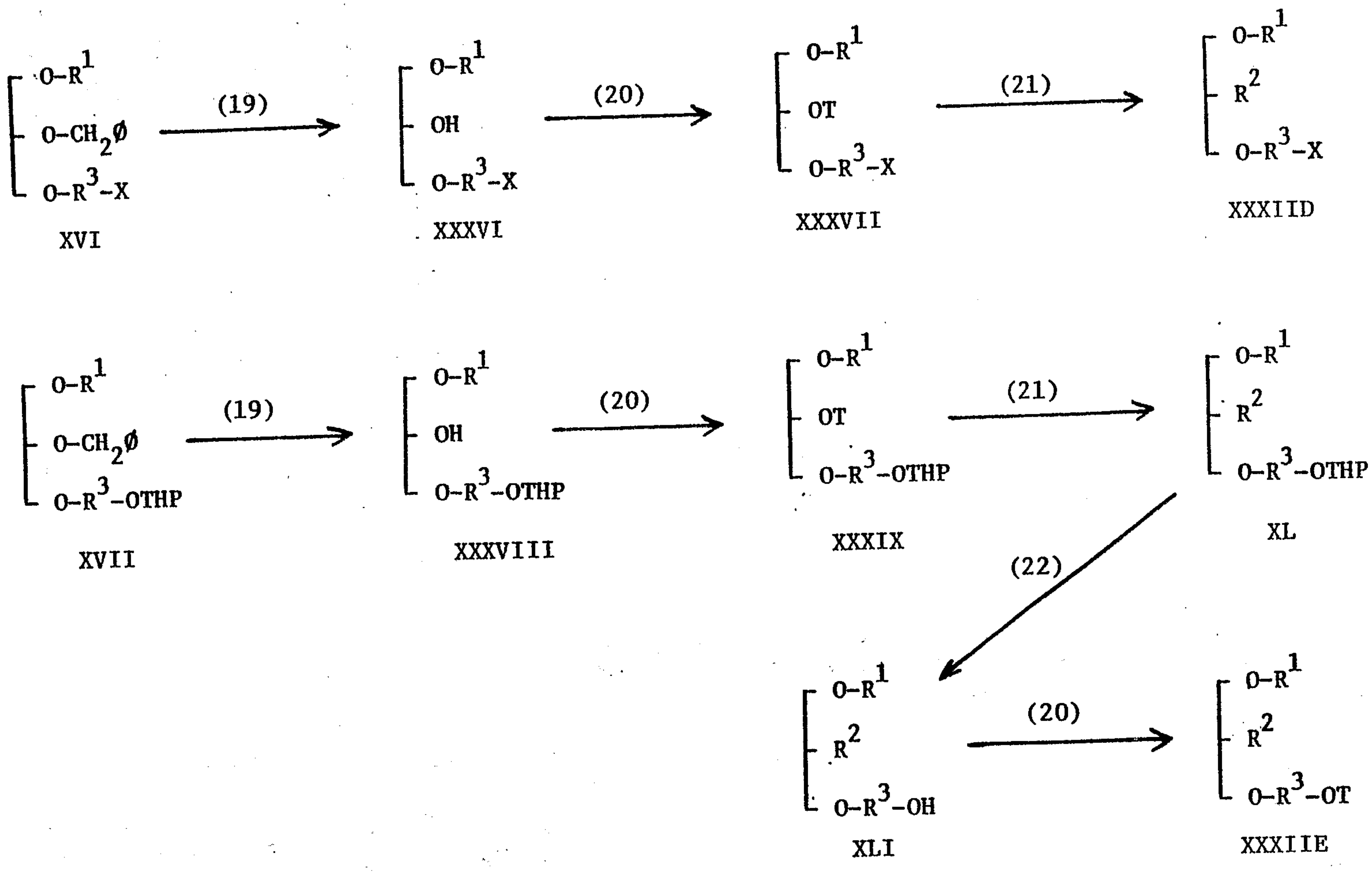


XVI, XXXVI, XXXVII, XXXIID

XVII, XXXVIII, XXXIX, XL

XLI, XXXIIE

Scheme C

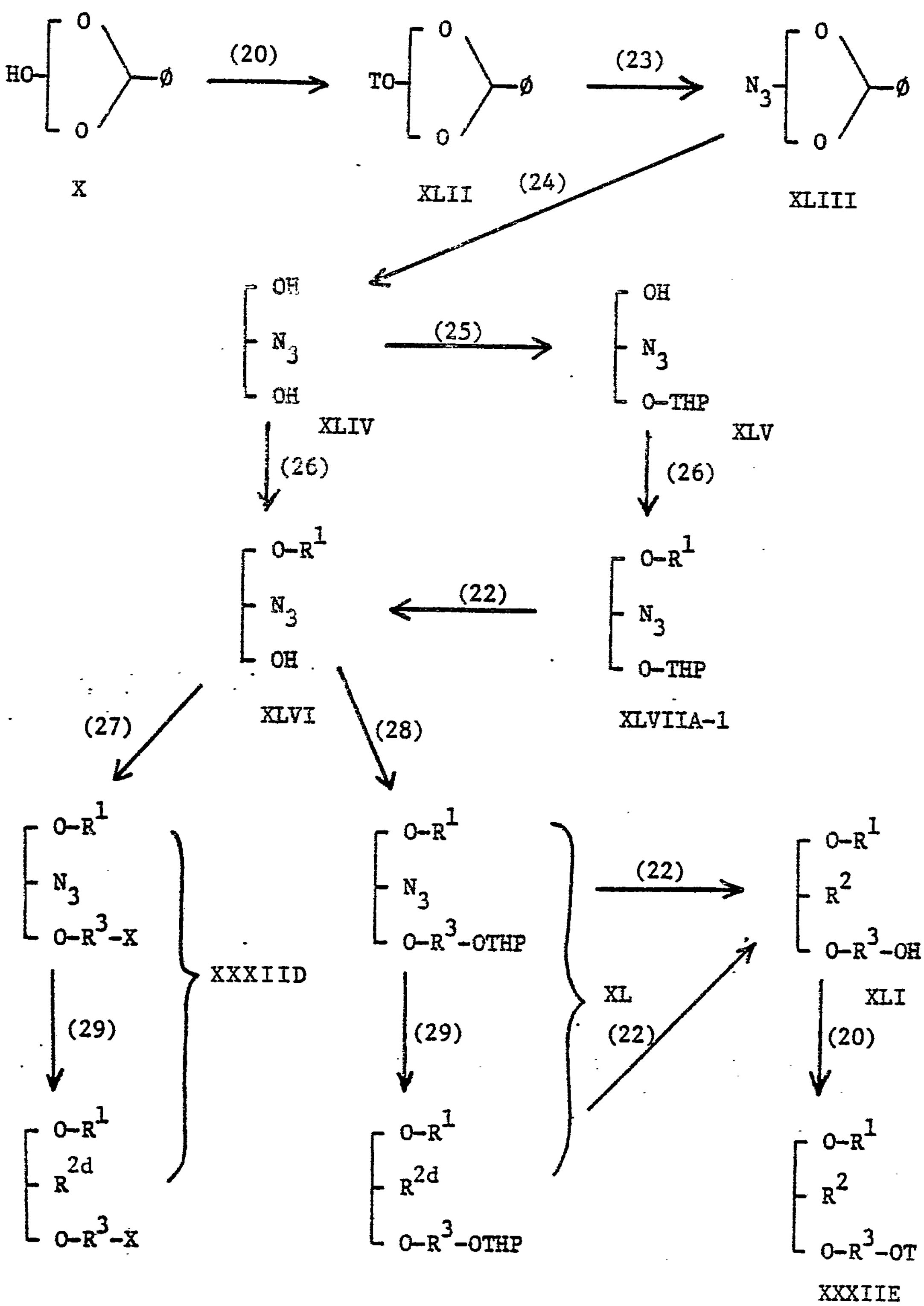
(20)
(23)
(24)
(25)
(26)
(26)
(22)
(27)
(28)
(29)
(29)
(22)
(22)
(20)
X
XLII
XLIII
XLIV
XLV
XLVI
XLVIIA-1
XXXIID
XL
XLI
XXXIIE

In Scheme B and C, each step can be effected using methods hereinbefore described.

Step (19) may be carried out by methods hereinbefore described for the conversion of compounds of the general formula IXA to those of the general formula VIIIA.

Step (20) may be carried out by methods hereinbefore described for the conversion of compounds of the general formula VIIIA to those of the general formula IIA.

Step (21) may be carried out by methods hereinbefore described as method A, B, C, D and E.

Step (22) may be carried out by methods hereinbefore described for step (5) in Scheme A.

Step (23) may be carried out by methods hereinbefore described as method A.

Step (24) may be carried out by methods hereinbefore described for the step (2) in Scheme A.

Step (25) and (26) may be carried out by methods hereinbefore described for step (3) and (4) in Scheme A, respectively.

Step (27) and (28) may be carried out by methods hereinbefore described for step (6) and (8) in Scheme A, respectively.

Step (29) may be carried out by methods hereinbefore described as method B, C, D and E.

According to another feature of the present invention, glycerol derivatives of the general formula I, wherein A represents a carbonyloxy group, $R^2$ represents a group other than an amino group and the other symbols are as hereinbefore defined, i.e. compounds of the general formula:

$$\begin{array}{l} -O-R^1 \\ -R^{2c} \\ -O-CO-R^3-R^4 \end{array} \qquad IB$$

(wherein the various symbols are as hereinbefore defined) may be prepared by the following methods:

(i) glycerol derivatives of the general formula IB, wherein $R^4$ represents a group of the general formula: $-\overset{\oplus}{N}(R^7)_3\cdot\overset{\ominus}{Y}$, in which the various symbols are as hereinbefore defined, i.e. compounds of the general formula:

$$\begin{array}{l} -O-R^1 \\ -R^{2c} \\ -O-CO-R^3-\overset{\oplus}{N}(R^7)_3\cdot\overset{\ominus}{Y} \end{array} \qquad IB\text{-}1$$

(wherein the various symbols are as hereinbefore defined) may be prepared from a compound of the general formula:

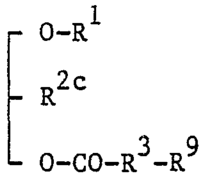

XLVIII

(wherein the various symbols are as hereinbefore defined) by methods hereinbefore described for the step (12) and, if desired, step (14) in Scheme A.

Compounds of the general formula XLVIII, wherein $R^9$ represents a halogen atom, i.e. compounds of the general formula:

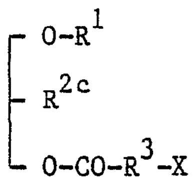

XLVIIIA

(wherein the various symbols are as hereinbefore defined) may be prepared by esterification of a compound of the general formula:

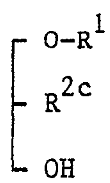

XLIX

(wherein the various symbols are as hereinbefore defined) with an acid of the general formula:

HOOC-$R^3$-X L

(wherein the various symbols are as hereinbefore defined) or a reactive derivative thereof. The esterification may be carried out in an inert organic solvent such as ethyl acetate, benzene, diethyl ether, chloroform, methylene chloride, THF or a mixture of two or more of them at a temperature from -10°C to 50°C (preferably at a temperature from ambient to 0°C). Examples of the reactive derivatives of an acid of the general formula L are an acid halide such as acyl chloride or acyl bromide, a mixed acid anhydride with pivaloyl chloride, isobutyryl chloride or isobutyl chloroformate, or an active ester with dicyclohexylcarbodiimide. Preferably, the reactive derivatives of an acid of the general formula L may be prepared in the presence of a base such as pyridine or triethylamine in a suitable inert organic solvent at 0°C.

Compounds of the general formula XLVIII, wherein $R^9$ represents an alkylsulfonyl group or an arylsulfonyl group, i.e. compounds of the general formula:

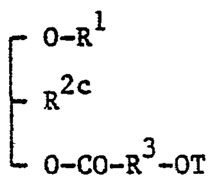

XLVIIIB

(wherein the various symbols are as hereinbefore defined) may be

prepared by esterification of a compound of the general formula XLIX with an acid of the general formula:

$$HOOC-R^3-OTHP \qquad LI$$

(wherein the various symbols are as hereinbefore defined) or a reactive derivative thereof by methods hereinbefore described for esterification, and then subjecting the resulting compound of the general formula:

$O-R^1$
$R^{2c}$ LII
$O-CO-R^3-OTHP$

(wherein the various symbols are as hereinbefore defined) to methods hereinbefore described for step (5) and (10) in Scheme A to obtain a compound of the general formula XLVIIIB.

(ii) Glycerol derivatives of the general formula IB, wherein $R^4$ represents a group of the general formula: $-N(R^7)_2$, in which $R^7$ is as hereinbefore defined, i.e. compounds of the general formula:

$O-R^1$
$R^{2c}$ IB-2
$O-CO-R^3-N(R^7)_2$

(wherein the various symbols are as hereinbefore defined) may be prepared by esterification of a compound of the general formula XLIX with an acid of the general formula:

$$HOOC-R^3-N(R^7)_2 \qquad LIII$$

(wherein the various symbols are as hereinbefore defined) or a reactive derivative thereof by methods hereinbefore described for esterification.

(iii) Glycerol derivatives of the general formula IB, wherein $R^4$ represents an amino group or a group of the general formula: $-NHR^7$, in which $R^7$ is as hereinbefore defined, i.e. compounds of the general formula:

$$\begin{array}{l} \ulcorner O-R^1 \\ \vdash R^{2c} \\ \llcorner O-CO-R^3-NHR^8 \end{array} \qquad IB-3$$

(wherein the various symbols are as hereinbefore defined) may be prepared by esterification of a compound of the general formula XLIX with an acid of the general formula:

$$HOOC-R^3-NR^8-boc \qquad LIV$$

(wherein the various symbols are as hereinbefore defined) or a reactive derivative thereof by methods hereinbefore described for esterification, and then eliminating a boc group of the resulting compound of the general formula:

$$\left[\begin{array}{l} O-R^{1} \\ R^{2c} \\ O-CO-R^{3}-NR^{8}-boc \end{array}\right. \qquad LV$$

(wherein the various symbols are as hereinbefore defined) by methods hereinbefore described for the conversion of compounds of the general formula VII to those of the general formula IA-2.

(iv) Glycerol derivatives of the general formula IB, wherein $R^4$ represents a group of the general formula: $-NHCOR^6$, in which $R^6$ is as hereinbefore defined, i.e. compounds of the general formula:

$$\left[\begin{array}{l} O-R^{1} \\ R^{2c} \\ O-CO-R^{3}-NHCOR^{6} \end{array}\right. \qquad IB-4$$

(wherein the various symbols are as hereinbefore defined) may be prepared by acylation of a compound of the general formula IB-3,

wherein $R^8$ represents a hydrogen atom, i.e. a compound of the general formula:

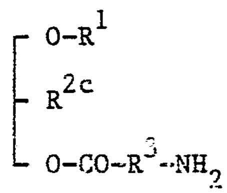

IB-31

(wherein the various symbols are as hereinbefore defined) by method hereinbefore described for step (18) in Scheme A.

Compounds of the general formula XLIX may be prepared by the series of reactions depicted schematically below in Scheme D, wherein the various symbols are as hereinbefore defined.

Scheme D

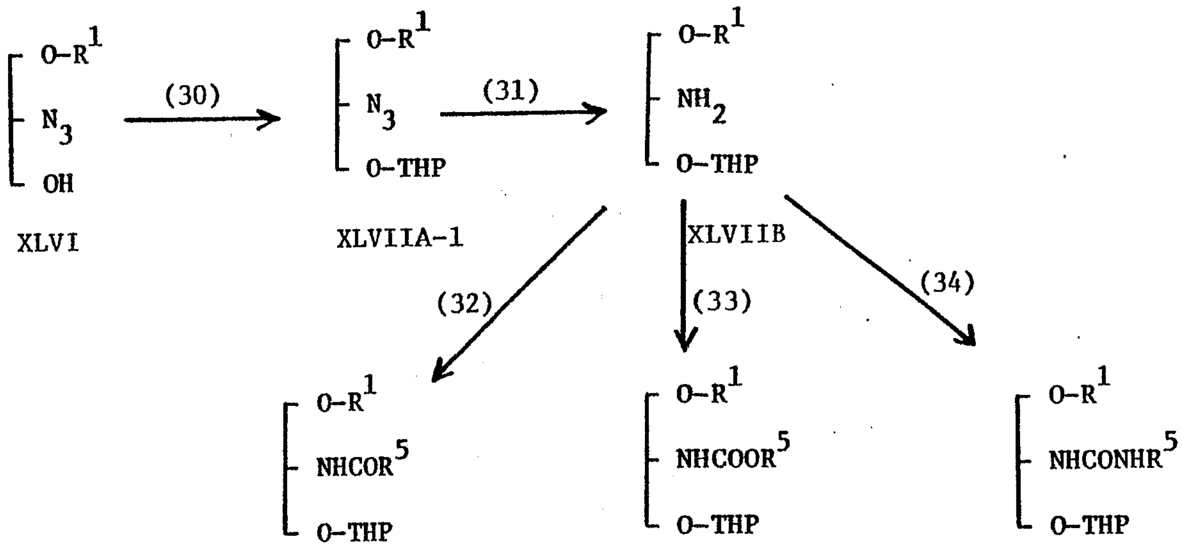


XLVI XLVIIA-1 XLVIIB

XLVIIA-2 XLVIIA-3 XLVIIA-4

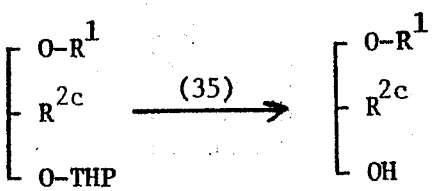


XLVIIA XLIX

In Scheme D, each step can be effected using methods hereinbefore described.

Step (30) can be carried out by methods hereinbefore described for step (3) in Scheme A.

Step (31), (32), (33) and (34) may be carried out by methods hereinbefore described as method B, C, D and E, respectively.

Step (35) may be carried out by methods hereinbefore described for step (5) in Scheme A.

Compounds of the general formulae III to VI, XXIV to XXXI, XXXIII, L, LI, LIII and LIV are well known <u>per se</u> or may be prepared by methods known <u>per se</u>.

Glycerol 1,3-benzylidene ether of the formula X, employed as the starting material is a known compound described in J. Amer. Chem. Soc., <u>50</u>, 2235 (1928).

Glycerol derivatives of the general formula I, wherein $R^2$ represents a group other than an amino group and $R^4$ represents a group of general formula: $-\overset{\oplus}{N}(R^7)_3 \cdot \overset{\ominus}{Y}$, in which Y represents a halogen atom, i.e. compounds of the general formula:

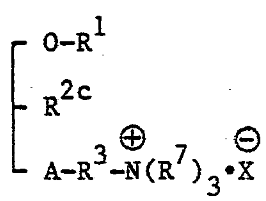

IC

(wherein the various symbols are as hereinbefore defined) may also be prepared by reaction of a compound of the general formula:

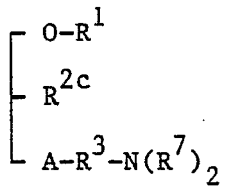

ID

(wherein the various symbols are as hereinbefore defined) with an alkyl halide such as methyl iodide in a lower alkanol such as methanol or ethanol at a temperature from ambient to the reflux temperature of the reaction mixture.

Glycerol derivatives of the general formula I, wherein $R^4$ represents a group of the general formula: $-\overset{\oplus}{N}(R^7)_3 \cdot \overset{\ominus}{Y}$, in which Y represents a group other than a halogen atom and other than a OT group, i.e. compounds of the general formula I, wherein $R^4$ represents a group of the general formula: $-\overset{\oplus}{N}(R^7)_3 \cdot \overset{\ominus}{Y^1}$, in the various symbols are as hereinbefore defined may be easily prepared from a glycerol derivative of the general formula I, wherein $R^4$ represents a group of the general formula: $-\overset{\oplus}{N}(R^7)_3 \cdot \overset{\ominus}{R^9}$, in which the various symbols are as hereinbefore defined, by salt-exchange, for example, by neutralizing of a compound of the general formula I, wherein $R^4$ represents a group of the general formula: $-\overset{\oplus}{N}(R^7)_3 \cdot \overset{\ominus}{R^9}$ with an aqueous solution of

sodium hydroxide, and then reacting with a desirable inorganic or organic acid, or by ion-exchange resin.

Glycerol derivatives of the general formula I, wherein $R^2$ represents an amino group or $R^4$ represents an amino group or a group of the general formula: $-NHR^7$ or $-N(R^7)_2$ may be converted into acid addition salts thereof. Preferably, acid addition salts are non-toxic salts and are water-soluble. Suitable acid addition salts are, for example, inorganic acid salts such as hydrochloride, hydrobromide, hydroiodide, sulfate, phosphorate, nitrate, or organic acid salts such as acetate, lactate, tartrate, benzoate citrate, methanesulphonate, ethanesulphonate, benzenesulphonate, toluenesulphonate or isethionate. Acid addition salts may be prepared by methods known per se, for example, reacting of stoichiometric quantities of a compound of the general formula I, wherein $R^2$ represents an amino group or $R^4$ represents an amino group or a group of the general formula: $-NHR^7$ or $-N(R^7)_2$ and a desirable acid in a suitable solvent.

Glycerol derivatives of the general formula I and acid addition salts thereof have antagonistic effect on PAF, hypotensive effect like PAF, inhibitory effect on phospholipase and further, inhibitory effect on growth of tumour cell or induction of differentiation, and are, therefore, useful as a platelet aggregation inhibitor, anti-asthmatic agent, anti-allergic agent, anti-inflammatory agent, hypotensive agent

and anti-tumour agent for mammals including human beings, especially human beings. These effect were confirmed by standard laboratory test and, for example, antagonistic effect on PAF was confirmed by the following screening system.

Inhibitory effect on PAF-induced platelet aggregation

A mixture of 9 parts of blood obtained from male guinea-pig, and one part of 3.8% trisodium citrate was centrifuged for 10 minutes at 120 x g at room temperature to obtain platelet rich plasma (PRP). Using obtained PRP, PAF (10nM)-induced aggregation was determined by the method of Born [cf. J. Physiol., 162, 67 (1962)].

The evaluation is conducted using the concentration of compounds of the present invention required to produce a 50% inhibition of the effect, i.e. $IC_{50}$. The results are shown in the following table.

| Example No. of tested compounds | Inhibitory effect on PAF-induced platelet aggregation ($IC_{50}$, M) |
|---|---|
| 2 (a) | $4.3 \times 10^{-6}$ |
| 3 (a) | $1.3 \times 10^{-5}$ |
| 8 | $3.8 \times 10^{-6}$ |
| 8 (j) | $1.9 \times 10^{-6}$ |
| 8 (r) | $3.5 \times 10^{-6}$ |
| 7 (b) | $9.8 \times 10^{-6}$ |
| 10 (j) | $1.90 \times 10^{-6}$ |

On the other hand, it was confirmed that the acute toxicity of all extent of the compounds of the present invention was more than 30 mg/kg by intravenous administration. Therefore, glycerol derivatives of the present invention may be considered to be sufficiently safe and suitable for medical use. For example, the mortality (dead/used) in glycerol derivatives of the present invention, i.e. compounds prepared in Example 8, 8(j) and 8(r) was 2/10, 0/10 and 0/10, respectively, when each glycerol compound was intravenously administered to tail vein in ten mice at the dose of 30 mg/kg.

For the purpose hereinbefore described, glycerol derivatives of the general formula I, or non-toxic acid addition salt thereof may normally be administered systemically or partially usually by oral or parenteral (e.g. intravenous, subcutaneous or intramuscular) administration. The dose to be administered is determined depending upon, for example, age, body weight, symptoms, the desired therapeutic effect, the route of administration, and the duration of the treatment.

In the human adult, the doses per person are generally between 1 mg and 1 g, preferably between 20 and 200 mg by oral administration, and between 100 μg and 100 mg, preferably between 1 and 10 mg by parenteral administration and can be administered up to several times per day.

As mentioned above, the doses to be used depend on various conditions. Therefore, there may be cases in which doses greater than the ranges specified above, or lower than the ranges specified above, may be used.

The present invention includes within its scope pharmaceutical compositions which comprise at least one glycerol derivatives of the general formula I, or non-toxic acid addition salt thereof, together with a pharmaceutical carrier or coating.

Solid compositions according to the present invention for oral administration include compressed tablets, pills, dispersible powders and granules. In such solid compositions, one or more of the active compound(s) is, or are, admixed with

at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, and, disintegrating agents such as cellulose calcium gluconate. The tablets or pills may, if desired, be made into enteric film-coated or gastric film-coated tablets or pills, such as sugar-coated, gelatin-coated, hydroxypropylcellulose-coated or hydroxypropylmethylcellulose phthalate-coated tablets or pills; two or more layers may be used.

The compositions for oral administration also include capsules of absorbable material such as gelatin, containing one or more of the active substances with or without the addition of diluents or excipients.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise at least one compound of the present invention.

Preparations according to the present invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, Polysorbate 80 (registered Trade Mark). These compositions may also include ajuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration. Such compositions are prepared by known methods.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when required for administration by injection; for oral administration the preparations will normally contain at least 0.1% by weight of active substance.

Preferred glycerol derivatives of the present invention are as follows:

(2RS)-1-O-hexadecyl-2-dehydroxy-2-azido-3-O-[4-(N,N,N-trimethylammonio)butyl]glycerol•halide or its mesylate,

(2RS)-1-O-hexadecyl-2-dehydroxy-2-azido-3-O-[5-(N,N,N-trimethylammonio)pentyl]glycerol•halide or its mesylate,

(2RS)-1-O-hexadecyl-2-dehydroxy-2-azido-3-O-[6-(N,N,N-trimethylammonio)hexyl]glycerol•halide or its mesylate,

(2RS)-1-O-hexadecyl-2-dehydroxy-2-azido-3-O-[7-(N,N,N-trimethylammonio)heptyl]glycerol•halide or its mesylate,

(2RS)-1-O-hexadecyl-2-dehydroxy-2-azido-3-O-[8-(N,N,N-trimethylammonio)octyl]glycerol•halide or its mesylate,

(2RS)-1-O-hexadecyl-2-dehydroxy-2-amino-3-O-[4-(N,N,N-trimethylammonio)butyl]glycerol•halide or its mesylate,

(2RS)-1-O-hexadecyl-2-dehydroxy-2-amino-3-O-[5-(N,N,N-trimethyl-

ammonio)pentyl]glycerol•halide or its mesylate,

(2RS)-1-O-hexadecyl-2-dehydroxy-2-amino-3-O-[6-(N,N,N-trimethylammonio)hexyl]glycerol•halide or its mesylate,

(2RS)-1-O-hexadecyl-2-dehydroxy-2-amino-3-O-[7-(N,N,N-trimethylammonio)heptyl]glycerol•halide or its mesylate,

(2RS)-1-O-hexadecyl-2-dehydroxy-2-amino-3-O-[8-(N,N,N-trimethylammonio)octyl]glycerol•halide or its mesylate,

(2RS)-1-O-hexadecyl-2-dehydroxy-2-benzoylamino-3-O-[4-(N,N,N-trimethylammonio)butyl]glycerol•halide or its mesylate,

(2RS)-1-O-hexadecyl-2-dehydroxy-2-benzoylamino-3-O-[5-(N,N,N-trimethylammonio)pentyl]glycerol•halide or its mesylate,

(2RS)-1-O-hexadecyl-2-dehydroxy-2-benzoylamino-3-O-[6-(N,N,N-trimethylammonio)hexyl]glycerol•halide or its mesylate,

(2RS)-1-O-hexadecyl-2-dehydroxy-2-benzoylamino-3-O-[7-(N,N,N-trimethylammonio)heptyl]glycerol•halide or its mesylate,

(2RS)-1-O-hexadecyl-2-dehydroxy-2-benzoylamino-3-O-[8-(N,N,N-trimethylammonio)octyl]glycerol•halide or its mesylate,

(2RS)-1-O-hexadecyl-2-dehydroxy-2-acetylamino-3-O-[4-(N,N,N-trimethylammonio)butyl]glycerol•halide or its mesylate,

(2RS)-1-O-hexadecyl-2-dehydroxy-2-acetylamino-3-O-[5-(N,N,N-trimethylammonio)pentyl]glycerol•halide or its mesylate,

(2RS)-1-O-hexadecyl-2-dehydroxy-2-acetylamino-3-O-[6-(N,N,N-trimethylammonio)hexyl]glycerol•halide or its mesylate,

(2RS)-1-O-hexadecyl-2-dehydroxy-2-acetylamino-3-O-[7-(N,N,N-trimethylammonio)heptyl]glycerol•halide or its mesylate,

(2RS)-1-O-hexadecyl-2-dehydroxy-2-acetylamino-3-O-[8-(N,N,N-trimethylammonio)octyl]glycerol•halide or its mesylate,

(2RS)-1-O-hexadecyl-2-dehydroxy-2-propionylamino-3-O-[4-(N,N,N-trimethylammonio)butyl]glycerol•halide or its mesylate,

(2RS)-1-O-hexadecyl-2-dehydroxy-2-propionylamino-3-O-[5-(N,N,N-trimethylammonio)pentyl]glycerol•halide or its mesylate,

(2RS)-1-O-hexadecyl-2-dehydroxy-2-propionylamino-3-O-[6-(N,N,N-trimethylammonio)hexyl]glycerol•halide or its mesylate,

(2RS)-1-O-hexadecyl-2-dehydroxy-2-propionylamino-3-O-[7-(N,N,N-trimethylammonio)heptyl]glycerol•halide or its mesylate,

(2RS)-1-O-hexadecyl-2-dehydroxy-2-propionylamino-3-O-[8-(N,N,N-trimethylammonio)octyl]glycerol•halide or its mesylate,

(2RS)-1-O-hexadecyl-2-dehydroxy-2-butyrylamino-3-O-[4-(N,N,N-trimethylammonio)butyl]glycerol•halide or its mesylate,

(2RS)-1-O-hexadecyl-2-dehydroxy-2-butyrylamino-3-O-[5-(N,N,N-trimethylammonio)pentyl]glycerol•halide or its mesylate,

(2RS)-1-O-hexadecyl-2-dehydroxy-2-butyrylamino-3-O-[6-(N,N,N-trimethylammonio)hexyl]glycerol•halide or its mesylate,

(2RS)-1-O-hexadecyl-2-dehydroxy-2-butyrylamino-3-O-[7-(N,N,N-trimethylammonio)heptyl]glycerol•halide or its mesylate,

(2RS)-1-O-hexadecyl-2-dehydroxy-2-butyrylamino-3-O-[8-(N,N,N-trimethylammonio)octyl]glycerol•halide or its mesylate,

(2RS)-1-O-hexadecyl-2-dehydroxy-2-valerylamino-3-O-[4-(N,N,N-trimethylammonio)butyl]glycerol•halide or its mesylate,

(2RS)-1-O-hexadecyl-2-dehydroxy-2-valerylamino-3-O-[5-(N,N,N-trimethylammonio)pentyl]glycerol•halide or its mesylate,
(2RS)-1-O-hexadecyl-2-dehydroxy-2-valerylamino-3-O-[6-(N,N,N-trimethylammonio)hexyl]glycerol•halide or its mesylate,
(2RS)-1-O-hexadecyl-2-dehydroxy-2-valerylamino-3-O-[7-(N,N,N-trimethylammonio)heptyl]glycerol•halide or its mesylate,
(2RS)-1-O-hexadecyl-2-dehydroxy-2-valerylamino-3-O-[8-(N,N,N-trimethylammonio)octyl]glycerol•halide or its mesylate,
(2RS)-1-O-hexadecyl-2-dehydroxy-2-hexanoylamino-3-O-[4-(N,N,N-trimethylammonio)butyl]glycerol•halide or its mesylate,
(2RS)-1-O-hexadecyl-2-dehydroxy-2-hexanoylamino-3-O-[5-(N,N,N-trimethylammonio)pentyl]glycerol•halide or its mesylate,
(2RS)-1-O-hexadecyl-2-dehydroxy-2-hexanoylamino-3-O-[6-(N,N,N-trimethylammonio)hexyl]glycerol•halide or its mesylate,
(2RS)-1-O-hexadecyl-2-dehydroxy-2-hexanoylamino-3-O-[7-(N,N,N-trimethylammonio)heptyl]glycerol•halide or its mesylate,
(2RS)-1-O-hexadecyl-2-dehydroxy-2-hexanoylamino-3-O-[8-(N,N,N-trimethylammonio)octyl]glycerol•halide or its mesylate,
(2RS)-1-O-hexadecyl-2-dehydroxy-2-phenoxycarbonylamino-3-O-[4-(N,N,N-trimethylammonio)butyl]glycerol•halide or its mesylate,
(2RS)-1-O-hexadecyl-2-dehydroxy-2-phenoxycarbonylamino-3-O-[5-(N,N,N-trimethylammonio)pentyl]glycerol•halide or its mesylate,
(2RS)-1-O-hexadecyl-2-dehydroxy-2-phenoxycarbonylamino-3-O-[6-(N,N,N-trimethylammonio)hexyl]glycerol•halide or its mesylate,
(2RS)-1-O-hexadecyl-2-dehydroxy-2-phenoxycarbonylamino-3-O-[7-(N,N,N-trimethylammonio)heptyl]glycerol•halide or its mesylate,

(2RS)-1-O-hexadecyl-2-dehydroxy-2-phenoxycarbonylamino-3-O-[8-(N,N,N-trimethylammonio)octyl]glycerol•halide or its mesylate,

(2RS)-1-O-hexadecyl-2-dehydroxy-2-methoxycarbonylamino-3-O-[4-(N,N,N-trimethylammonio)butyl]glycerol•halide or its mesylate,

(2RS)-1-O-hexadecyl-2-dehydroxy-2-methoxycarbonylamino-3-O-[5-(N,N,N-trimethylammonio)pentyl]glycerol•halide or its mesylate,

(2RS)-1-O-hexadecyl-2-dehydroxy-2-methoxycarbonylamino-3-O-[6-(N,N,N-trimethylammonio)hexyl]glycerol•halide or its mesylate,

(2RS)-1-O-hexadecyl-2-dehydroxy-2-methoxycarbonylamino-3-O-[7-(N,N,N-trimethylammonio)heptyl]glycerol•halide or its mesylate,

(2RS)-1-O-hexadecyl-2-dehydroxy-2-methoxycarbonylamino-3-O-[8-(N,N,N-trimethylammonio)octyl]glycerol•halide or its mesylate,

(2RS)-1-O-hexadecyl-2-dehydroxy-2-ethoxycarbonylamino-3-O-[4-(N,N,N-trimethylammonio)butyl]glycerol•halide or its mesylate,

(2RS)-1-O-hexadecyl-2-dehydroxy-2-ethoxycarbonylamino-3-O-[5-(N,N,N-trimethylammonio)pentyl]glycerol•halide or its mesylate,

(2RS)-1-O-hexadecyl-2-dehydroxy-2-ethoxycarbonylamino-3-O-[6-(N,N,N-trimethylammonio)hexyl]glycerol•halide or its mesylate,

(2RS)-1-O-hexadecyl-2-dehydroxy-2-ethoxycarbonylamino-3-O-[7-(N,N,N-trimethylammonio)heptyl]glycerol•halide or its mesylate,

(2RS)-1-O-hexadecyl-2-dehydroxy-2-ethoxycarbonylamino-3-O-[8-(N,N,N-trimethylammonio)octyl]glycerol•halide or its mesylate,

(2RS)-1-O-hexadecyl-2-dehydroxy-2-propoxycarbonylamino-3-O-[4-(N,N,N-trimethylammonio)butyl]glycerol•halide or its mesylate,

(2RS)-1-O-hexadecyl-2-dehydroxy-2-propoxycarbonylamino-3-O-[5-

(N,N,N-trimethylammonio)pentyl]glycerol halide or its mesylate,

(2RS)-1-O-hexadecyl-2-dehydroxy-2-propoxycarbonylamino-3-O-[6-(N,N,N-trimethylammonio)hexyl]glycerol halide or its mesylate,

(2RS)-1-O-hexadecyl-2-dehydroxy-2-propoxycarbonylamino-3-O-[7-(N,N,N-trimethylammonio)heptyl]glycerol halide or its mesylate,

(2RS)-1-O-hexadecyl-2-dehydroxy-2-propoxycarbonylamino-3-O-[8-(N,N,N-trimethylammonio)octyl]glycerol halide or its mesylate,

(2RS)-1-O-hexadecyl-2-dehydroxy-2-butoxycarbonylamino-3-O-[4-(N,N,N-trimethylammonio)butyl]glycerol halide or its mesylate,

(2RS)-1-O-hexadecyl-2-dehydroxy-2-butoxycarbonylamino-3-O-[5-(N,N,N-trimethylammonio)pentyl]glycerol halide or its mesylate,

(2RS)-1-O-hexadecyl-2-dehydroxy-2-butoxycarbonylamino-3-O-[6-(N,N,N-trimethylammonio)hexyl]glycerol halide or its mesylate,

(2RS)-1-O-hexadecyl-2-dehydroxy-2-butoxycarbonylamino-3-O-[7-(N,N,N-trimethylammonio)heptyl]glycerol halide or its mesylate,

(2RS)-1-O-hexadecyl-2-dehydroxy-2-butoxycarbonylamino-3-O-[8-(N,N,N-trimethylammonio)octyl]glycerol halide or its mesylate,

(2RS)-1-O-hexadecyl-2-dehydroxy-2-isobutoxycarbonylamino-3-O-[4-(N,N,N-trimethylammonio)butyl]glycerol halide or its mesylate,

(2RS)-1-O-hexadecyl-2-dehydroxy-2-isobutoxycarbonylamino-3-O-[5-(N,N,N-trimethylammonio)pentyl]glycerol halide or its mesylate,

(2RS)-1-O-hexadecyl-2-dehydroxy-2-isobutoxycarbonylamino-3-O-[6-(N,N,N-trimethylammonio)hexyl]glycerol halide or its mesylate,

(2RS)-1-O-hexadecyl-2-dehydroxy-2-isobutoxycarbonylamino-3-O-[7-(N,N,N-trimethylammonio)heptyl]glycerol halide or its mesylate,

(2RS)-1-O-hexadecyl-2-dehydroxy-2-isobutoxycarbonylamino-3-O-[8-(N,N,N-trimethylammonio)octyl]glycerol•halide or its mesylate,

(2RS)-1-O-hexadecyl-2-dehydroxy-2-pentyloxycarbonylamino-3-O-[4-(N,N,N-trimethylammonio)butyl]glycerol•halide or its mesylate,

(2RS)-1-O-hexadecyl-2-dehydroxy-2-pentyloxycarbonylamino-3-O-[5-(N,N,N-trimethylammonio)pentyl]glycerol•halide or its mesylate,

(2RS)-1-O-hexadecyl-2-dehydroxy-2-pentyloxycarbonylamino-3-O-[6-(N,N,N-trimethylammonio)hexyl]glycerol•halide or its mesylate,

(2RS)-1-O-hexadecyl-2-dehydroxy-2-pentyloxycarbonylamino-3-O-[7-(N,N,N-trimethylammonio)heptyl]glycerol•halide or its mesylate,

(2RS)-1-O-hexadecyl-2-dehydroxy-2-pentyloxycarbonylamino-3-O-[8-(N,N,N-trimethylammonio)octyl]glycerol•halide or its mesylate,

(2RS)-1-O-hexadecyl-2-dehydroxy-2-(N'-phenylureido)-3-O-[4-(N,N,N-trimethylammonio)butyl]glycerol•halide or its mesylate,

(2RS)-1-O-hexadecyl-2-dehydroxy-2-(N'-phenylureido)-3-O-[5-(N,N,N-trimethylammonio)pentyl]glycerol•halide or its mesylate,

(2RS)-1-O-hexadecyl-2-dehydroxy-2-(N'-phenylureido)-3-O-[6-(N,N,N-trimethylammonio)hexyl]glycerol•halide or its mesylate,

(2RS)-1-O-hexadecyl-2-dehydroxy-2-(N'-phenylureido)-3-O-[7-(N,N,N-trimethylammonio)heptyl]glycerol•halide or its mesylate,

(2RS)-1-O-hexadecyl-2-dehydroxy-2-(N'-phenylureido)-3-O-[8-(N,N,N-trimethylammonio)octyl]glycerol•halide or its mesylate,

(2RS)-1-O-hexadecyl-2-dehydroxy-2-(N'-methylureido)-3-O-[4-(N,N,N-trimethylammonio)butyl]glycerol•halide or its mesylate,

(2RS)-1-O-hexadecyl-2-dehydroxy-2-(N'-methylureido)-3-O-[5-

(N,N,N-trimethylammonio)pentyl]glycerol•halide or its mesylate,

(2RS)-1-O-hexadecyl-2-dehydroxy-2-(N'-methylureido)-3-O-[6-(N,N,N-trimethylammonio)hexyl]glycerol•halide or its mesylate,

(2RS)-1-O-hexadecyl-2-dehydroxy-2-(N'-methylureido)-3-O-[7-(N,N,N-trimethylammonio)heptyl]glycerol•halide or its mesylate,

(2RS)-1-O-hexadecyl-2-dehydroxy-2-(N'-methylureido)-3-O-[8-(N,N,N-trimethylammonio)octyl]glycerol•halide or its mesylate,

(2RS)-1-O-hexadecyl-2-dehydroxy-2-(N'-ethylureido)-3-O-[4-(N,N,N-trimethylammonio)butyl]glycerol•halide or its mesylate,

(2RS)-1-O-hexadecyl-2-dehydroxy-2-(N'-ethylureido)-3-O-[5-(N,N,N-trimethylammonio)pentyl]glycerol•halide or its mesylate,

(2RS)-1-O-hexadecyl-2-dehydroxy-2-(N'-ethylureido)-3-O-[6-(N,N,N-trimethylammonio)hexyl]glycerol•halide or its mesylate,

(2RS)-1-O-hexadecyl-2-dehydroxy-2-(N'-ethylureido)-3-O-[7-(N,N,N-trimethylammonio)heptyl]glycerol•halide or its mesylate,

(2RS)-1-O-hexadecyl-2-dehydroxy-2-(N'-ethylureido)-3-O-[8-(N,N,N-trimethylammonio)octyl]glycerol•halide or its mesylate,

(2RS)-1-O-hexadecyl-2-dehydroxy-2-(N'-propylureido)-3-O-[4-(N,N,N-trimethylammonio)butyl]glycerol•halide or its mesylate,

(2RS)-1-O-hexadecyl-2-dehydroxy-2-(N'-propylureido)-3-O-[5-(N,N,N-trimethylammonio)pentyl]glycerol•halide or its mesylate,

(2RS)-1-O-hexadecyl-2-dehydroxy-2-(N'-propylureido)-3-O-[6-(N,N,N-trimethylammonio)hexyl]glycerol•halide or its mesylate,

(2RS)-1-O-hexadecyl-2-dehydroxy-2-(N'-propylureido)-3-O-[7-(N,N,N-trimethylammonio)heptyl]glycerol•halide or its mesylate,

(2RS)-1-O-hexadecyl-2-dehydroxy-2-(N'-propylureido)-3-O-[8-(N,N,N-trimethylammonio)octyl]glycerol•halide or its mesylate,

(2RS)-1-O-hexadecyl-2-dehydroxy-2-(N'-butylureido)-3-O-[4-(N,N,N-trimethylammonio)butyl]glycerol•halide or its mesylate,

(2RS)-1-O-hexadecyl-2-dehydroxy-2-(N'-butylureido)-3-O-[5-(N,N,N-trimethylammonio)pentyl]glycerol•halide or its mesylate,

(2RS)-1-O-hexadecyl-2-dehydroxy-2-(N'-butylureido)-3-O-[6-(N,N,N-trimethylammonio)hexyl]glycerol•halide or its mesylate,

(2RS)-1-O-hexadecyl-2-dehydroxy-2-(N'-butylureido)-3-O-[7-(N,N,N-trimethylammonio)heptyl]glycerol•halide or its mesylate,

(2RS)-1-O-hexadecyl-2-dehydroxy-2-(N'-butylureido)-3-O-[8-(N,N,N-trimethylammonio)octyl]glycerol•halide or its mesylate,

(2RS)-1-O-hexadecyl-2-dehydroxy-2-(N'-pentylureido)-3-O-[4-(N,N,N-trimethylammonio)butyl]glycerol•halide or its mesylate,

(2RS)-1-O-hexadecyl-2-dehydroxy-2-(N'-pentylureido)-3-O-[5-(N,N,N-trimethylammonio)pentyl]glycerol•halide or its mesylate,

(2RS)-1-O-hexadecyl-2-dehydroxy-2-(N'-pentylureido)-3-O-[6-(N,N,N-trimethylammonio)hexyl]glycerol•halide or its mesylate,

(2RS)-1-O-hexadecyl-2-dehydroxy-2-(N'-pentylureido)-3-O-[7-(N,N,N-trimethylammonio)heptyl]glycerol•halide or its mesylate,

(2RS)-1-O-hexadecyl-2-dehydroxy-2-(N'-pentylureido)-3-O-[8-(N,N,N-trimethylammonio)octyl]glycerol•halide or its mesylate,

and the corresponding 3-O-acyl analogues.

The following Reference Examples and Examples illustrate, but not limit, the preparation of compound of the present invention. In the Reference Examples and Examples, 'TLC', 'NMR', 'IR' and 'MS' represent 'Thin layer chromatography', 'Nuclear magnetic reasonance spectrum', 'Infrared absorption spectrum' and 'Mass spectrum', respectively. The solvents in parentheses specified in chromatographic separations show the developing solvents used : ratios are by volume. Except when specified otherwise, infrared absorption spectra were recorded by the liquid film method and nuclear magnetic resonance spectra were recorded in deutero-chloroform ($CDCl_3$) solution.

Various abbreviated words in Reference Example and Examples donate the following words:

THP : tetrahydropyran-2-yl group
Ø : phenyl group
Ms : mesyl group
Ts : tosyl group
AcOEt : ethyl acetate
DMF : dimethylformamide
$CH_2Cl_2$: methylene chloride
MeOH : methanol
AcOH : acetic acid
THF : tetrahydrofuran
HMPA : hexamethylphosphoramide
$CHCl_3$ : chloroform
NaCl : sodium chloride
$NaHCO_3$: sodium bicarbonate
NaOH : sodium hydroxide
sat. : saturated
aq. : aqueous

Reference Example 1

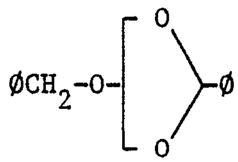

A mixture of 161 g of 1,3-O-benzylideneglycerol [the compound is described in J. Amer. Chem. Soc., 50, 2235 (1928)], 50 g of sodium hydride (content: 64.1%) and 800 ml of dry DMF was stirred for one hour at 60°C. After cooling with ice-bath, 227 g of benzyl bromide was added thereto, and the mixture was stirred for 10 minutes at room temperature and further for 2 hours at 60°C. To the reaction mixture was added water and then the mixture was concentrated under reduced pressure. Ethyl acetate was added to the residue thus obtained, and the solution was washed with water and a sat. aq. solution of NaCl, successively, dried over anhydrous sodium sulfate and concentrated under reduced pressure. The residue was purified by column chromatography on silica gel (eluent: a mixture of AcOEt and n-hexane) to give 218 g of the title compound having the following physical data:

TLC (AcOEt:n-hexane=1:2): Rf=0.47;

NMR: δ=7.53-7.07 (10H, m), 5.47 (1H, s), 4.63 (2H, s), 4.20 (4H, dt), 3.33 (1H, m).

Reference Example 2

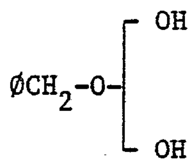

A mixture of 218 g of the benzylidene compound (prepared in Reference Example 1), 2 liters of MeOH and 2 g of p-toluenesulfonic acid was stirred overnight at room temperature. After adding triethylamine thereto, the reaction mixture was concentrated under reduced pressure. The residue was purified by column chromatography on silica gel (eluent: AcOEt) to give 138 g of the title compound having the following physical data:

TLC ($CHCl_3$:acetone=10:1): Rf=0.12;

NMR: δ=8.22 (5H, s), 4.53 (2H, s), 3.63 (5H, m), 2.86 (2H, m);

MS: m/e=182 ($M^+$), 107.

Reference Example 3

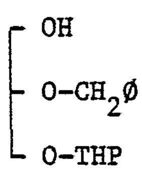

A mixture of 49 g of the dihydroxy compound (prepared in Reference Example 2), 22.6 g of 2,3-dihydropyran, 200 mg of p-toluenesulfonic acid and 300 ml of $CH_2Cl_2$ was stirred for 30 minutes at room temperature. The reaction mixture was neutralized with a sat. aq. solution of $NaHCO_3$ and the solution was extracted with AcOEt. The extract was washed with water and a sat. aq. solution of NaCl, successively, dried over anhydrous sodium sulfate and concentrated under reduced pressure. The residue was purified by column chromatography on silica gel (eluent: a mixture of AcOEt and cyclohexane) to give 37.4 g of the title compound having the following physical data:

TLC ($CHCl_3$:acetone=10:1): Rf=0.39;

NMR: $\delta$=7.28 (5H, s), 4.8-4.4 (3H, m), 4.0-3.3 (7H, m), 2.54-2.25 (1H, m), 1.92-1.2 (6H, m);

IR: $\nu$=3450, 2930, 2860, 1440, 1350 $cm^{-1}$.

Reference Example 4

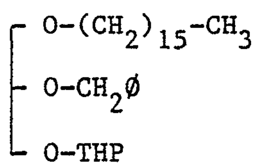

Under an atmosphere of argon, to a suspension of 10.4 g of sodium hydride (content: 64.1%) in 500 ml of dry DMF was added a solution of 37 g of the hydroxy compound (prepared in

Reference Example 3) in 150 ml of dry DMF. The mixture was stirred for one hour at 60°C. After cooling to 40°C, thereto was added a suspension of 84.8 g of hexadecyl bromide in 150 ml of dry DMF and the mixture obtained was stirred for 10 minutes at room temperature and further, for 3 hours at 60°C - 80°C. After adding 50 ml of water thereto, the reaction mixture was concentrated under reduced pressure. The residue was purified by column chromatography on silica gel (eluent: a mixture of $CH_2Cl_2$ and n-hexane, and $CH_2Cl_2$ only, successively) to give 57 g of the title compound having the following physical data:

TLC (AcOEt:cyclohexane=1:5): Rf=0.63;

NMR: δ=7.16 (5H, s), 4.6 (2H, s), 4.63-4.36 (1H, m), 4.0-3.16 (9H, m), 1.8-0.7 (35H, m);

IR: ν=2920, 2840, 1460, 1110 $cm^{-1}$.

By the same procedure as described in Reference Example 4, the compound having the following physical data was prepared.

(a)

$O-(CH_2)_5-CH_3$

$O-CH_2Ø$

O-THP

Starting Material: the hydroxy compound prepared in Reference Example 3 and hexyl bromide;

TLC (AcOEt:n-hexane=1:5): Rf=0.49;

NMR: δ=7.4-7.0 (5H, m), 4.61 (2H, s), 4.7-4.4 (1H, m), 4.0-3.1 (9H, m), 2.0-0.6 (17H, m);

IR: ν=2930, 2860, 1120 $cm^{-1}$.

Reference Example 5

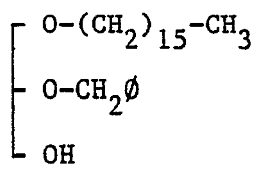

A mixture of 57 g of the 3-O-(tetrahydropyran-2-yl)-glycerol compound (prepared in Reference Example 4), 150 ml of $CH_2Cl_2$, 300 ml of MeOH and 500 mg of p-toluenesulfonic acid was stirred for 4 hours at room temperature. To the reaction mixture was added 100 ml of a sat. aq. solution of $NaHCO_3$ and concentrated under reduced pressure to distill off MeOH. To the residue was added 2 liters of AcOEt and the solution was washed with water and a sat. aq. solution of NaCl, successively, dried over anhydrous sodium sulfate and concentrated under reduced pressure. The residue was purified by column chromatography on silica gel (eluent: a mixture of $CH_2Cl_2$ and AcOEt) to give 34.8 g of the title compound having the following physical data:
TLC (AcOEt:cyclohexane=1:5): Rf=0.24;

NMR: δ=7.2 (5H, s), 4.6 (2H, s), 3.8-3.2 (7H, m), 2.3-2.0 (1H, m), 1.7-0.7 (31H, m);

IR: ν=3450, 2920, 2840, 1460, 1110 $cm^{-1}$.

By the same procedure as described in Reference Example 5, the compound having the following physical data was prepared.

(a)

$O-(CH_2)_5-CH_3$
$O-CH_2\phi$
OH

Starting Material: the 3-O-(tetrahydropyran-2-yl)glycerol compound prepared in Reference Example 4(a);

TLC (AcOEt:n-hexane=1:5): Rf=0.18;

NMR: δ=7.3-7.0 (5H, m), 4.56 (2H, s), 3.7-3.1 (7H, m), 2.43 (1H, s), 1.8-0.6 (11H, m);

IR: ν=3450, 2940, 2860, 1110 $cm^{-1}$.

MS: m/e=266 ($M^+$), 175.

<u>Reference Example 6</u>

$O-(CH_2)_{15}-CH_3$
$O-CH_2\phi$
$O-(CH_2)_4-Cl$

Under an atmosphere of argon, to a suspension of 1.1 g of sodium hydride (content: 64.1%) in 50 ml of dry DMF was added a solution of 10 g of the 3-hydroxy compound (prepared in Reference Example 5) in 50 ml of dry DMF and the mixture was stirred for 30 minutes at 70°C. A solution of 7.7 g of 4-(p-toluenesulfonyloxy)butyl chloride in 50 ml of dry DMF was added thereto at room temperature, and the mixture obtained was stirred for 15 minutes at 70°C. To the reaction mixture was added 10 ml of water and adjusted to pH 4 by adding 1N hydrochloric acid and then concentrated under reduced pressure. To the residue was added 500 ml of AcOEt and the solution was washed with water and a sat. aq. solution of NaCl, successively, dried over anhydrous sodium sulfate and concentrated under reduced pressure. The residue was purified by column chromatography on silica gel (eluent: a mixture of AcOEt and n-hexane) to give 5.8 g of the title compound having the following physical data:

TLC (AcOEt:hexane=1:4): Rf=0.69;

NMR: δ=7.18 (5H, s), 4.62 (2H, s), 3.8-3.13 (11H, m), 2.13-0.63 (37H, m);

IR: ν=2920, 2850, 1460, 1450, 1115, 735, 695 $cm^{-1}$;

MS: m/e=496 ($M^+$), 434, 405, 390, 214.

By the same procedure as described in Reference Example 6, the compounds having the following physical data in Table 1 were prepared.

Table 1

| Reference Example No. | Starting Materials | Structure of Product | Physical Data | | | |
|---|---|---|---|---|---|---|
| | | | TLC (developing solvent) | NMR | IR | MS |
| 6 (a) | Compound prepared in RE5 and TsO-$(CH_2)_5$-Cl | O-$(CH_2)_{15}$-$CH_3$<br>O-$CH_2$ø<br>O-$(CH_2)_5$-Cl | Rf=0.60 (AcOEt: cyclohexane: $CH_2Cl_2$=2:10:1) | 6.83 (5H,s), 4.57 (2H,s), 3.77-3.05 (11H,m), 2.30-0.97 (37H,m), 0.97-0.50 (3H,t) | 3060, 3030, 2920 2850, 1460, 1450, 1375, 1305, 1205, 1115, 1060, 730, 695 | |
| 6 (b) | Compound prepared in RE5 and MsO-$(CH_2)_7$-OTHP | O-$(CH_2)_{15}$-$CH_3$<br>O-$CH_2$ø<br>O-$(CH_2)_7$-OTHP | Rf=0.55 (AcOEt:n-hexane =1:5) | 7.2 (5H,s), 4.63 (2H,s), 4.6-4.3 (1H,m), 4.0-3.0 (13H,m), 2.0-0.6 (47H,m ) | | 604($M^+$), 519 |
| 6 (c) | Compound prepared in RE5 (a) and Br-$(CH_2)_8$-Br | O-$(CH_2)_5$-$CH_3$<br>O-$CH_2$ø<br>O-$(CH_2)_8$-Br | Rf=0.63 (AcOEt:n-hexane =1:5) | 7.4-7.0 (5H,m), 4.61 (2H, s), 3.8-3.1 (11H,m), 2.1-0.6 (23H,m) | | 458($M^+$), 456($M^+$), 376, 365 |

"RE" represents "Reference Example".

Reference Example 7

$O-(CH_2)_{15}-CH_3$
$O-CH_2\emptyset$
$O-(CH_2)_7-OH$

By the same procedure as described in Reference Example 5, the title compound having the following physical data was prepared from the tetrahydropyran-2-yloxy compound prepared in Reference Example 6 (b).

TLC (AcOEt:n-hexane:1:5): Rf=0.15;

IR: $\nu$=3450, 2940, 2870, 1460, 1120 $cm^{-1}$;

MS: m/e=520 ($M^+$), 429, 414.

Reference Example 8

$O-(CH_2)_{15}-CH_3$
$O-CH_2\emptyset$
$O-(CH_2)_7-OMs$

A mixture of 2.9 g of the hydroxy compound (prepared in Reference Example 7), 3 ml of triethylamine, 20 ml of $CH_2Cl_2$ and 0.55 ml of mesyl chloride was stirred for 30 minutes under cooling with ice-bath. After adding water thereto, the reaction mixture was extracted with AcOEt, and the extract was washed with

water and a sat. aq. solution of NaCl, successively, dried over anhydrous sodium sulfate and concentrated under reduced pressure to give 3.4 g of the title compound as crude product, having the following physical data:

TLC ($CHCl_3$:acetone=10:1): Rf=0.81;

NMR: $\delta$=7.2 (5H, s), 4.63 (2H, s), 4.13 (2H, t), 3.8-3.2 (9H, m),
2.9 (3H, s), 2.0-0.7 (41H, m);

MS: m/e=598 ($M^+$).

Reference Example 9

$O-(CH_2)_{15}-CH_3$

$O-CH_2\emptyset$

$O-(CH_2)_4-N(CH_3)_2$

A mixture of 5.5 g of the chlorobutylglycerol compound (prepared in Reference Example 6), 45 ml of a 40% aqueous solution of dimethylamine and 130 ml of DMF was stirred for 6.5 hours at 60°C. The reaction mixture was concentrated under reduced pressure and to the residue thus obtained was added 300 ml of AcOEt. The resulting solution was washed with 1N aq. solution of NaOH and a sat. aq. solution of NaCl, successively, dried over potassium carbonate and concentrated under reduced pressure. The residue was purified by column chromatography on silica gel (eluent: a mixture of $CHCl_3$, MeOH and triethylamine)

to give 5.1 g of the title compound having the following physical data:

TLC (AcOEt:AcOH:water=3:1:1): Rf=0.67;

NMR: $\delta$=7.22 (5H, s), 4.63 (2H, s), 3.87-3.20 (9H, m), 2.50-2.0 (2H, m and 6H, s), 1.83-0.67 (35H, m);

IR: $\nu$=3070, 3045, 2940, 2860, 1470, 1455, 1380, 1120, 740, 700 $cm^{-1}$;

MS: m/e=506, 505, 414, 398, 280.

By the same procedure as described in Reference Example 9, the compound having the following physical data in Table 2, were prepared.

Table 2

| Reference Example No. | Reference Example No. of Starting Materials | Structure of Product | Physical Data | | | |
|---|---|---|---|---|---|---|
| | | | TLC (developing solvent) | NMR | IR | MS |
| 9 (a) | 6 (a) | $O-(CH_2)_{15}-CH_3$<br>$O-CH_2\phi$<br>$O-(CH_2)_5-N(CH_3)_2 \cdot HCl$ | Rf=0.66 (AcOEt:AcOH:water =3:1:1) | 9.8-9.4 (1H,m), 7.23 (5H,s), 4.63 (2H,s), 3.9-2.7 (11H, m), 2.5 (6H,s), 2.0-0.8 (37H,m) | 2920, 2840, 2600, 2480, 1460, 1110 | |
| 9 (b) | 6 (c) | $O-(CH_2)_5-CH_3$<br>$O-CH_2\phi$<br>$O-(CH_2)_8-N(CH_3)_2$ | Rf=0.5 (AcOEt:AcOH:water =3:1:1) | 7.2 (5H,s), 4.6 (2H,s), 3.9-3.2 (9H,m), 2.2 (6H,s), 2.4-2.1 (2H,m), 2.0-0.7 (23H,m) | | 421($M^+$), 406 330, 314 |
| 9 (c) | 8 | $O-(CH_2)_{15}-CH_3$<br>$O-CH_2\phi$<br>$O-(CH_2)_7-N(CH_3)_2$ | Rf=0.54 (AcOEt:AcOH:water =3:1:1) | 7.2 (5H,s), 4.63 (2H,s), 3.83-3.1 (9H,m), 2.4-2.0 (2H,m), 2.16 (6H,s), 1.80-0.6 (41H,m) | 2940, 2860, 1460 1120 | 547($M^+$), 456, 440 |
| 9 (d) | 8 (used diethylamine) | $O-(CH_2)_{15}-CH_3$<br>$O-CH_2\phi$<br>$O-(CH_2)_7-N(C_2H_5)_2$ | Rf=0.56 (AcOEt:AcOH:water =3:1:1) | 7.2 (5H,s), 3.63 (2H,s), 3.9-3.2 (9H,m), 2.55 (4H,q), 2.5-2.2 (2H,m), 2.1-0.7 (41H,m), 1.05 (6H,t) | | 575($M^+$), 560, 545, 484, 468 |

Reference Example 10

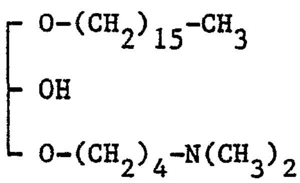

Under an atmosphere of hydrogen, a mixture of 5.1 g of the 2-O-benzylglycerol compound (prepared in Reference Example 9), 500 mg of palladium on carbon (content: 10%), 50 ml of EtOH and 0.7 ml of a concentrated hydrochloric acid was stirred for 3 hours at room temperature. The catalyst was filtered off and the filtrate was concentrated under reduced pressure. To the residue thus obtained was added 300 ml of AcOEt and the solution was washed with 1N aq. solution of NaOH, dried over potassium carbonate and concentrated under reduced pressure to give 4.1 g of the title compound having the following physical data:

TLC (AcOEt:AcOH:water=3:1:1): Rf=0.61;

NMR: $\delta$=4.08-3.63 (1H, m), 3.63-3.15 (8H, m), 2.93 (1H, s), 2.45-2.08 (8H, m), 2.18 (6H, s), 1.92-0.67 (35H, m);

IR: $\nu$=3420, 2925, 2855, 1465, 1375, 1265, 1115, 755 $cm^{-1}$.

By the same procedure as described in Reference Example 10, the compounds having the following physical data in Table 3 were prepared.

Table 3

| Reference Example No. | Reference Example No. of Starting Materials | Structure of Product | Physical Data | | | |
|---|---|---|---|---|---|---|
| | | | TLC (developing solvent) | NMR | IR | MS |
| 10 (a) | 9 (a) | $O-(CH_2)_{15}-CH_3$<br>$OH$<br>$O-(CH_2)_5-N(CH_3)_2$ | Rf=0.53 ($CH_2Cl_2$:AcOH:EtOH:water=8:4:2:1) | 4.1-3.7 (1H,m), 3.7-3.3 (8H,m), 3.1-2.9 (1H,m), 2.5-2.1 (2H,m and 6H,s), 1.8-1.05 (34H,m), 1.0-0.7 (3H,m) | 3600-3000, 2920, 2850, 1460, 1110 | |
| 10 (b) | 9 (b) | $O-(CH_2)_5-CH_3$<br>$OH$<br>$O-(CH_2)_8-N(CH_3)_2$ | Rf=0.48 (AcOEt:AcOH:water =3:1:1) | 4.1-3.2 (9H,m), 2.83 (1H,s), 2.2 (6H,s), 2.46-2.0 (2H,m), 2.0-0.7 (23H,m) | 3400, 2940, 2860, 1460, 1120 | 331($M^+$), 316, 313 |
| 10 (c) | 9 (c) | $O-(CH_2)_{15}-CH_3$<br>$OH$<br>$O-(CH_2)_7-N(CH_3)_2\cdot HCl$ | Rf=0.46 (AcOEt:AcOH:water =3:1:1) | 4.1-3.2 (9H,m), 3.1-2.4 (10H,m), 2.0-0.6 (41H,m) | | 456 |
| 10 (d) | 9 (d) | $O-(CH_2)_{15}-CH_3$<br>$OH$<br>$O-(CH_2)_7-N(C_2H_5)_2\cdot HCl$ | Rf=0.58 (AcOEt:AcOH:water =3:1:1) | | 3450, 2930, 2850 1470, 1110<br>(KBr method) | 485($M^+$), 470, 456 |

Reference Example 11

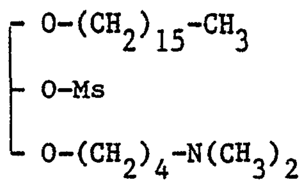

To a mixture of 3.4 g of the 2-hydroxy compound (prepared in Reference Example 10), 4 ml of triethylamine and 50 ml of $CH_2Cl_2$ was added 0.76 ml of mesyl chloride at 0°C and the mixture was stirred for one hour at the same temperature. After adding 300 ml of diethyl ether thereto, the reaction mixture was washed with 1N aq. solution of NaOH and a sat. aq. solution of NaCl, successively, dried over anhydrous sodium sulfate and concentrated under reduced pressure to give 3.86 g of the title compound having the following physical data:

TLC (AcOEt:AcOH:water=3:1:1): Rf=0.61;

NMR: δ=4.93-4.53 (1H, m), 3.80-3.16 (8H, m), 3.0 (3H, s), 2.16 (6H, s), 2.46-2.0 (2H, m), 1.83-0.66 (35H, m);

IR: ν=2920, 2840, 1450, 1350, 1170, 1110 $cm^{-1}$.

By the same procedure as described in Reference Example 11, the compounds having the following physical data in Table 4 were prepared.

Table 4

| Reference Example No. | Reference Example No. of Starting Materials | Structure of Product | Physical Data | | |
|---|---|---|---|---|---|
| | | | TLC (developing solvent) | NMR | IR |
| 11 (a) | 10 (a) | $O-(CH_2)_{15}-CH_3$<br>$O-Ms$<br>$O-(CH_2)_5-N(CH_3)_2$ | Rf=0.42 ($CHCl_3$:MeOH: triethylamine =10:1:0.05) | 4.9-4.5 (1H,m), 3.7-3.1 (8H,m), 3.0 (3H,s), 2.4-2.0 (2H,m and 6H,s), 2.0-0.6 (37H,m) | 2920, 2850, 1460, 1360, 1170 |
| 11 (b) | 10 (b) | $O-(CH_2)_5-CH_3$<br>$O-Ms$<br>$O-(CH_2)_8-N(CH_3)_2$ | Rf=0.47 (AcOEt:AcOH:water =3:1:1) | 4.93-4.5 (1H,m), 3.7-3.0 (8H,m), 3.0 (3H,s), 2.2 (6H,s), 2.4-2.0 (2H,m), 1.9-0.7 (23H,m) | |
| 11 (c) | 10 (c) | $O-(CH_2)_{15}-CH_3$<br>$O-Ms$<br>$O-(CH_2)_7-N(CH_3)_2$ | Rf=0.57 (AcOEt:AcOH:water =3:1:1) | 4.86-4.5 (1H,m), 3.66-3.3 (8H,m), 3.0 (3H,s), 2.4-2.0 (2H,m), 2.2 (6H,s), 1.9-0.7 (41H,m) | |
| 11 (d) | 10 (d) | $O-(CH_2)_{15}-CH_3$<br>$O-Ms$<br>$O-(CH_2)_7-N(C_2H_5)_2$ | Rf=0.71 (AcOEt:AcOH:water =3:1:1) | 4.9-4.5 (1H,m), 3.7-3.1 (8H,m), 3.0 (3H,s), 2.65 (4H,q), 2.7-2.3 (2H,m), 1.9-0.7 (41H,m), 1.1 (6H,t) | |

Example 1

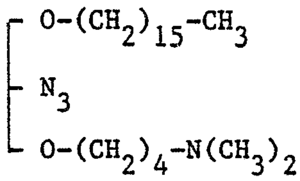

A mixture of 3.86 g of the 2-O-mesylglycerol compound (prepared in Reference Example 11), 1.5 g of sodium azide and 50 ml of HMPA was stirred overnight at 50°C. After adding 300 ml of diethyl ether thereto, the reaction mixture was washed with 1N aq. solution of NaOH, water and a sat. aq. solution of NaCl, successively, dried over anhydrous sodium sulfate and concentrated under reduced pressure. The residue was purified by column chromatography on silica gel (eluent: a mixture of $CH_2Cl_2$, MeOH and triethylamine) to give 2.92 g of the title compound having the following physical data:

TLC ($CHCl_3$:AcOH:EtOH:water=20:4:3:1): Rf=0.76;

NMR: $\delta$=3.74-3.38 (9H, m), 2.22 (6H, s), 2.36-2.20 (2H, m), 1.70-1.48 (6H, m), 1.25 (26H, m), 0.88 (3H, m);

IR: $\nu$=2920, 2840, 2110, 2090, 1460, 1260, 1110 $cm^{-1}$;

MS: m/e=440 ($M^+$), 398.

By the same procedure as described in Example 1, the compounds having the following physical data in Table 5 were prepared.

Table 5

| Example No. | Reference Example No. of Starting Materials | Structure of Product | Physical Data: TLC (developing solvent) | NMR | IR | MS |
|---|---|---|---|---|---|---|
| 1 (a) | 11 (a) | $O-(CH_2)_{15}-CH_3$<br>$N_3$<br>$O-(CH_2)_5-N(CH_3)_2$ | Rf=0.60 ($CHCl_3$:AcOH:EtOH: water=20:4:3:1) | 3.9-3.1 (9H,m), 2.6-2.0 (2H,m and 6H,s), 1.9-0.7 (37H,m) | 2920, 2840, 2110, 2080, 1450, 1260, 1110 | |
| 1 (b) | 11 (b) | $O-(CH_2)_5-CH_3$<br>$N_3$<br>$O-(CH_2)_8-N(CH_3)_2$ | Rf=0.47 (AcOEt:AcOH: water=3:1:1) | 3.8-3.1 (9H,m), 2.2 (6H,s), 2.4-2.0 (2H,m), 1.9-0.7 (23H,m) | 2940, 2870, 2100 1460, 1120 | 356($M^+$), 314 |
| 1 (c) | 11 (c) | $O-(CH_2)_{15}-CH_3$<br>$N_3$<br>$O-(CH_2)_7-N(CH_3)_2$ | Rf=0.72 (AcOEt:AcOH: water=3:1:1) | 3.7-3.1 (9H,m), 2.1 (6H,s), 2.4-2.0 (2H,m), 1.8-0.6 (41H,m) | 2930, 2860, 2200 1460, 1120 | 482($M^+$), 440, 410 |
| 1 (d) | 11 (d) | $O-(CH_2)_{15}-CH_3$<br>$N_3$<br>$O-(CH_2)_7-N(C_2H_5)_2$ | Rf=0.78 (AcOEt:AcOH: water=3:1:1) | 3.7-3.1 (9H,m), 2.45 (4H,q), 2.5-2.1 (2H,m), 1.9-0.7 (41H,m), 0.96 (6H,t) | 2930, 2850, 2100 1460, 1110 | 510($M^+$), 495, 481, 468 |

Example 2

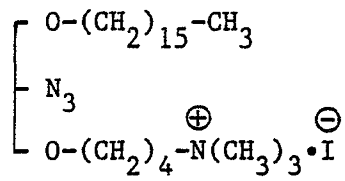

A mixture of 180 mg of the dimethylaminoglycerol compound (prepared in Example 1), 0.3 ml of methyl iodide and 5 ml of MeOH was stirred for one hour at room temperature, and then the reaction mixture was concentrated under reduced pressure. The residue was purified by column chromatography on silica gel (eluent: a mixture of $CHCl_3$ and MeOH, and a mixture of $CHCl_3$, MeOH and water, successively) to give 224 mg of the title compound having the following physical data:

Melting Point: 50 - 52°C;

TLC (AcOEt:AcOH:water=3:1:1): Rf=0.49;

NMR: $\delta$=3.42 (9H, s), 3.8-3.4 (11H, m), 2.04-1.5 (6H, m), 1.25 (26H, m), 0.88 (3H, m);

IR (KBr method): $\nu$=3450, 2910, 2850, 2100, 1460, 1270, 1110 $cm^{-1}$; MS: m/e=398, 310.

By the same procedure as described in Example 2, the compound having the following physical data was prepared.

(a) 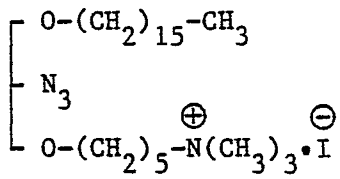

Starting Material: the dimethylaminoglycerol compound prepared in Example 1(a), with the proviso that potassium bicarbonate was used in the reaction;

Melting Point: 47 - 51°C;

TLC ($CH_2Cl_2$:AcOH:EtOH:water=8:4:2:1): Rf=0.46;

NMR: δ=3.8-3.3 (11H, m and 9H, s), 2.1-1.0 (34H, m), 1.0-0.7 (3H, m)

IR (KBr method): ν=3600-3100, 2910, 2840, 2120, 2080, 1460, 1270, 1110 $cm^{-1}$.

Example 3

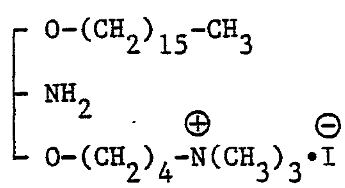

Under an atmosphere of hydrogen, a mixture of 194 mg of the 2-azidoglycerol compound (prepared in Example 2), 100 mg of palladium on carbon (content: 10%) and 12 ml of MeOH was stirred for 2 hours at room temperature. The catalyst was filtered off

and the filtrate was concentrated under reduced pressure. The residue was purified by column chromatography on silica gel (eluent: a mixture of $CHCl_3$, MeOH and water) to give 100.3 mg of the title compound having the following physical data:

TLC (AcOEt:AcOH:water=3:1:1): Rf=0.15;

NMR: δ=3.86-3.56 (8H, m), 3.45 (9H, s), 3.56-3.38 (3H, m), 2.2-2.0 (2H, m), 1.86-1.68 (2H, m), 1.68-1.50 (2H, m), 1.26 (26H, m), 0.88 (3H, m);

IR: ν=3450, 2920, 2840, 1460, 1110 $cm^{-1}$;

MS: m/e=414, 399, 370.

By the same procedure as described in Example 3, the compounds having the following physical data in Table 6 were prepared.

Table 6

| Example No. | Example No. of Starting Materials | Structure of Product | Physical Data | | | |
|---|---|---|---|---|---|---|
| | | | TLC (developing solvent) | NMR | IR | MS |
| 3 (a) | 2 (a) | $O-(CH_2)_{15}-CH_3$<br>$NH_2$<br>$O-(CH_2)_5-\overset{\oplus}{N}(CH_3)_3 \cdot \overset{\ominus}{I}$ | Rf=0.25 ($CH_2Cl_2$:AcOH: EtOH:water =8:4:2:1)<br>Melting point: 35 - 38°C | 4.2-3.1 (12H,m and 9H,s), 2.9-2.76 (1H,m), 2.0-1.0 (34H,m), 0.98-0.8 (3H,m) | 3600-3100, 2910, 2840, 1460, 1110<br>(KBr method) | |
| 3 (b) | 1 | $O-(CH_2)_{15}-CH_3$<br>$NH_2$<br>$O-(CH_2)_4-N(CH_3)_2$ | Rf=0.56 ($CH_2Cl_2$:AcOH: EtOH:water =8:4:2:1) | 3.70-3.23 (8H,m), 3.23-3.08 (1H,m), 2.32 (6H,s), 2.50-2.30 (2H,m), 1.70-1.46 (6H,m), 1.24 (26H,m), 0.87 (3H,m) | 2920, 2840, 1460 1110 | $414(M^+)$, 399, 398, 369 |
| 3 (c) | 1 (a) | $O-(CH_2)_{15}-CH_3$<br>$NH_2$<br>$O-(CH_2)_5-N(CH_3)_2$ | Rf=0.40 ($CH_2Cl_2$:AcOH: EtOH:water =8:4:2:1) | 3.7-2.9 (9H,m), 2.5-2.0 (2H,m and 6H,s), 2.0-1.7 (39H,m) | 2920, 2840, 1460 1110 | |

Example 4

$O-(CH_2)_5-CH_3$
$NH_2$
$O-(CH_2)_8-N(CH_3)_2$

A mixture of 173 mg of the 2-azidoglycerol compound [prepared in Example 1(b)], 100 mg of lithium aluminium hydride and 10 ml of diethyl ethyl was refluxed for one hour. To the reaction mixture was added a 10% aq. solution of NaOH until the mixture turned to white and then was added an adequate amount of magnesium sulfate and the mixture was filtered. The filtrate was concentrated under reduced pressure to give 153 mg of the title compound having the following physical data:

TLC (AcOEt:AcOH:water=3:1:1): Rf=0.38;

NMR: $\delta$=3.8-3.2 (9H, m), 2.2 (6H, s), 2.4-2.0 (2H, m), 2.0-0.7 (25H, m);

IR: $\nu$=3400, 2950, 2860, 1460, 1110 $cm^{-1}$;

MS: m/e=331, 286.

By the same procedure as described in Example 4, the compounds having the following physical data were prepared.

(a)

$O-(CH_2)_{15}-CH_3$
$NH_2$
$O-(CH_2)_7-N(CH_3)_2$

Starting Material: the 2-azidoglycerol compound prepared in Example 1(c);

TLC (AcOEt:AcOH:water=3:1:1): Rf=0.42;

NMR: δ=3.6-3.0 (9H, m), 2.4-2.0 (2H, m), 2.1 (6H, s), 1.9-0.6 (43H, m);

IR: ν=2940, 2860, 1460, 1120 $cm^{-1}$.

(b)

$O-(CH_2)_{15}-CH_3$
$NH_2$
$O-(CH_2)_7-N(C_2H_5)_2$

Starting Material: the 2-azidoglycerol compound prepared in Example 1(d);

TLC (AcOEt:AcOH:water=3:1:1): Rf=0.56;

MS: m/e=484 ($M^+$), 470, 456, 412.

Example 5

$O-(CH_2)_{15}-CH_3$
NHCOØ
$O-(CH_2)_4-N(CH_3)_2$

To a mixture of 106.5 mg of the 2-aminoglycerol compound [prepared in Example 3(b)], 0.1 ml of triethylamine and 5 ml of $CH_2Cl_2$ was added 35.9 μl of benzoyl chloride at 0°C

and the mixture was stirred for one hour at room temperature and then concentrated under reduced pressure. The residue was purified by column chromatography on silica gel (eluent: a mixture of $CHCl_3$, MeOH and triethylamine) to give 42.5 mg of the title compound having the following physical data:

TLC (AcOEt:AcOH:water=3:1:1): Rf=0.64;

NMR: δ=7.82-7.71 (2H, m), 7.54-7.35 (3H, m), 7.60-6.48 (1H, m), 4.45-4.32 (1H, m), 3.70-3.38 (8H, m), 2.20 (3H, s), 2.35-2.18 (2H, m), 1.69-1.44 (6H, m), 1.25 (26H, m), 0.87 (3H, m);

IR: ν=3300, 2920, 2850, 1640, 1460, 1110 $cm^{-1}$;

MS: m/e=518 ($M^+$), 503, 474, 402, 388.

By the same procedure as described in Example 5, the compounds having the following physical data in Table 7 were prepared.

Table 7(1)

| Example No. | Starting Materials | Structure of Product | Physical Data: TLC (developing solvent) | Physical Data: NMR | Physical Data: IR | Physical Data: MS |
|---|---|---|---|---|---|---|
| 5 (a) | Compound prepared in E3(b) and acetic anhydride | $O-(CH_2)_{15}-CH_3$<br>$NHCOCH_3$<br>$O-(CH_2)_4-N(CH_3)_2$ | Rf=0.57 (AcOEt:AcOH: water=3:1:1) Melting Point: 36 - 38°C | 5.95-5.75 (1H,m), 4.26-4.10 (1H,m), 3.60-3.34 (8H,m), 2.22 (6H,s), 2.36-2.2 (2H, m), 1.98 (3H,s), 1.65-1.44 (6H,m), 1.25 (26H,m), 0.88 (3H,m) | 3280, 2920, 2850, 1650, 1550, 1460, 1380, 1110 (KBr method) | 456($M^+$), 441 412 |
| 5 (b) | Compound prepared in E3(b) and butyryl chloride | $O-(CH_2)_{15}-CH_3$<br>$NHCO-(CH_2)_2-CH_3$<br>$O-(CH_2)_4-N(CH_3)_2$ | Rf=0.61 (AcOEt:AcOH: water=3:1:1) Melting Point: 37 - 39°C | 5.85-5.74 (1H,m), 4.25-4.12 (1H,m), 5.59-3.35 (8H,m), 2.21 (6H,s), 2.14 (2H,t), 2.36-2.18 (2H,m), 1.75-1.44 (8H,m), 1.24 (26H,m), 0.93 (3H,t), 0.86 (3H,m) | 3300, 2930, 2850, 1650, 1540, 1460, 1110 (KBr method) | 484($M^+$), 469, 454, 441, 368 |
| 5 (c) | Compound prepared in E3(b) and hexanoyl chloride | $O-(CH_2)_{15}-CH_3$<br>$NHCO-(CH_2)_4-CH_3$<br>$O-(CH_2)_4-N(CH_3)_2$ | Rf=0.67 (AcOEt:AcOH: water=3:1:1) Melting Point: 40 - 41°C | 5.86-5.77 (1H,m), 4.28-4.12 (1H,m), 3.60-3.35 (8H,m), 2.22 (6H,s), 2.16 (2H,t), 2.36-2.15 (2H,m), 1.70-1.44 (8H,m), 1.25 (26H,m), 0.98-0.80 (6H,m) | 3300, 2900, 1640, 1540, 1460, 1380 (KBr method) | 512($M^+$), 497, 483, 468 |
| 5 (d) | Compound prepared in E3(c) and benzoyl chloride | $O-(CH_2)_{15}-CH_3$<br>NHCOØ<br>$O-(CH_2)_5-N(CH_3)_2$ | Rf=0.79 ($CH_2Cl_2$:AcOH; EtOH:water= 8:4:2:1) | 7.9-7.1 (5H,m), 6.7-6.0 (1H,m), 4.6-4.0 (1H,m), 3.8-3.1 (8H,m), 2.5-2.0 (2H,m and 6H,s), 1.8-0.7 (37H,m) | 3600-3100, 2920, 2840, 1640, 1600, 1530, 1460, 1110 | |

"E" represents "Example".

Table 7(2)

| Example No. | Starting Materials | Structure of Product | Physical Data | | | |
|---|---|---|---|---|---|---|
| | | | TLC (developing solvent) | NMR | IR | MS |
| 5 (e) | Compound prepared in E3(a) and acetic anhydride | $O-(CH_2)_{15}-CH_3$<br>NHCOCH$_3$<br>$O-(CH_2)_5-\overset{\oplus}{N}(CH_3)_3 \cdot \overset{\ominus}{I}$ | Rf=0.51 ($CH_2Cl_2$:AcOH: EtOH:water= 8:4:2:1) Melting Point: 73 - 76°C | 6.06-5.96 (1H,m), 4.24-4.1 (1H,m), 3.74-3.3 (10H,m and 9H,s), 2.0 (3H,s), 1.99-1.2 (34H,m), 1.0-0.8 (3H,m) | 3600-3100,2920, 2840, 1640, 1460, 1110 | |
| 5 (f) | Compound prepared in E4(a) and acetic anhydride | $O-(CH_2)_{15}-CH_3$<br>NHCOCH$_3$<br>$O-(CH_2)_7-N(CH_3)_2 \cdot AcOH$ | Rf=0.48 (AcOEt:AcOH: water=3:1:1) | 9.68 (1H,s), 6.0-5.63 (1H,m), 4.4-3.9 (1H,m), 3.8-3.1 (8H,m), 2.8-2.4 (2H,m), 2.46 (6H,s), 1.96 (6H,s), 1.8-0.6 (41H,m) | 3300, 2920, 2850, 1650, 1120 | 498($M^+$), 484, 455 |
| 5 (g) | Compound prepared in E4(a) and benzoyl chloride | $O-(CH_2)_{15}-CH_3$<br>NHCOØ<br>$O-(CH_2)_7-N(CH_3)_2$ | Rf=0.53 (AcOEt:AcOH: water=3:1:1) | 7.8-7.2 (5H,m), 6.6-6.3 (1H,m), 4.6-4.1 (1H,m), 3.8-3.1 (8H,m), 2.4-2.0 (2H,m), 2.2 (6H,s), 1.8-0.7 (41H,m) | 3300, 2930, 2850, 1640, 1120 | 560($M^+$), 545, 516 |

"E" represents "Example".

Example 6

$O-(CH_2)_{15}-CH_3$

$NHCOO\phi$

$O-(CH_2)_4-N(CH_3)_2$

To a mixture of 90.9 mg of the 2-aminoglycerol compound [prepared in Example 3(b)], 0.1 ml of triethylamine and 5 ml of $CH_2Cl_2$ was added 50 µl of phenyl chloroformate at 0°C. The mixture was stirred overnight at room temperature and then concentrated under reduced pressure. The residue was purified by column chromatography on silica gel (eluent: a mixture of $CHCl_3$, MeOH and triethylamine) to give 35.8 mg of the title compound having the following physical data:

TLC (AcOEt:AcOH:water=3:1:1): Rf=0.55;

NMR: δ=7.4-6.9 (5H, m), 5.56-5.2 (1H, m), 4.1-3.1 (9H, m), 2.2 (6H, s), 2.5-2.0 (2H, m), 2.0-0.7 (35H, m);

IR: $\nu$=1740 $cm^{-1}$;

MS: m/e=534 ($M^+$), 440, 398.

By the same procedure as described in Example 6, the compounds having the following physical data in Table 8 were prepared.

Table 8(1)

| Example No. | Starting Materials | Structure of Product | Physical Data | | | |
|---|---|---|---|---|---|---|
| | | | TLC (developing solvent) | NMR | IR | MS |
| 6 (a) | Compound prepared in E3(b) and methyl chloroformate | O-$(CH_2)_{15}$-$CH_3$<br>$NHCOOCH_3$<br>O-$(CH_2)_4$-$N(CH_3)_2$ | Rf=0.54 (AcOEt:AcOH: water=3:1:1) | 5.12-5.00 (1H,m), 3.95-3.78 (1H,m), 3.65 (3H,s), 3.57-3.34 (8H,m), 2.20 (6H,s), 2.33-2.18 (2H,m), 1.64-1.42 (6H,m), 1.25 (26H,m), 0.87 (3H,m) | 3500, 2940, 2860, 1730, 1470, 1110 | 472($M^+$), 457 441, 398 |
| 6 (b) | Compound prepared in E3(b) and ethyl chloroformate | O-$(CH_2)_{15}$-$CH_3$<br>$NHCOOC_2H_5$<br>O-$(CH_2)_4$-$N(CH_3)_2$ | Rf=0.58 (AcOEt:AcOH: water=3:1:1) | 5.15-4.9 (1H,m), 4.11 (2H, q), 4.0-3.78 (1H,m), 3.62-3.34 (8H,m), 2.22 (6H,s), 2.38-2.2 (2H,m), 1.65-1.46 (6H,m), 1.26 (29H,m), 0.88 (3H,m) | 2910, 2840, 1720 | 486($M^+$), 471, 441 |
| 6 (c) | Compound prepared in E3(b) and butyl chloroformate | O-$(CH_2)_{15}$-$CH_3$<br>NHCOO-$(CH_2)_3$-$CH_3$<br>O-$(CH_2)_4$-$N(CH_3)_2$ | Rf=0.57 (AcOEt:AcOH: water=3:1:1) | 5.08-4.95 (1H,m), 4.03 (2H, t), 3.95-3.80 (1H,m), 3.58-3.35 (8H,m), 2.20 (6H,s), 2.32-2.18 (2H,m), 1.74-1.32 (10H,m), 1.25 (26H,m), 0.92 (3H,t), 0.88 (3H,m) | 3500, 2930, 2850, 1720, 1460, 1110 | 514($M^+$), 499, 485, 470, 441 |
| 6 (d) | Compound prepared in E3(b) and isobutyl chloroformate | O-$(CH_2)_{15}$-$CH_3$<br>NHCOO-$CH_2$-$CH<^{CH_3}_{CH_3}$<br>O-$(CH_2)_4$-$N(CH_3)_2$ | Rf=0.57 (AcOEt:AcOH: water=3:1:1) | 5.08-4.96 (1H,m), 3.82 (2H, d), 3.96-3.78 (1H,m), 3.58-3.36 (8H,m), 2.21 (6H,s), 2.34-2.16 (2H,m), 2.00-1.44 (7H,m), 1.26 (26H,m), 0.92 (6H,d), 0.88 (3H,m) | 2940, 2860, 1720, 1460, 1110 | 514($M^+$), 499, 485, 470 |

"E" represents "Example".

Table 8(2)

| Example No. | Starting Materials | Structure of Product | Physical Data | | | |
|---|---|---|---|---|---|---|
| | | | TLC (developing solvent) | NMR | IR | MS |
| 6 (e) | Compound prepared in E3(c) and ethyl chloroformate | 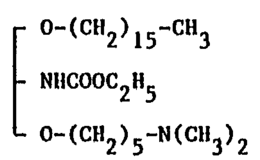 | Rf=0.79 ($CH_2Cl_2$:AcOH: EtOH:water= 8:4:2:1) | 5.2-4.9 (1H,m), 4.12 (2H,q), 4.0-3.3 (9H,m), 2.4-2.1 (2H,m and 6H,s), 1.80-1.02 (34H,m), 1.0-0.7 (3H,m) | 2920, 2840, 1720, 1460, 1110 | |
| 6 (f) | Compound prepared in E4 and isobutyl chloroformate | 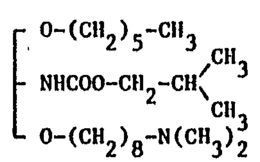 | Rf=0.54 (AcOEt:AcOH: water=3:1:1) | 5.10-4.96 (1H,m), 3.96-3.78 (1H,m), 3.84 (2H,d), 3.60-3.35 (9H,m), 2.37-2.2 (2H,m) 2.26 (6H,s), 2.00-1.80 (1H,m), 1.65-1.20 (20H,m), 0.93 (6H,d), 0.89 (3H,m) | 2940, 2860, 1720, 1460, 1120 | 430($M^+$), 415 386, 357 |
| 6 (g) | Compound prepared in E4(a) and methyl chloroformate | 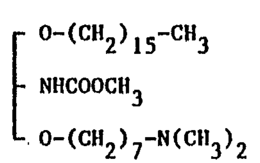 | Rf=0.51 (AcOEt:AcOH: water=3:1:1) | 5.1-4.8 (1H,m), 3.56 (3H,s), 4.0-3.2 (9H,m), 2.5-2.0 (2H,m), 2.2 (6H,s), 1.9-0.7 (41H,m) | 2930, 2850, 1720, 1110 | 514($M^+$), 499, 483, 470, 440 |
| 6 (h) | Compound prepared in E4(b) and isobutyl chloroformate | 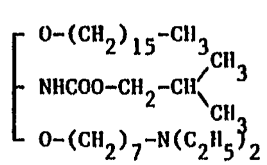 | Rf=0.71 (AcOEt:AcOH: water=3:1:1) | 5.1-4.7 (1H,m), 4.2-4.0 (1H,m), 4.0-3.1 (10H,m), 2.55 (4H,q), 2.5-2.2 (2H,m), 2.0-0.7 (42H,m), 1.0 (6H,t), 0.88 (6H,d) | 3500, 2940, 2860, 1730, 1470, 1120 | 584($M^+$), 570, 556, 511 |

"E" represents "Example".

Example 7

$$\left[\begin{array}{l} O-(CH_2)_{15}-CH_3 \\ NHCONH\phi \\ O-(CH_2)_4-N(CH_3)_2 \end{array}\right.$$

A mixture of 99.3 mg of the 2-aminoglycerol compound [prepared in Example 3(b)], 0.1 ml of phenyl isocyanate, 0.1 ml of triethylamine and 5 ml of $CHCl_3$ was stirred for one hour at room temperature and then concentrated under reduced pressure. The residue was purified by column chromatography on silica gel (eluent: a mixture of $CHCl_3$, MeOH and triethylamine) to give 91.2 mg of the title compound having the following physical data:

Melting Point: 35 - 38°C;

TLC (AcOEt:AcOH:water=3:1:1): Rf=0.69;

NMR: $\delta$=7.44-7.18 (5H, m), 7.06-6.94 (1H, m), 5.36-5.27 (1H, m), 4.10-3.96 (1H, m), 3.64-3.35 (8H, m), 2.22 (6H, s), 2.36-2.18 (2H, m), 1.64-1.45 (6H, m), 1.24 (26H, m), 0.86 (3H, m);

IR (KBr method): $\nu$=3340, 2920, 2850, 1650, 1600, 1545, 1500, 1120 $cm^{-1}$; MS: m/e=533 ($M^+$), 518, 489, 441, 413.

By the same procedure as described in Example 7, the compounds having the following physical data in Table 9 were prepared.

Table 9

| Example No. | Starting Materials | Structure of Product | Physical Data: TLC (developing solvent) | Physical Data: NMR | Physical Data: IR | Physical Data: MS |
|---|---|---|---|---|---|---|
| 7 (a) | Compound prepared in E3(b) and methyl isocyanate | $O-(CH_2)_{15}-CH_3$<br>$NHCONHCH_3$<br>$O-(CH_2)_4-N(CH_3)_2$ | Rf=0.73 (AcOEt:AcOH: water=3:1:1) Melting Point: 41 - 43°C | | 3350, 2940, 2860, 1640, 1580<br>(KBr method) | 471($M^+$), 456, 441, 427, 398, 355 |
| 7 (b) | Compound prepared in E3(b) and propyl isocyanate | $O-(CH_2)_{15}-CH_3$<br>$NHCONH-(CH_2)_2-CH_3$<br>$O-(CH_2)_4-N(CH_3)_2$ | Rf=0.52 (AcOEt:AcOH: water=3:1:1) | 4.96-4.69 (2H,m), 3.98-3.8 (1H,m), 3.60-3.34 (8H,m), 3.20-3.04 (2H,m), 2.22 (6H, s), 2.38-2.18 (2H,m), 1.68-1.40 (8H,m), 1.26 (26H,m), 0.91 (3H,t), 0.87 (3H,m) | 3350, 2930, 2860, 1630, 1570, 1460, 1110 | 499($M^+$), 484, 470, 455 |
| 7 (c) | Compound prepared in E3(b) and butyl isocyanate | $O-(CH_2)_{15}-CH_3$<br>$NHCONH-(CH_2)_3-CH_3$<br>$O-(CH_2)_4-N(CH_3)_2$ | Rf=0.69 (AcOEt:AcOH: water=3:1:1) Melting Point: 35 - 38°C | 4.94-4.70 (2H,m), 3.96-3.84 (1H,m), 3.58-3.35 (8H,m), 3.22-3.08 (2H,m), 2.22 (6H, s), 2.38-2.20 (2H,m), 1.70-1.20 (34H,m), 0.91 (3H,t), 0.87 (3H,m). | 3350, 2930, 2860, 1640, 1570, 1460, 1380, 1270, 1130<br>(KBr method) | 513($M^+$), 498, 484, 469, 414 |
| 7 (d) | Compound prepared in E4(a) and methyl isocyanate | $O-(CH_2)_{15}-CH_3$<br>$NHCONHCH_3$<br>$O-(CH_2)_7-N(CH_3)_2$ | Rf=0.46 (AcOEt:AcOH: water=3:1:1) | 5.3-4.8 (2H,m), 4.1-3.7 (1H,m), 3.6-3.2 (8H,m), 2.83-2.6 (3H,m), 2.5-2.0 (2H,m), 2.2 (6H,s), 1.8-0.7 (41H,m) | 3350, 2940, 2860, 1640, 1120 | 513($M^+$), 469, 455, 440 |

"E" represents "Example".

Example 8

$$\begin{cases} O-(CH_2)_{15}-CH_3 \\ NHCO\phi \\ O-(CH_2)_4-\overset{\oplus}{N}(CH_3)_3\cdot\overset{\ominus}{I} \end{cases}$$

A mixture of 101 mg of the dimethylaminoglycerol compound (prepared in Example 5), 0.2 ml of methyl iodide, 5 ml of MeOH and 20 mg of potassium bicarbonate was stirred for one hour at room temperature and then the reaction mixture was concentrated under reduced pressure. The residue was purified by column chromatography on silica gel (eluent: a mixture of $CHCl_3$ and MeOH, and a mixture of $CHCl_3$, MeOH and water, successively) to give 75.3 mg of the title compound having the following physical data:

Melting Point: 86 - 90°C;

TLC (AcOEt:AcOH:water=3:1:1): Rf=0.63;

NMR: $\delta$=7.90-7.78 (2H, m), 7.60-7.38 (3H, m), 6.90-6.79 (1H, m), 4.56-4.44 (1H, m), 3.80-3.36 (10H, m), 3.25 (9H, s), 2.0-1.46 (6H, m), 1.25 (26H, m), 0.87 (3H, m);

IR (KBr method): $\nu$=3400, 3300, 2920, 2840, 1635, 1535, 1470, 1110 $cm^{-1}$;

MS: m/e=518, 503, 474, 402.

By the same procedure as described in Example 8, the compounds having the following physical data in Table 10 were prepared.

Table 10(1)

| Example No. | Example No. of Starting Materials | Structure of Product | Physical Data: TLC (developing solvent) | NMR | IR | MS |
|---|---|---|---|---|---|---|
| 8 (a) | 5 (a) (without potassium bicarbonate) | $O-(CH_2)_{15}-CH_3$<br>$NHCOCH_3$<br>$O-(CH_2)_4-\overset{\oplus}{N}(CH_3)_3 \cdot \overset{\ominus}{I}$ | Rf=0.46 (AcOEt:AcOH: water=3:1:1) Melting Point: 88 - 92°C | 6.40-6.28 (1H,m), 4.30-4.16 (1H,m), 3.78-3.64 (2H,m), 3.39 (9H,s), 3.64-3.32 (8H, m), 2.02 (3H,s), 2.0-1.44 (6H,m), 1.24 (26H,m), 0.86 (3H,m) | 3450, 3280, 2920, 2840, 1640, 1540, 1460, 1380, 1120 (KBr method) | 456, 441, 412, 340 |
| 8 (b) | 5 (b) | $O-(CH_2)_{15}-CH_3$<br>$NHCO-(CH_2)_2-CH_3$<br>$O-(CH_2)_4-\overset{\oplus}{N}(CH_3)_3 \cdot \overset{\ominus}{I}$ | Rf=0.57 (AcOEt:AcOH: water=3:1:1) Melting Point: 78 - 80°C | 6.26-6.14 (1H,m), 4.32-4.18 (1H,m), 3.80-3.64 (2H,m), 3.41 (9H,s), 3.60-3.35 (8H, m), 2.21 (2H,t), 1.78-1.46 (8H,m), 1.25 (26H,m), 0.94 (3H,t), 0.87 (3H,m) | 3450, 3300, 2930 2850, 1640, 1535, 1465, 1110 (KBr method) | 484, 469, 440, 368, 354 |
| 8 (c) | 5 (c) | $O-(CH_2)_{15}-CH_3$<br>$NHCO-(CH_2)_4-CH_3$<br>$O-(CH_2)_4-\overset{\oplus}{N}(CH_3)_3 \cdot \overset{\ominus}{I}$ | Rf=0.39 (AcOEt:AcOH: water=3:1:1) Melting Point: 58 - 61°C | 6.14-6.04 (1H,m), 4.28-4.14 (1H,m), 3.78-3.63 (2H,m), 3.41 (9H,s), 3.60-3.34 (8H, m), 2.20 (3H,td), 2.0-1.46 (8H,m), 1.25 (30H,m), 0.99-0.8 (6H,m) | 3480, 2940, 2860, 1660, 1460, 1120 ($CHCl_3$ solution) | 512, 497, 483, 468 |
| 8 (d) | 5 (d) | $O-(CH_2)_{15}-CH_3$<br>NHCOØ<br>$O-(CH_2)_5-\overset{\oplus}{N}(CH_3)_3 \cdot \overset{\ominus}{I}$ | Rf=0.71 ($CH_2Cl_2$::AcOH: EtOH:water= 8:4:2:1) Melting Point: 85 - 88°C | 7.9-7.74 (2H,m), 7.6-7.38 (3H,m), 6.7-6.58 (1H,m), 4.5-4.3 (1H,m), 3.75-3.3 (10H,m and 9H,s), 1.95-1.4 (8H,m), 1.4-1.15 (26H,m), 1.88 (3H,t) | 3600-3100, 2910 2840, 1630, 1530, 1460, 1110 (KBr method) | 532($M^+$), 518 439, 402, 398 |

Table 10(2)

| Example No. | Example No. of Starting Materials | Structure of Product | Physical Data: TLC (developing solvent) | Physical Data: NMR | Physical Data: IR | Physical Data: MS |
|---|---|---|---|---|---|---|
| 8 (e) | 5 (f) | 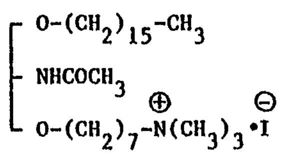 | Rf=0.44 (AcOEt:AcOH: water=3:1:1) | 3.98-3.85 (1H,m), 4.25-4.10 (1H,m), 3.70-3.35 (10H,m), 3.44 (9H,s), 1.99 (3H,s), 1.90-1.20 (38H,m), 0.88 (3H,m) | 3300, 2930, 2860, 1640, 1120 | 498, 483, 454 |
| 8 (f) | 5 (g) | 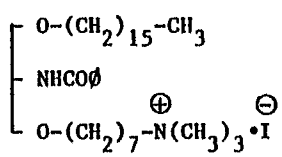 | Rf=0.48 (AcOEt:AcOH: water=3:1:1) Melting Point: 113 - 115°C | 7.86-7.7 (2H,m), 7.60-7.38 (3H,m), 6.65-6.52 (1H,m), 4.50-4.32 (1H,m), 3.72-3.40 (10H,m), 3.42 (9H,s), 1.90-1.17 (38H,m), 0.88 (3H,m) | 3350, 2920, 2850, 1640, 1120 (KBr method) | 560, 545, 516 |
| 8 (g) | 6 (without potassium bicarbonate) | 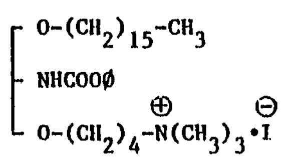 | Rf=0.57 (AcOEt:AcOH: water=3:1:1) Melting Point: 84 - 89°C | 7.44-7.04 (5H,m), 5.66-5.52 (1H,m), 4.10-3.90 (1H,m), 3.75-3.35 (10H,m), 3.2 (9H, s), 1.96-1.44 (6H,m), 1.24 (26H,m), 0.86 (3H,s) | 3450, 3340, 2920, 2850, 1700, 1530, 1480, 1200, 1110 (KBr method) | |
| 8 (h) | 6 (a) (without potassium bicarbonate) | 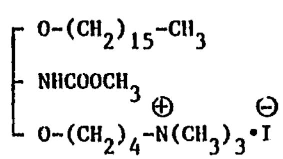 | Rf=0.46 (AcOEt:AcOH: water=3:1:1) Melting Point: 36 - 38°C | 5:22 (1H,d), 4.00-3.82 (1H, m), 3.64 (3H,s), 3.76-3.62 (2H,m), 3.42 (9H,s), 3.78-3.45 (8H,m), 2.00-1.45 (6H, m), 1.24 (26H,m), 0.87 (3H, m) | 3500, 3350, 2930, 2860, 1710, 1530, 1470, 1110 (KBr method) | 441, 398 |

Table 10(3)

| Example No. | Example No. of Starting Materials | Structure of Product | Physical Data: TLC (developing solvent) | Physical Data: NMR | Physical Data: IR | Physical Data: MS |
|---|---|---|---|---|---|---|
| 8 (i) | 6 (b) (without potassium bicarbonate) | 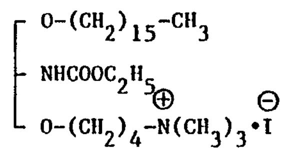 | Rf=0.53 (AcOEt:AcOH: water=3:1:1) Melting Point: 60 - 63°C | 5.18-5.06 (1H,m), 4.15-3.99 (2H,m), 3.96-3.82 (1H,m), 3.74-3.60 (2H,m), 3.40 (9H, s), 3.58-3.34 (8H,m), 1.98-1.44 (6H,m), 1.24 (29H,m), 0.86 (3H,m) | 3480, 3300, 2900 1680, 1530, 1460 (KBr method) | |
| 8 (j) | 6 (c) (without potassium bicarbonate) | 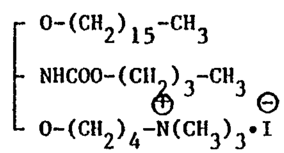 | Rf=0.52 (AcOEt:AcOH: water=3:1:1) | 5.1 (1H,d), 4.10-3.80 (3H, m), 3.76-3.60 (2H,m), 3.42 (9H,s), 3.6-3.35 (8H,m), 2.00-1.34 (10H,m), 1.25 (26H,m), 0.92 (3H,t), 0.87 (3H,m) | 3500, 3300, 2930, 2860, 1710, 1510, 1460, 1110 | 514, 499, 470, 441 |
| 8 (k) | 6 (d) (without potassium bicarbonate) | 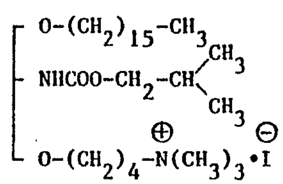 | Rf=0.52 (AcOEt:AcOH: water=3:1:1) | 5.12 (1H,d), 3.96-3.60 (5H, m), 3.42 (9H, s), 3.60-3.34 (8H,m), 2.00-1.44 (7H,m), 1.25 (26H,m), 0.91 (6H,d), 0.87 (3H,m) | 3500, 3300, 2930, 2850, 1710, 1460, 1110 | 514, 499, 485, 470, 441 |
| 8 (l) | 6 (e) | 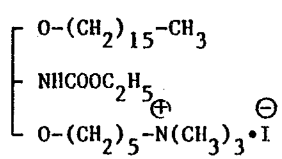 | Rf=0.41 ($CH_2Cl_2$:AcOH: EtOH:water= 8:4:2:1) Melting Point: 63 - 65°C | 5.25-4.9 (1H,m), 4.09 (2H, q), 3.96-3.78 (1H,m), 3.73-3.35 (10H,m and 9H,s), 1.98-1.45 (8H,m), 1.45-1.19 (29H,m), 0.88 (3H,m) | 3600-3100, 2910, 2840, 1690, 1460, 1110 (KBr method) | 500, 499, 453 |

Table 10(4)

| Example No. | Example No. of Starting Materials | Structure of Product | Physical Data | | | |
|---|---|---|---|---|---|---|
| | | | TLC (developing solvent) | NMR | IR | MS |
| 8 (m) | 6 (f) | 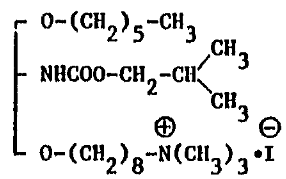 | Rf=0.54 (AcOEt:AcOH: water=3:1:1) | 5.10-4.95 (1H,m), 3.98-3.80 (1H,m), 3.84 (2H,d), 3.70-3.34 (11H,m), 3.46 (9H,s), 2.00-1.20 (21H,m), 0.93 (6H,d) | 3500, 2950, 2870, 1710, 1470, 1120 | 430, 457, 455, 315 |
| 8 (n) | 6 (g) | 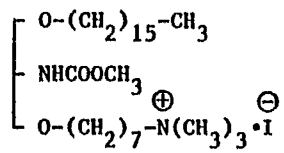 | Rf=0.47 (AcOEt:AcOH: water=3:1:1) Melting Point: 64 - 69°C | 5.12-4.99 (1H,m), 3.93-3.80 (1H,m), 3.66 (3H,s), 3.70-3.34 (8H,m), 3.44 (9H,s), 1.90-1.10 (38H,m), 0.88 (3H,m) | 3300, 2900, 2840, 1690, 1120 (KBr method) | 499, 481 |
| 8 (o) | 6 (h) | 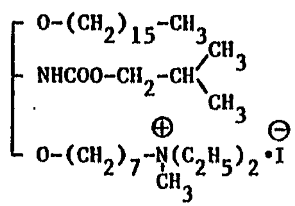 | Rf=0.52 (AcOEt:AcOH: water=3:1:1) | 5.10-4.92 (1H,m), 3.82 (2H,d), 3.94-3.75 (1H,m), 3.61 (4H,q), 3.70-3.33 (10H,m), 3.26 (3H,s), 2.20-1.20 (45H,m), 0.91 (6H,d), 0.87 (3H,m) | 3330, 2920, 2850, 1695, 1460, 1120 | 584, 570, 555, 541, 511 |
| 8 (p) | 7 | 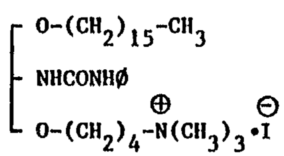 | Rf=0.38 (AcOEt:AcOH: water=3:1:1) Melting Point: 70 - 74°C | 8.36 (1H,bs), 7.53 (2H,d), 7.18 (2H,t), 6.90 (1H,t), 6.24 (1H,d), 4.28-4.10 (1H,m), 3.72-3.30 (10H,m), 3.13 (9H,s), 2.04-1.40 (6H,m), 1.24 (26H,m), 0.86 (3H,m) | 3300, 2930, 2830, 1680, 1600, 1540 ($CHCl_3$ solution) | 533, 518, 489, 441 |

Table 10(5)

| Example No. | Example No. of Starting Materials | Structure of Product | Physical Data | | | |
|---|---|---|---|---|---|---|
| | | | TLC (developing solvent) | NMR | IR | MS |
| 8 (q) | 7 (a) | ┌ $O-(CH_2)_{15}-CH_3$<br>├ $NHCONHCH_3$<br>└ $O-(CH_2)_4-\overset{\oplus}{N}(CH_3)_3 \cdot \overset{\ominus}{I}$ | Rf=0.31 (AcOEt:AcOH: water=3:1:1) Melting Point: 160 - 163°C | 6.95-5.79 (2H,m), 4.13-3.90 (1H,m), 3.36 (9H,s), 3.80-3.30 (10H,m), 2.71 (3H,d), 2.05-1.40 (6H,m), 1.24 (26H,m), 0.86 (3H,m) | 3400, 3350, 2920, 2840, 1630, 1570, 1460 (KBr method) | 471, 456, 441, 427 |
| 8 (r) | 7 (b) (without potassium bicarbonate) | ┌ $O-(CH_2)_{15}-CH_3$<br>├ $NHCONH-(CH_2)_2-CH_3$<br>└ $O-(CH_2)_4-\overset{\oplus}{N}(CH_3)_3 \cdot \overset{\ominus}{I}$ | Rf=0.58 (AcOEt:AcOH: water=3:1:1) Melting Point: 126 - 127°C | 5.86-5.68 (2H,m), 4.14-3.95 (1H,m), 3.81-3.65 (2H,m), 3.65-3.48 (4H,m), 3.36 (9H, s), 3.48-3.30 (4H,m), 3.22-2.98 (2H,m), 2.14-1.88 (2H, m), 2.61-1.40 (4H,m), 1.25 (26H,m), 0.90 (3H,t), 0.87 (3H,m) | 3450, 3320, 2920, 2860, 1620, 1580, 1470, 1270, 1110 (KBr method) | 499, 484, 470, 455 |
| 8 (s) | 7 (c) | ┌ $O-(CH_2)_{15}-CH_3$<br>├ $NHCONH-(CH_2)_3-CH_3$<br>└ $O-(CH_2)_4-\overset{\oplus}{N}(CH_3)_3 \cdot \overset{\ominus}{I}$ | Rf=0.37 (AcOEt:AcOH: water=3:1:1) Melting Point: 61 - 64°C | 5.82-5.65 (2H,m), 4.10-3.95 (1H,m), 3.80-3.66 (2H,m), 3.63-3.48 (4H,m), 3.37 (9H, s), 3.48-3.32 (4H,m), 3.21-3.0 (2H,m), 2.05-1.88 (2H, m), 1.80-1.65 (2H,m), 1.62-1.20 (30H,m), 0.90 (3H,t), 0.88 (3H,m) | 3500, 3300, 2900, 1620, 1460, 1380 (KBr method) | 513, 498, 483, 469 |
| 8 (t) | 7 (d) | ┌ $O-(CH_2)_{15}-CH_3$<br>├ $NHCONHCH_3$<br>└ $O-(CH_2)_7-\overset{\oplus}{N}(CH_3)_3 \cdot \overset{\ominus}{I}$ | Rf=0.41 (AcOEt:AcOH: water=3:1:1) Melting Point: 73 - 77°C | 5.62-5.50 (1H,m), 5.24 (1H, bd), 4.00-3.85 (1H,m), 3.76-3.60 (2H,m), 3.56-3.35 (8H,m), 3.41 (9H,s), 2.73 (3H,d), 2.00-1.20 (38H,m), 0.88 (3H,m) | 3350, 2920, 2850, 1640, 1120 (KBr method) | |

Reference Example 12

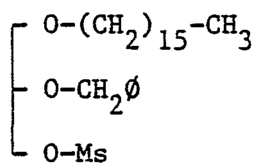

By the same procedure as described in Reference Example 8, the title compound having the following physical data was prepared.

Starting Material: the 3-hydroxy compound prepared in Reference Example 5;

TLC ($CHCl_3$:acetone=10:1): Rf=0.86;

NMR: δ=7.2 (5H, s), 4.6 (2H, s), 4.4-4.1 (2H, m), 3.9-3.2 (5H, m), 2.93 (3H, s), 1.8-0.6 (31H, m);

IR: ν=2930, 2850, 1470, 1360, 1120 $cm^{-1}$;

MS: m/e=484 ($M^+$), 393.

Reference Example 13

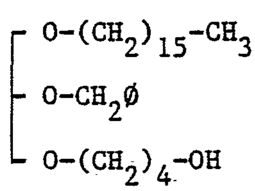

Under an atmosphere of nitrogen, to a suspension of 2.5 g of sodium hydride (content: 64.1%) in 200 ml of dry DMF was

added 4 g of 1,4-butanediol and the mixture was stirred for 10 minutes at room temperature. To the mixture was added a solution of 14.5 g of the mesylate compound (prepared in Reference Example 12) in 100 ml of dry DMF and the mixture was stirred for 2 hours at 70°C. To the reaction mixture was added 4 ml of water and the mixture was concentrated under reduced pressure. To the residue thus obtained was added 500 ml of AcOEt, and the solution was washed with water and a sat. aq. solution of NaCl, successively, dried over anhydrous sodium sulfate and concentrated under reduced pressure. The residue was purified by column chromatography on silica gel (eluent: a mixture of AcOEt and n-hexane) to give 8.4 g of the title compound having the following physical data:

TLC ($CHCl_3$:acetone=10:1): Rf=0.43;

NMR: $\delta$=7.2 (5H, bs), 4.6 (2H, s), 3.9-3.2 (11H, m), 2.75 (1H, bs), 2.0-0.8 (35H, m);

IR: $\nu$=3400, 2930, 2850, 1460, 1110 $cm^{-1}$;

MS: m/e=478 ($M^+$), 406, 372, 297, 282.

Reference Example 14

$O-(CH_2)_{15}-CH_3$

$O-CH_2Ø$

$O-(CH_2)_4-OTHP$

By the same procedure as described in Reference Example 3, the title compound having the following physical data was prepared.

Starting Material: the hydroxy compound prepared in Reference Example 13;

TLC (AcOEt:n-hexane=1:5): Rf=0.32;

NMR: δ=7.23 (5H, bs), 4.66 (2H, s), 4.66-4.43 (1H, m), 3.9-3.1 (13H, m), 1.8-0.7 (41H, m);

IR: ν=2930, 2850, 1450, 1120 $cm^{-1}$;

MS: m/e=562 ($M^+$), 478, 461.

Reference Example 15

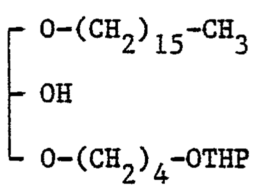

Under an atmosphere of hydrogen, a mixture of 8.6 g of the 2-O-benzylglycerol compound (prepared in Reference Example 14), 6 g of palladium on carbon (content: 10%) and 100 ml of EtOH was stirred for 2 days at 50°C. The catalyst was filtered off and the filtrate was concentrated under reduced pressure to give 7.8 g of the title compound as crude product, having the following physical data:

TLC ($CHCl_3$:acetone=10:1): Rf=0.38;

NMR: δ=4.70-4.45 (1H, m), 4.2-3.1 (13H, m), 2.0-0.7 (41H, m).

Reference Example 16

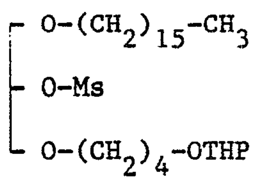

By the same procedure as described in Reference Example 11, the title compound having the following physical data was prepared.

Starting Material: the 2-hydroxy compound prepared in Reference Example 15;

TLC ($CHCl_3$:acetone=10:1): Rf=0.71;

NMR: δ= 4.93-4.4 (2H, m), 3.95-3.1 (12H, m), 3.05 (3H, s), 2.0-0.7 (31H, m).

Reference Example 17

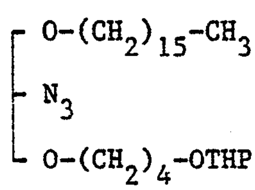

By the same procedure as described in Example 1, the title compound having the following physical data was prepared.

Starting Material: the 2-O-mesylglycerol compound prepared in Reference Example 16;

TLC (AcOEt:n-hexane=1:5): Rf=0.49;

NMR: δ=4.66-4.36 (1H, m), 4.0-3.1 (13H, m), 2.0-0.7 (41H, m);

IR: ν=2940, 2850, 2150, 2090, 1460, 1120 $cm^{-1}$;

MS: m/e=469, 412, 396.

Reference Example 18

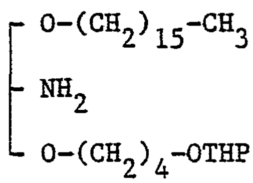

By the same procedure as described in Example 3, the title compound having the following physical data was prepared.

Starting Material: the 2-azidoglycerol compound prepared in Reference Example 17;

TLC ($CHCl_3$:MeOH=10:1): Rf=0.53;

NMR: δ= 4.63-4.4 (1H, m), 4.0-3.0 (13H, m), 2.0-0.7 (41H, m);

IR: ν=2930, 2850, 1460, 1120 $cm^{-1}$.

Reference Example 19

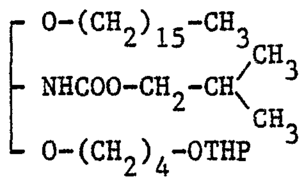

By the same procedure as described in Example 6, the title compound having the following physical data was prepared.

Starting Material: the 2-aminoglycerol compound prepared in Reference Example 18 and isobutyl chloroformate;

TLC (AcOEt:n-hexane=1:5): Rf=0.31;

NMR: δ=5.15-4.8 (1H, m), 4.7-4.43 (1H, m), 4.1-3.1 (15H, m), 2.0-0.7 (42H, m and 6H, d);

IR: ν=2920, 2850, 1720, 1500, 1460, 1110 $cm^{-1}$.

Reference Example 20

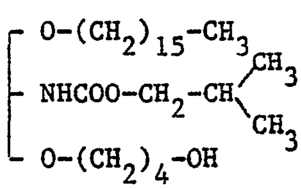

By the same procedure as described in Reference Example 5, the title compound having the following physical data was prepared.

Starting Material: the 3-O-(tetrahydropyran-2-yl)glycerol compound prepared in Reference Example 19;

TLC ($CHCl_3$:MeOH=10:1): Rf=0.66;

NMR: δ=5.15-4.8 (1H, m), 4.0-3.1 (11H, m), 2.2-0.7 (38H, m), 0.9 (6H, d).

Reference Example 21

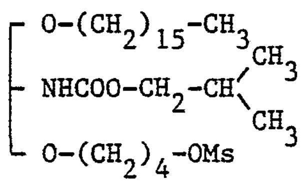

By the same procedure as described in Reference Example 8, the title compound having the following physical data was prepared.

Starting Material: the hydroxy compound prepared in Reference Example 20;

TLC (AcOEt:n-hexane=1:2): Rf=0.33;

NMR: δ=5.15-4.8 (1H, m), 4.2 (2H, t), 4.0-3.2 (11H, m), 3.0 (3H, s), 2.1-0.7 (36H, m), 0.93 (6H, d);

IR: ν=2930, 2860, 1720, 1510, 1470, 1360, 1180, 1120 $cm^{-1}$.

Example 9

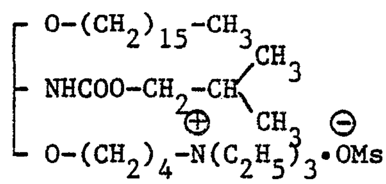

A mixture of 113 mg of the mesylate compound (prepared in Reference Example 21), 0.1 ml of triethylamine and 3 ml of DMF was stirred overnight at 70°C and then the reaction mixture was concentrated under reduced pressure. The residue was purified by column chromatography on silica gel (eluent: a mixture of $CHCl_3$ and MeOH, and a mixture of $CHCl_3$, MeOH and water, successively) to give 51 mg of the title compound having the following physical data:

Melting Point: 30 - 31°C;

TLC (AcOEt:AcOH:water=3:1:1): Rf=0.65;

NMR: δ=5.28-5.17 (1H, m), 3.96-3.72 (3H, m), 3.65-3.28 (16H, m), 2.76 (3H, s), 2.10-1.46 (7H, m), 1.36 (9H, t), 1.25 (26H, m), 0.92 (6H, d), 0.88 (3H, m);

IR: ν=3450, 2930, 2860, 1700, 1460, 1190, 1120 $cm^{-1}$;

MS: m/e=542, 527, 513, 469.

Reference Example 22

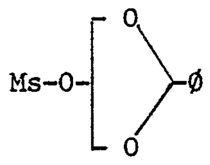

By the same procedure as described in Reference Example 11, the title compound as white crystal having the following physical data was prepared.

Starting Material: 1,3-O-benzylideneglycerol [the compound is described in J. Amer. Chem. Soc., 50, 2235 (1928)];

TLC (AcOEt:n-hexane=1:1): Rf=0.39;

NMR: δ=7.50-7.10 (5H, m), 5.40 (1H, s), 4.62-4.42 (1H, m), 4.30-4.10 (4H, m), 3.00 (3H, s);

IR (KBr method): ν=1330, 1170, 940 $cm^{-1}$.

Reference Example 23

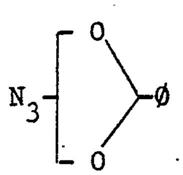

By the same procedure as described in Example 1, the title compound having the following physical data was prepared.

Starting Material: the mesylate compound prepared in Reference Example 22;

TLC (benzene): Rf=0.51;

NMR: δ=7.40-7.10 (5H, m), 5.30 (1H, s), 4.40-4.00 (2H, m), 3.80-3.50 (3H, m);

IR: ν=2100, 1280, 1100, 970, 750, 700 $cm^{-1}$.

Reference Example 24

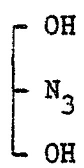

By the same procedure as described in Reference Example 2, the title compound having the following physical data was prepared.

Starting Material: the benzylidene compound prepared in Reference Example 23;

TLC (diethyl ether): Rf=0.41;

IR: ν=3350, 2150, 1270, 1050 $cm^{-1}$.

Reference Example 25

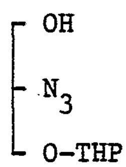

By the same procedure as described in Reference Example 3, the title compound having the following physical data was prepared.

Starting Material: the dihydroxy compound prepared in Reference Example 24;

TLC (AcOEt:n-hexane=1:5): Rf=0.10;

NMR: δ=4.60-4.45 (1H, m), 4.00-3.30 (7H, m), 2.40-2.10 (1H, m);

IR: ν=3450, 2250, 1125, 1070, 1030 $cm^{-1}$.

Reference Example 26

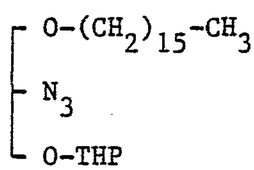

By the same procedure as described in Reference Example 4, the title compound having the following physical data was prepared.

Starting Material: the hydroxy compound prepared in Reference

Example 25;

TLC (AcOEt:n-hexane=1:10): Rf=0.29;

NMR: δ=4.70-4.60 (1H, m), 3.94-3.68 (3H, m), 3.60-3.40 (6H, m), 1.80-1.45 (8H, m), 1.26 (29H, s), 0.88 (3H, bt);

IR: ν=2225, 2200, 1470, 1270, 1120, 1040 $cm^{-1}$;

MS: m/e=425 ($M^+$), 397, 367.

Reference Example 27

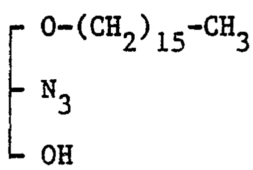

By the same procedure as described in Reference Example 5, the title compound having the following physical data was prepared.

Starting Material: the tetrahydropyran-2-yloxy compound prepared in Reference Example 26;

TLC (AcOEt:n-hexane=1:5): Rf=0.24;

NMR: δ=3.9-3.2 (7H, m), 2.3-1.9 (1H, m), 1.9-0.7 (31H, m);

IR ($CHCl_3$ solution): ν=3500, 2940, 2860, 2140, 2120, 1460, 1120 $cm^{-1}$;

MS: m/e=282, 255, 225.

Reference Example 28

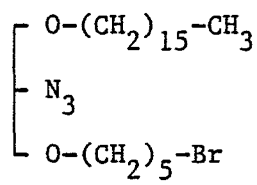

By the same procedure as described in Reference Example 6, the title compound having the following physical data was prepared.

Starting Material: the 3-hydroxy compound prepared in Reference Example 27 and 1,5-dibromopentane;

TLC (AcOEt:n-hexane=1:5): Rf=0.64;

MS: m/e=461, 446, 382.

By the same procedure as described in Reference Example 28, the compounds having the following physical data in Table 11, were prepared.

Table 11

| Reference Example No. | Starting Materials | Structure of Product | Physical Data: TLC (AcOEt:n-hexane =1:5) | Physical Data: MS |
|---|---|---|---|---|
| 28 (a) | Compound prepared in RE27 and $Br-(CH_2)_6-Br$ | $O-(CH_2)_{15}-CH_3$<br>$N_3$<br>$O-(CH_2)_6-Br$ | Rf=0.63 | 475, 396 |
| 28 (b) | Compound prepared in RE27 and $Br-(CH_2)_7-Br$ | $O-(CH_2)_{15}-CH_3$<br>$N_3$<br>$O-(CH_2)_7-Br$ | Rf=0.61 | 489, 410 |
| 28 (c) | Compound prepared in RE27 and $Br-(CH_2)_8-Br$ | $O-(CH_2)_{15}-CH_3$<br>$N_3$<br>$O-(CH_2)_8-Br$ | Rf=0.65 | 503, 424 |
| 28 (d) | Compound prepared in RE27 and $Br-(CH_2)_9-Br$ | $O-(CH_2)_{15}-CH_3$<br>$N_3$<br>$O-(CH_2)_9-Br$ | Rf=0.60 | 517, 438 |
| 28 (e) | Compound prepared in RE27 and $Br-(CH_2)_{10}-Br$ | $O-(CH_2)_{15}-CH_3$<br>$N_3$<br>$O-(CH_2)_{10}-Br$ | Rf=0.55 | 531, 452 |

"RE" represents "Reference Example".

Reference Example 29

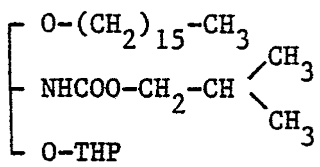

Under an atmosphere of hydrogen, a mixture of 2.16 g of the 2-azidoglycerol compound (prepared in Reference Example 26), 1.1 g of palladium on carbon (content: 10%) and 30 ml of EtOH was stirred for 4 hours at room temperature. The catalyst was filtered off and the filtrate was concentrated under reduced pressure. The residue thus obtained was dissolved in 36 ml of $CH_2Cl_2$ and thereto was added 3.6 ml of triethylamine. To the solution was added dropwise 1.036 g of isobutyl chloroformate with stirring under cooling with ice-bath. The reaction mixture was concentrated under reduced pressure at a low temperature and the residue thus obtained was extracted with AcOEt. The extract was washed with water, dried over anhydrous sodium sulfate and concentrated under reduced pressure. The residue was purified by column chromatography on silica gel (eluent: a mixture of AcOEt and n-hexane) to give 1.78 g of the title compound having the following physical data:

TLC (AcOEt:n-hexane=1:5): Rf=0.33;

NMR: $\delta$=4.65-4.50 (1H, m), 3.35 (2H, d), 4.00-3.20 (9H, m), 0.98 (6H, d);

IR: $\nu$=3350, 1730, 1505, 1470, 1120, 1070, 1040 $cm^{-1}$;

MS: m/e=499 ($M^+$), 469, 427, 415.

By the same procedure as described in Reference Example 29, the compounds having the following physical data in Table 12, were prepared.

Table 12(1)

| Reference Example No. | Reference Example No. of Starting Materials | Structure of Product | Physical Data | | | |
|---|---|---|---|---|---|---|
| | | | TLC (developing solvent) | NMR | IR | MS |
| 29 (a) | 28 | 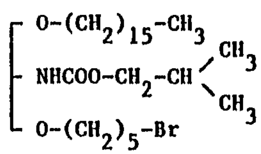 | Rf=0.36 (AcOEt:n-hexane =1:5) | 5.1-4.7 (1H,m), 3.8 (2H, d), 4.0-3.0 (11H,m), 2.1-0.7 (44H,m) | 2930, 2860, 1720 1460, 1120 | 563($M^+$), 548, 490, 462, 446, 398, 384 |
| 29 (b) | 28 (a) | 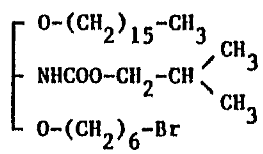 | Rf=0.37 (AcOEt:n-hexane =1:5) | 5.1-4.8 (1H,m), 4.0-3.0 (11H,m), 3.8 (2H,d), 2.1-0.7 (46H,m) | 2930, 2860, 1720, 1460, 1120 | 577($M^+$), 562, 498, 399, 384, |
| 29 (c) | 28 (b) | 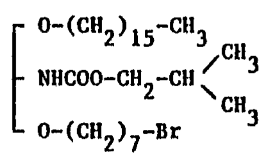 | Rf=0.39 (AcOEt:n-hexane =1:5) | 5.1-4.8 (1H,m), 4.0-3.0 (11H,m), 3.8 (2H,d), 2.0-0.6 (48H,m) | 2930, 2850, 1720, 1460, 1120 | 591($M^+$), 512, 474, 399, 384 |
| 29 (d) | 28 (c) | 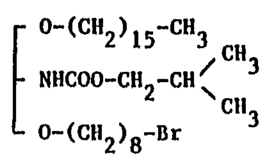 | Rf=0.41 (AcOEt:n-hexane =1:5) | 5.1-4.8 (1H,m), 4.0-3.1 (11H,m), 3.8 (2H,d), 2.0-0.6 (50H,m) | 2930, 2850, 1720, 1460, 1120 | 605($M^+$), 526, 488, 399, 384 |

Table 12(2)

| Reference Example No. | Reference Example No. of Starting Materials | Structure of Product | Physical Data | | | |
|---|---|---|---|---|---|---|
| | | | TLC (developing solvent) | NMR | IR | MS |
| 29 (e) | 28 (d) | 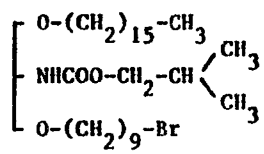 | Rf=0.46 (AcOEt:n-hexane =1:5) | 5.1-4.8 (1H,m), 4.0-3.0 (11H,m), 3.8 (2H,d), 2.1-0.7 (52H,m) | 2930, 2850, 1720, 1460, 1120 | 619($M^+$), 540, 502, 399, 384 |
| 29 (f) | 28 (e) | 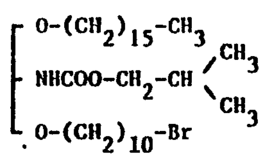 | Rf=0.49 (AcOEt:n-hexane =1:5) | 5.0-4.7 (1H,m), 4.0-3.1 (11H,m), 3.8 (2H,d), 2.0-0.7 (54H,m) | 2930, 2850, 1720, 1460, 1120 | 633($M^+$), 554, 516, 399, 384 378 |

Reference Example 30

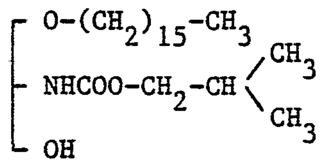

By the same procedure as described in Reference Example 5, the title compound having the following physical data was prepared.

Starting Material: the tetrahydropyran-2-yloxy compound prepared in Reference Example 29;

TLC (AcOEt:n-hexane=1:5): Rf=0.09;

NMR: δ=5.50-5.10 (1H, m), 3.35 (2H, d), 3.35-3.25 (7H, m), 3.10-2.80 (1H, m), 0.98 (6H, d);

IR (KBr method): ν=3575, 3330, 1680, 1540, 1280, 1100 $cm^{-1}$;

MS: m/e=415 ($M^+$), 384, 328.

Reference Example 31

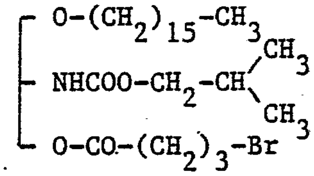

To a mixture of 120 mg of 4-bromobutanoic acid, 98 mg of isobutyl chloroformate and 5 ml of $CH_2Cl_2$ was added dropwise 0.25 ml of triethylamine under cooling with ice-bath. To the mixture was added a solution of 150 mg of the 3-hydroxy compound (prepared in Reference Example 30) in 2 ml of $CH_2Cl_2$ at room temperature and the mixture was stirred for 12 hours at room temperature and then concentrated under reduced pressure. The residue was purified by column chromatography on silica gel (eluent: a mixture of AcOEt and n-hexane) to give 82 mg of the title compound having the following physical data:

TLC (AcOEt:n-hexane=1:5): Rf=0.39;

NMR: $\delta$=4.90 (1H, bd), 4.20-3.90 (3H, m), 3.77 (2H, d), 3.50-3.20 (6H, m), 0.88 (6H, d);

IR: $\nu$=3350, 1730, 1610, 1470 $cm^{-1}$;

MS: m/e=565, 563 ($M^+$), 550, 548.

By the same procedure as described in Reference Example 31, the compounds having the following physical data in Table 13, were prepared.

Table 13

| Reference Example No. | Starting Materials | Structure of Product | Physical Data | | | |
|---|---|---|---|---|---|---|
| | | | TLC (developing solvent) | NMR | IR | MS |
| 31 (a) | Compound prepared in RE30 and $Br-(CH_2)_4-COOH$ | 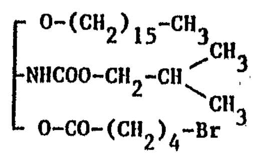 | Rf=0.27 (AcOEt:n-hexane =1:5) | 4.95 (1H,bd), 4.15-3.90 (3H, m), 3.80 (2H,d), 3.55-3.20 (6H,m), 2.30 (2H,bt), 0.92 (6H,d) | 3330, 1730, 1695, 1550, 1460, 1280 | 579, 577($M^+$), 498 |
| 31 (b) | Compound prepared in RE30 and $Br-(CH_2)_5-COOH$ | 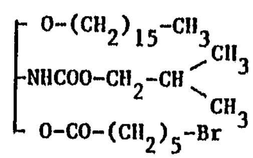 | Rf=0.45 (AcOEt:n-hexane =1:3) | 5.05-4.80 (1H,m), 4.25-4.00 (3H,m), 3.80 (2H,d), 3.50-3.20 (6H,m), 2.30 (2H,t), 0.95 (6H,d) | 3350, 1730, 1510, 1460, 1230 | 593, 591($M^+$), 520, 518 |
| 31 (c) | Compound prepared in RE30 and $THPO-(CH_2)_6-COOH$ | 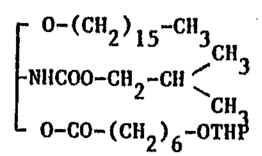 | Rf=0.44 (AcOEt:n-hexane =1:3) | 5.05-4.80 (1H,m), 4.60-4.40 (1H,m), 4.30-3.95 (3H,m), 3.82 (2H,d), 3.70-3.20 (8H,m), 2.30 (2H,bt), 0.90 (6H,d) | 3350, 1730, 1610, 1470, 1120, 1080, 1040 | |
| 31 (d) | Compound prepared in RE30 and $THPO-(CH_2)_7-COOH$ | 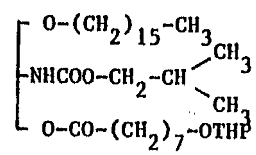 | Rf=0.51 (AcOEt:n-hexane =1:3) | 5.10-4.80 and 4.60-4.40 (1H,m), 4.20-3.95 (3H,m), 3.80 (2H,d), 3.70-3.15 (8H,m), 2.28 (2H,bt), 0.90 (6H,d) | 3350, 1730, 1520, 1470, 1120, 1080, 1040 | |

"RE" represents "Reference Example".

Reference Example 32

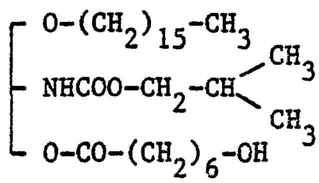

A mixture of 313 mg of the tetrahydropyran-2-yloxy compound [prepared in Reference Example 31(c)] and 5 ml of a mixed solvent of AcOH, water and THF (3:1:1) was stirred for one hour at 60°C. The reaction mixture was poured into an aq. solution of $NaHCO_3$ and neutralized with AcOH and then extracted with AcOEt. The extract was dried over anhydrous sodium sulfate and concentrated under reduced pressure. The residue was purified by column chromatography on silica gel (eluent: a mixture of AcOEt and n-hexane) to give 252 mg of the title compound having the following physical data:

TLC (AcOEt:n-hexane=1:3): Rf=0.26;

NMR: δ=5.10-4.70 (1H, m), 4.20-3.90 (3H, m), 3.80 (2H, d), 3.70-3.20 (6H, m), 2.30 (2H, t), 0.90 (6H, d);

IR: ν=3500, 3350, 1730, 1710, 1620, 1470, 1250, 1080 $cm^{-1}$;

MS: m/e=543 ($M^+$), 528, 525, 513.

By the same procedure as described in Reference Example 32, the compound having the following physical data was prepared.

(a) 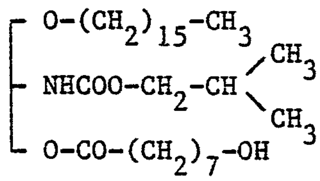

Starting Material: the tetrahydropyran-2-yloxy compound prepared in Reference Example 31 (d);

TLC (AcOEt:n-hexane=1:1): Rf=0.50;

NMR: δ=5.20-4.80 (1H, m), 4.30-4.00 (3H, m), 3.80 (2H, d), 3.70-3.20 (6H, m), 2.30 (2H, t), 0.95 (6H, d);

IR: ν=3450, 3325, 1730, 1710, 1620, 1460, 1240, 1070 $cm^{-1}$

MS: m/e=557 ($M^+$), 542, 539, 527.

Reference Example 33

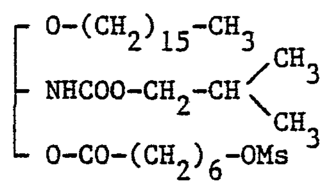

By the same procedure as described in Reference Example 8, the title compound having the following physical data was prepared.

Starting Material: the hydroxy compound prepared in Reference Example 32;

TLC (AcOEt:benzene=1:3): Rf=0.52;

NMR: δ=5.15-4.85 (1H, m), 4.35-4.00 (5H, m), 3.85 (2H, d), 3.60-3.30 (4H, m), 2.35 (2H, t), 0.98 (6H, d);

IR: ν=3375, 1725, 1520, 1470, 1360, 1180 $cm^{-1}$;

MS: m/e=621 ($M^+$), 564.

By the same procedure as described in Reference Example 33, the compound having the following physical data was prepared.

(a) 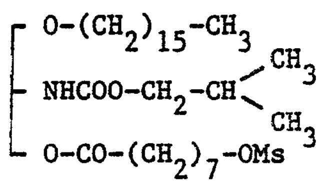

Starting Material: the hydroxy compound prepared in Reference Example 32 (a);

TLC (AcOEt:benzene=1:2): Rf=0.57;

NMR: δ=5.20-4.80 (1H, m), 4.40-4.00 (5H, m), 3.90 (2H, d), 3.65-3.30 (4H, m), 3.05 (3H, s), 2.40 (2H, bt), 0.98 (6H, d);

IR: ν=3350, 1730, 1620, 1465, 1250, 1175 $cm^{-1}$;

MS: m/e=636, 562, 556.

Example 10

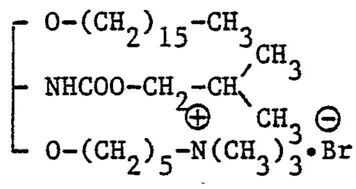

A mixture of 74 mg of the bromo compound [prepared in Reference Example 29 (a)], 3 ml of a 30% aqueous solution of trimethylamine and 9 ml of DMF was stirred for 4 hours at 50°C and then the reaction mixture was concentrated under reduced pressure. The residue was purified by column chromatography on silica gel (eluent: a mixture of $CHCl_3$ and MeOH, and a mixture of $CHCl_3$, MeOH and water, successively) to give 41 mg of the title compound having the following physical data:

TLC (AcOEt:AcOH:water=3:1:1): Rf=0.45;

NMR ($CDCl_3$ + $CD_3OD$ solution):

δ=5.55-5.2 (1H, m), 3.98-3.77 (3H, m), 3.58-3.35 (10H, m), 3.21 (9H, s), 2.00-1.39 (9H, m), 1.26 (26H, m), 0.93 (6H, d), 0.88 (3H, m);

IR: ν=3400, 2920, 2850, 1700, 1460, 1120 $cm^{-1}$;

MS: m/e=528, 514, 455, 453.

By the same procedure as described in Example 10, the compounds having the following physical data in Table 14, were prepared.

Table 14(1)

| Example No. | Reference Example No. of Starting Materials | TLC Structure of Product | Physical Data (developing solvent) | NMR | IR | MS |
|---|---|---|---|---|---|---|
| 10 (a) | 29 (b) | 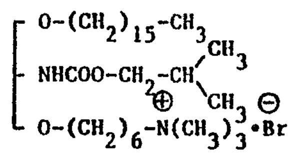 | Rf=0.46 (AcOEt:AcOH: water=3:1:1) | 5.27-5.15 (1H,m), 3.95-3.75 (3H,m), 3.58-3.35 (10H,m), 3.27 (9H,s), 2.00-1.36 (11H, m), 1.26 (26H,m), 0.93 (6H, d), 0.88 (3H,m) ($CDCl_3$ + $CD_3OD$ solution) | 3350, 2940, 2860, 1710, 1470, 1120 | 542, 527, 498, 469 |
| 10 (b) | 29 (c) | 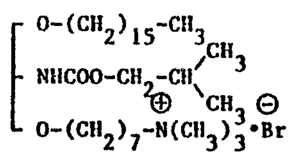 | Rf=0.47 (AcOEt:AcOH: water=3:1:1) | 5.08-4.96 (1H,m), 3.94-3.78 (3H,m), 3.68-3.30 (10H,m), 3.46 (9H,s), 2.00-1.18 (39H, m), 0.93 (6H,d), 0.88 (3H,m) | 3400, 2930, 2860, 1710, 1470, 1120 | 556, 541, 512, 483 |
| 10 (c) | 29 (d) | 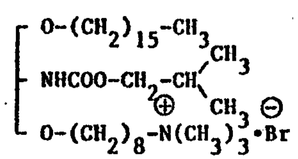 | Rf=0.48 (AcOEt:AcOH: water=3:1:1) Melting Point: 31°C | 5.07-4.97 (1H,m), 3.93-3.78 (3H,m), 3.65-3.32 (10H,m), 3.46 (9H,s), 2.00-1.46 (7H, m), 1.25 (34H,m), 0.91 (6H, d), 0.87 (3H,m) | 3420, 2920, 2850, 1690, 1460, 1120 | 570, 555, 526, 497 |
| 10 (d) | 29 (e) | 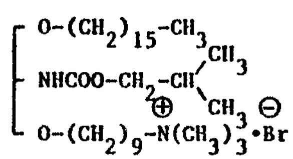 | Rf=0.49 (AcOEt:AcOH: water=3:1:1) Melting Point: 32 - 40°C | 5.09-4.96 (1H,m), 3.96-3.78 (3H,m), 3.67-3.33 (10H,m), 3.47 (9H,s), 2.00-1.47 (7H, m), 1.27 (36H,m), 0.92 (6H, d), 0.88 (3H,m) | 3330, 2920, 2850, 1690, 1540, 1460 1120 | 584, 569, 540, 511 |

Table 14(2)

| Example No. | Reference Example No. of Starting Materials | Structure of Product | Physical Data: TLC (developing solvent) | NMR | IR | MS |
|---|---|---|---|---|---|---|
| 10 (e) | 29 (f) | 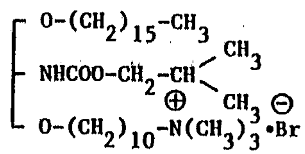 | Rf=0.51 (AcOEt:AcOH: water=3:1:1) Melting Point: 38 - 42°C | 5.08-4.94 (1H,m), 3.95-3.78 (3H,m), 3.62-3.28 (10H,m), 3.45 (9H,s), 2.00-1.45 (7H, m), 1.25 (38H,m), 0.91 (6H, d), 0.87 (3H,m) | 3330, 2930, 2850, 1690, 1540, 1470, 1120 | 598, 583, 554, 525 |
| 10 (f) | 31 | 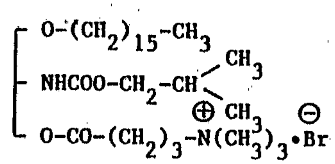 | Rf=0.43 (AcOEt:AcOH: water=3:1:1) | 4.35-3.96 (3H,m), 3.84 (2H, d), 3.60-3.40 (6H,m), 2.54 (2H,t), 0.96 (6H,d), 0.89 (3H,bt) ($CDCl_3$ + $CD_3OD$ solution) | 3300, 1730-1710, 1510, 1470 (KBr method) | 528, 513, 455 |
| 10 (g) | 31 (a) | 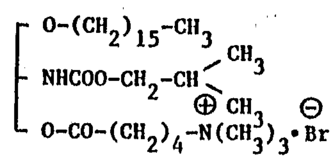 | Rf=0.32 (AcOEt:AcOH: water=3:1:1) | 4.30-4.10 (2H,m), 4.10-3.95 (1H,m), 3.84 (2H,d), 3.55-3.40 (6H,m), 2.48 (2H,t), 0.95 (6H,d), 0.89 (3H,bt) ($CDCl_3$ + $CD_3OD$ solution) | 3375, 1730-1700, 1510, 1470 | 542, 527 |
| 10 (h) | 31 (b) | 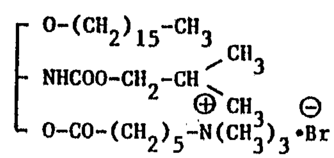 | Rf=0.42 (AcOEt:AcOH: water=3:1:1) | 4.30-4.10 (2H,m), 4.10-3.95 (1H,m), 3.84 (2H,d), 3.55-3.40 (6H,m), 3.16 (9H,s), 2.42 (2H,t), 0.96 (6H,d), 0.89 (3H,bt) ($CDCl_3$ + $CD_3OD$ solution) | 3300, 1730, 1695, 1565, 1470, 1280 (KBr method) | 556, 541 |

Table 14(3)

| Example No. | Reference Example No. of Starting Materials | Structure of Product | Physical Data | | | |
|---|---|---|---|---|---|---|
| | | | TLC (developing solvent) | NMR | IR | MS |
| 10 (i) | 33 | 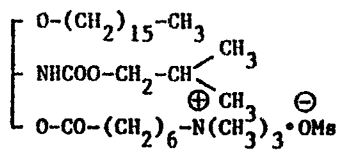 | Rf=0.42 (AcOEt:AcOH: water=3:1:1) | 4.26-4.10 (2H,m), 4.06-3.96 (1H,m), 3.82 (2H,d), 3.54-3.40 (4H,m), 3.38-3.26 (2H, m), 3.14 (9H,m), 2.74 (3H, s), 2.37 (2H,t), 0.95 (6H, d), 0.88 (3H,t)<br>($CDCl_3$ + $CD_3OD$ solution) | 3325, 1740, 1690 1545, 1470, 1200<br>(KBr method) | 570, 555, 497 |
| 10 (j) | 33 (a) | 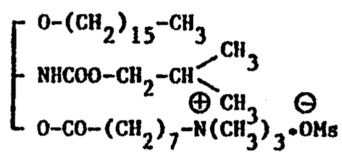 | Rf=0.48 (AcOEt:AcOH: water=3:1:1) | 4.22-4.12 (2H,m), 4.10-3.96 (1H,m), 3.84 (2H,d), 3.54-3.42 (4H,m), 3.40-3.26 (2H,m), 3.14 (9H,s), 2.76 (3H,s), 2.36 (2H,t), 0.96 (6H,d), 0.89 (3H,t)<br>($CDCl_3$ + $CD_3OD$ solution) | 3300, 1730, 1690, 1540, 1465, 1200<br>(KBr method) | 584, 569, 511 |

Example 11

5 g of (2RS)-1-O-hexadecyl-2-dehydroxy-2-butoxycarbonylamino-3-O-[4-(N,N,N-trimethylammonio)butyl]glycerol iodide, 200 mg of cellulose calcium gluconate (disintegrator), 100 mg of magnesium stearate (lubricant) and 9.7 g of crystalline cellulose were mixed and punched out in a usual manner to obtain tablets each containing 50 mg of the active ingredient.

CLAIMS:

1. A glycerol derivative of the general formula:

$$\begin{array}{ll} 1 & \ulcorner O-R^1 \\ 2 & \vdash R^2 \\ 3 & \llcorner A-R^3-R^4 \end{array} \qquad \text{I}$$

[wherein A represents an oxygen atom or a carbonyloxy group (i.e. -O-CO-, in which the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents an azido group ($-N_3$ group), an amino group ($-NH_2$ group) or a group of the general formula: $-NHCOR^5$, $-NHCOOR^5$ or $-NHCONHR^5$, in which $R^5$ represents an alkyl group of 1 to 10 carbon atoms or a phenyl group, with the proviso that, when A represents a carbonyloxy group, $R^2$ does not represent an amino group, $R^3$ represents an alkylene group of 1 to 12 carbon atoms and $R^4$ represents an amino group, or a group of the general formula: $-NHCOR^6$, $-NHR^7$, $-N(R^7)_2$ or $-\overset{\oplus}{N}(R^7)_3 \cdot \overset{\ominus}{Y}$, in which $R^6$ represents an alkyl group of 1 to 6 carbon atoms or a phenyl group, $R^7$ represents an alkyl group of 1 to 8 carbon atoms, with the proviso that, when $R^4$ represents a group of the formula: $-N(R^7)_2$ or $-\overset{\oplus}{N}(R^7)_3 \cdot \overset{\ominus}{Y}$, two or more of $R^7$ may be same or different to each other, and $\overset{\ominus}{Y}$ represents an anion

of an acid], or non-toxic acid addition salts thereof.

2. A glycerol derivative according to claim 1 which is:
(2RS)-1-O-hexyl-2-dehydroxy-2-azido-3-O-[8-(N,N-dimethyl amino)-octyl]glycerol,
(2RS)-1-O-hexadecyl-2-dehydroxy-2-azido-3-O-[4-(N,N-dimethylamino)butyl]glycerol,
(2RS)-1-O-hexadecyl-2-dehydroxy-2-azido-3-O-[4-(N,N,N-trimethylammonio)butyl]glycerol iodide,
(2RS)-1-O-hexadecyl-2-dehydroxy-2-azido-3-O-[5-(N,N-dimethylamino)pentyl]glycerol,
(2RS)-1-O-hexadecyl-2-dehydroxy-2-azido-3-O-[5-(N,N-dimethylamino)pentyl]glycerol.

(2RS)-1-O-hexadecyl-2-dehydroxy-2-azido-3-O-[5-(N,N,N-trimethyl-ammonio)pentyl]glycerol iodide,

(2RS)-1-O-hexadecyl-2-dehydroxy-2-azido-3-O-[7-(N,N-dimethyl-amino)heptyl]glycerol or

(2RS)-1-O-hexadecyl-2-dehydroxy-2-azido-3-O-[7-(N,N-diethyl-amino)heptyl]glycerol.

3. A glycerol derivative according to claim 1 which is:

(2RS)-1-O-hexyl-2-dehydroxy-2-amino-3-O-[8-(N,N-dimethylamino)-octyl]glycerol,

(2RS)-1-O-hexadecyl-2-dehydroxy-2-amino-3-O-[4-(N,N-dimethyl-amino)butyl]glycerol,

(2RS)-1-O-hexadecyl-2-dehydroxy-2-amino-3-O-[4-(N,N,N-trimethyl-ammonio)butyl]glycerol iodide,

(2RS)-1-O-hexadecyl-2-dehydroxy-2-amino-3-O-[5-(N,N-dimethyl-amino)pentyl]glycerol,

(2RS)-1-O-hexadecyl-2-dehydroxy-2-amino-3-O-[5-(N,N,N-trimethyl-ammonio)pentyl]glycerol iodide,

(2RS)-1-O-hexadecyl-2-dehydroxy-2-amino-3-O-[7-(N,N-dimethyl-amino)heptyl]glycerol or

(2RS)-1-O-hexadecyl-2-dehydroxy-2-amino-3-O-[7-(N,N-diethyl-amino)heptyl]glycerol.

4. A glycerol derivative according to claim 1 which is:

(2RS)-1-O-hexadecyl-2-dehydroxy-2-benzoylamino-3-O-[4-(N,N-dimethylamino)butyl]glycerol,

(2RS)-1-O-hexadecyl-2-dehydroxy-2-benzoylamino-3-O-[4-(N,N,N-trimethylammonio)butyl]glycerol iodide,

(2RS)-1-O-hexadecyl-2-dehydroxy-2-benzoylamino-3-O-[5-(N,N-dimethylamino)pentyl]glycerol,

(2RS)-1-O-hexadecyl-2-dehydroxy-2-benzoylamino-3-O-[5-(N,N,N-trimethylammonio)pentyl]glycerol iodide,

(2RS)-1-O-hexadecyl-2-dehydroxy-2-benzoylamino-3-O-[7-(N,N,-dimethylamino)heptyl]glycerol,

(2RS)-1-O-hexadecyl-2-dehydroxy-2-benzoylamino-3-O-[7-(N,N,N-trimethylammonio)heptyl]glycerol iodide,

(2RS)-1-O-hexadecyl-2-dehydroxy-2-acetylamino-3-O-[4-(N,N-dimethylamino)butyl]glycerol,

(2RS)-1-O-hexadecyl-2-dehydroxy-2-acetylamino-3-O-[4-(N,N,N-trimethylammonio)butyl]glycerol iodide,

(2RS)-1-O-hexadecyl-2-dehydroxy-2-acetylamino-3-O-[5-(N,N,N-trimethylammonio)pentyl]glycerol iodide,

(2RS)-1-O-hexadecyl-2-dehydroxy-2-acetylamino-3-O-[7-(N,N-dimethylamino)heptyl]glycerol,

(2RS)-1-O-hexadecyl-2-dehydroxy-2-acetylamino-3-O-[7-(N,N,N-trimethylammonio)heptyl]glycerol iodide,
(2RS)-1-O-hexadecyl-2-dehydroxy-2-butyrylamino-3-O-[4-(N,N-dimethylamino)butyl]glycerol,
(2RS)-1-O-hexadecyl-2-dehydroxy-2-butyrylamino-3-O-[4-(N,N,N-trimethylammonio)butyl]glycerol iodide,
(2RS)-1-O-hexadecyl-2-dehydroxy-2-hexanoylamino-3-O-[4-(N,N-dimethylamino)butyl]glycerol or
(2RS)-1-O-hexadecyl-2-dehydroxy-2-hexanoylamino-3-O-[4-(N,N,N-trimethylammonio)butyl]glycerol iodide.

5. A glycerol derivative according to claim 1 which is:
(2RS)-1-O-hexyl-2-dehydroxy-2-isobutoxycarbonylamino-3-O-[8-(N,N-dimethylamino)octyl]glycerol,
(2RS)-1-O-hexyl-2-dehydroxy-2-isobutoxycarbonylamino-3-O-[8-(N,N,N-trimethylammonio)octyl]glycerol iodide,
(2RS)-1-O-hexadecyl-2-dehydroxy-2-phenoxycarbonylamino-3-O-[4-(N,N-dimethylamino)butyl]glycerol,
(2RS)-1-O-hexadecyl-2-dehydroxy-2-phenoxycarbonylamino-3-O-[4-(N,N,N-trimethylammonio)butyl]glycerol iodide,
(2RS)-1-O-hexadecyl-2-dehydroxy-2-methoxycarbonylamino-3-O-[4-(N,N-dimethylamino)butyl]glycerol,

(2RS)-1-O-hexadecyl-2-dehydroxy-2-methoxycarbonylamino-3-O-[4-(N,N,N-trimethylammonio)butyl]glycerol iodide,

(2RS)-1-O-hexadecyl-2-dehydroxy-2-methoxycarbonylamino-3-O-[7-(N,N-dimethylamino)heptyl]glycerol,

(2RS)-1-O-hexadecyl-2-dehydroxy-2-methoxycarbonylamino-3-O-[7-(N,N,N-trimethylammonio)heptyl]glycerol iodide,

(2RS)-1-O-hexadecyl-2-dehydroxy-2-ethoxycarbonylamino-3-O-[4-(N,N-dimethylamino)butyl]glycerol,

(2RS)-1-O-hexadecyl-2-dehydroxy-2-ethoxycarbonylamino-3-O-[4-(N,N,N-trimethylammonio)butyl]glycerol iodide,

(2RS)-1-O-hexadecyl-2-dehydroxy-2-ethoxycarbonylamino-3-O-[5-(N,N-dimethylamino)pentyl]glycerol,

(2RS)-1-O-hexadecyl-2-dehydroxy-2-ethoxycarbonylamino-3-O-[5-(N,N,N-trimethylammonio)pentyl]glycerol iodide,

(2RS)-1-O-hexadecyl-2-dehydroxy-2-butoxycarbonylamino-3-O-[4-(N,N-dimethylamino)butyl]glycerol,

(2RS)-1-O-hexadecyl-2-dehydroxy-2-butoxycarbonylamino-3-O-[4-(N,N,N-trimethylammonio)butyl]glycerol iodide,

(2RS)-1-O-hexadecyl-2-dehydroxy-2-isobutoxycarbonylamino-3-O-[4-(N,N-dimethylamino)butyl]glycerol,

(2RS)-1-O-hexadecyl-2-dehydroxy-2-isobutoxycarbonylamino-3-O-[4-(N,N,N-trimethylammonio)butyl]glycerol iodide,

(2RS)-1-O-hexadecyl-2-dehydroxy-2-isobutoxycarbonylamino-3-O-[4-(N,N,N-triethylammonio)butyl]glycerol mesylate,

(2RS)-1-O-hexadecyl-2-dehydroxy-2-isobutoxycarbonylamino-3-O-[5-

(N,N,N-trimethylammonio)pentyl]glycerol bromide,
(2RS)-1-O-hexadecyl-2-dehydroxy-2-isobutoxycarbonylamino-3-O-[6-(N,N,N-trimethylammonio)hexyl]glycerol bromide,
(2RS)-1-O-hexadecyl-2-dehydroxy-2-isobutoxycarbonylamino-3-O-[7-(N,N,N-trimethylammonio)heptyl]glycerol bromide,
(2RS)-1-O-hexadecyl-2-dehydroxy-2-isobutoxycarbonylamino-3-O-[7-(N,N-diethylamino)heptyl]glycerol,
(2RS)-1-O-hexadecyl-2-dehydroxy-2-isobutoxycarbonylamino-3-O-[7-(N,N-diethyl-N-methylammonio)heptyl]glycerol iodide,
(2RS)-1-O-hexadecyl-2-dehydroxy-2-isobutoxycarbonylamino-3-O-[8-(N,N,N-trimethylammonio)octyl]glycerol bromide,
(2RS)-1-O-hexadecyl-2-dehydroxy-2-isobutoxycarbonylamino-3-O-[9-(N,N,N-trimethylammonio)nonyl]glycerol bromide,
(2RS)-1-O-hexadecyl-2-dehydroxy-2-isobutoxycarbonylamino-3-O-[10-(N,N,N-trimethylammonio)decyl]glycerol bromide,
(2RS)-1-O-hexadecyl-2-dehydroxy-2-isobutoxycarbonylamino-3-O-[4-(N,N,N-trimethylammonio)butyryl]glycerol bromide,
(2RS)-1-O-hexadecyl-2-dehydroxy-2-isobutoxycarbonylamino-3-O-[5-(N,N,N-trimethylammonio)valeryl]glycerol bromide,
(2RS)-1-O-hexadecyl-2-dehydroxy-2-isobutoxycarbonylamino-3-O-[6-(N,N,N-trimethylammonio)hexanoyl]glycerol bromide,
(2RS)-1-O-hexadecyl-2-dehydroxy-2-isobutoxycarbonylamino-3-O-[7-(N,N,N-trimethylammonio)heptanoyl]glycerol mesylate or
(2RS)-1-O-hexadecyl-2-dehydroxy-2-isobutoxycarbonylamino-3-O-[8-(N,N,N-trimethylammonio)octanoyl]glycerol mesylate.

6. A glycerol derivatives according to claim 1. which is:

(2RS)-1-O-hexadecyl-2-dehydroxy-2-(N'-phenylureido)-3-O-[4-(N,N-dimethylamino)butyl]glycerol,

(2RS)-1-O-hexadecyl-2-dehydroxy-2-(N'-phenylureido)-3-O-[4-(N,N,N-trimethylammonio)butyl]glycerol iodide,

(2RS)-1-O-hexadecyl-2-dehydroxy-2-(N'-methylureido)-3-O-[4-(N,N-dimethylamino)butyl]glycerol,

(2RS)-1-O-hexadecyl-2-dehydroxy-2-(N'-methlureido)-3-O-[4-(N,N,N-trimethylammonio)butyl]glycerol iodide,

(2RS)-1-O-hexadecyl-2-dehydroxy-2-(N'-methylureido)-3-O-[7-(N,N-dimethylamino)heptyl]glycerol,

(2RS)-1-O-hexadecyl-2-dehydroxy-2-(N'-methylureido)-3-O-[7-(N,N,N-trimethylammonio)heptyl]glycerol iodide,

(2RS)-1-O-hexadecyl-2-dehydroxy-2-(N'-propylureido)-3-O-[4-(N,N-dimethylamino)butyl]glycerol,

(2RS)-1-O-hexadecyl-2-dehydroxy-2-(N'-propylureido)-3-O-[4-(N,N,N-trimethylammonio)butyl]glycerol iodide or

(2RS)-1-O-hexadecyl-2-dehydroxy-2-(N'-butylureido)-3-O-[4-(N,N-dimethylamino)butyl]glycerol or

(2RS)-1-O-hexadecyl-2-dehydroxy-2-(N'-butylureido)-3-O-[4-(N,N,N-trimethylammonio)butyl]glycerol iodide.

7. A process for the preparation of glycerol derivatives as claimed in claim 1 and conforming to the general formula:

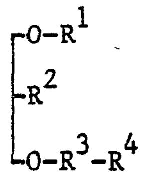

IA

(wherein the various symbols are as defined in claim 1) which comprises:

(i) reaction of a compound of the general formula:

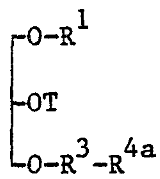

IIA

(wherein $R^{4a}$ represents a group of the general formula: $-NHCOR^6$, $-N(R^7)_2$ or $-\overset{\oplus}{N}(R^7)_3 \cdot \overset{\ominus}{Y}$, in which the various symbols are as defined in claim 1, T represents an alkylsulfonyl group, having 1 to 4 carbon atoms in the alkyl group, or a substituted or unsubstituted arylsulfonyl group, and the other symbols are as defined in claim 1) with sodium azide,

(ii) reduction of an azido group of a compound of the general formula:

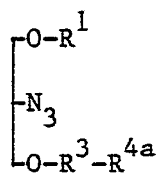

IA-11

(wherein the various symbols are as hereinbefore defined) into an amino group.

(iii) reaction of a compound of the general formula:

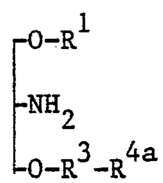

IA-12

(wherein the various symbols are as hereinbefore defined) with an acyl halide of the general formula:

$$X^1\text{-CO-}R^5 \qquad \text{III}$$

(wherein $X^1$ represents a halogen atom and $R^5$ is as defined in claim 1) or with an acid anhydride of the general formula:

$$R^5\text{-CO-O-CO-}R^5 \qquad \text{IV}$$

(wherein $R^5$ is as hereinbefore defined),

(iv) reaction of a compound of the general formula IA-12 hereinbefore depicted with an ester of haloformic acid of the general formula:

$$X^2\text{-}COOR^5 \qquad V$$

(wherein $X^2$ represents a halogen atom and $R^5$ is as hereinbefore defined),

(v) reaction of a compound of the general formula IA-12 hereinbefore depicted with an isocyanate of the general formula:

$$O{=}C{=}N\text{-}R^5 \qquad VI$$

(wherein $R^5$ is as hereinbefore defined),

(vi) elimination of tert-butoxycarbonyl group of a compound of the general formula:

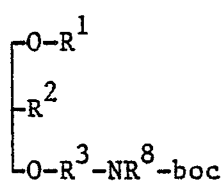

VII

(wherein boc represents tert-butoxycarbonyl group, $R^8$ represents a hydrogen atom or an alkyl group of 1 to 8 carbon atoms and the other symbols are as hereinbefore defined),

(vii) reaction of a compound of the general formula:

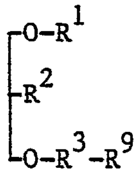

XXXII

(wherein $R^9$ represents a halogen atom or a group of the formula: -OT, in which T represents an alkylsulfonyl group, having to 4 carbon atoms in the alkyl group, or a substituted or unsubstituted arylsulfonyl group, and the other symbols are as hereinbefore defined) with a compound of the general formula:

$N(R^7)_3$ XXX

(wherein $R^7$ is as hereinbefore defined) and, if desired, subjecting methods for salt-exchange,

(viii) reaction of a compound of the general formula XXXII hereinbefore depicted with a compound of the general formula:

$HN(R^7)_2$ XXXI

(wherein $R^7$ is as hereinbefore defined),

(ix) reaction of a compound of the general formula XXXII hereinbefore depicted with a compound of the general formula:

$H_2NR^7$ XXXIII

(wherein $R^7$ is as hereinbefore defined),

(x) reaction of a compound of the general formula;

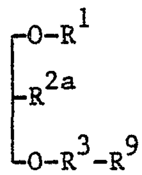

XXXIIA

(wherein $R^{2a}$ represents an azido group, an amino group or a group of the general formula: $-NHCONHR^5$, in which $R^5$ is as hereinbefore defined, and the other symbols are as hereinbefore defined) with phthalimide and then reacting the resulting compound with hydrazine,

(xi) reaction of a compound of the general formula;

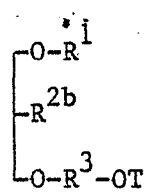

XXXIIB

(wherein $R^{2b}$ represents a group of the general formula: $-NHCOR^5$ or $-NHCOOR^5$, in which $R^5$ is as hereinbefore defined, and the other symbols are as hereinbefore defined) with sodium azide and then reducing an azido group of the resulting compound into an amino group,

(xii) acylation of a compound of the general formula:

$$\left[\begin{array}{l} -O-R^1 \\ -R^{2c} \\ -O-R^3-NH_2 \end{array}\right.$$ IA-37

(wherein $R^{2c}$ represents an azido group or a group of the general formula: $-NHCOR^5$, $-NHCOOR^5$ or $-NHCONHR^5$, in which $R^5$ is as hereinbefore defined, and the other symbols are as hereinbefore defined), or

(xiii) elimination of a tert-butoxycarbonyl group of a compound of the general formula:

$$\left[\begin{array}{l} -O-R^1 \\ -NH-boc \\ -O-R^3-NHCOR^6 \end{array}\right.$$ XXXV

(wherein the various symbols are as hereinbefore defined).

8. A process for the preparation of glycerol derivatives as claimed in claim 1 and conforming to the general formula:

$$\left[\begin{array}{l} -O-R^1 \\ -R^{2c} \\ -O-CO-R^3-R^4 \end{array}\right.$$ IB

(wherein the various symbols are as defined in claim 7) which comprises:

(i) reaction of a compound of the general formula:

$-O-R^1$
$-R^{2c}$
$-O-CO-R^3-R^9$ XLVIII

(wherein the various symbols are as defined in claim 7) with a compound of the general formula XXX depicted in claim 7, and, if desired, subjecting methods for salt-exchange,

(ii) esterification of a compound of the general formula:

$-O-R^1$
$-R^{2c}$
$-OH$ XLIX

(wherein the various symbols are as hereinbefore defined) with an acid of the general formula:

$HOOC-R^3-N(R^7)_2$ LIII

(wherein $R^3$ and $R^7$ are as hereinbefore defined) or a reactive derivative thereof,

(iii) elimination of tert-butoxycarbonyl group of a compound of the general formula:

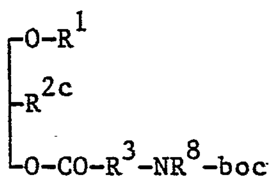

LV

(wherein $R^8$ and boc are as defined in claim 7 and the other symbols are as hereinbefore defined), or

(iv) acylation of a compound of the general formula:

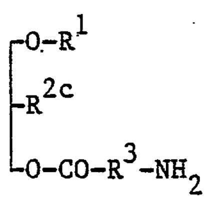

IB-31

(wherein the various symbols are as hereinbefore defined),

9. A process for the preparation of glycerol derivatives as claimed in claim 1 and conforming to the general formula:

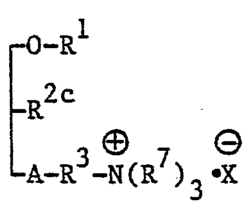

IC

(wherein X represents a halogen atom, $R^{2c}$ is as defined in claim 7 and the other symbols are as defined in claim 1) which comprises reaction of a compound of the general formula:

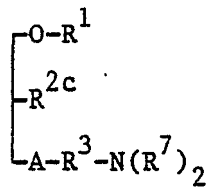

ID

(wherein the various symbols are as hereinbefore defined) with an alkyl halide.

10. A platelet aggregation inhibiting, anti-asthmatic, anti-allergic, anti-inflammatory, hypotensive or anti-tumour pharmaceutical composition which comprises, as active ingredient, an effective amount of at least one compound of the general formula I depicted in claim 1, wherein the various symbols are as defined in claim 1, or a non-toxic acid addition salt thereof, together with a pharmaceutical carrier or coating.

11. A method of inhibiting platelet aggregation, or for the prevention and treatment of asthma, allergy, inflammation, hypertension and tumour which comprises the oral or parenteral administration of an effective amount of a compound as claimed in claim 1, wherein the various symbols are as defined in claim 1, or a non-toxic acid addition salt thereof.